(19) Europäisches Patentamt
European Patent Office
Office européen des brevets

(11) Veröffentlichungsnummer: **0 303 573 B1**

(12) **EUROPÄISCHE PATENTSCHRIFT**

(45) Veröffentlichungstag der Patentschrift: **09.06.93**

(51) Int. Cl.[5]: **C07D 207/452**, C07D 209/48, A01N 43/36, C07C 255/54, C07C 255/57

(21) Anmeldenummer: **88810538.4**

(22) Anmeldetag: **05.08.88**

Die Akte enthält technische Angaben, die nach dem Eingang der Anmeldung eingereicht wurden und die nicht in dieser Patentschrift enthalten sind.

(54) **Pyrrolidin-2,5-dione und 4,5,6,7-Tetrahydroisoindol-1,3-dione.**

(30) Priorität: **14.08.87 CH 3132/87**

(43) Veröffentlichungstag der Anmeldung: **15.02.89 Patentblatt 89/07**

(45) Bekanntmachung des Hinweises auf die Patenterteilung: **09.06.93 Patentblatt 93/23**

(84) Benannte Vertragsstaaten: **AT BE CH DE ES FR GB GR IT LI LU NL SE**

(56) Entgegenhaltungen:
**EP-A- 0 190 755**
**EP-A- 0 207 894**
**EP-A- 0 216 243**
**DE-A- 3 013 162**
**DE-A- 3 109 035**
**US-A- 4 431 822**

**CHEMICAL ABSTRACTS, Band 74, Nr. 21, 24. Mai 1971, Seite 381, Spalte 2, Zusammenfassung-Nr. 111 779h, Columbus, Ohio, US; A. MORIMOTO et al.: "2-Alkoxy-4-amino-5-halobenzonitriles"**

(73) Patentinhaber: **CIBA-GEIGY AG**
**Klybeckstrasse 141**
**CH-4002 Basel(CH)**

(72) Erfinder: **Moser, Hans, Dr.**
**Stampfiweg 10**
**CH-4310 Rheinfelden(CH)**
Erfinder: **Pissiotas, Georg, Dr.**
**Am Sonnenrain 71**
**W-7850 Lörrach(DE)**
Erfinder: **Brunner, Hans-Georg, Dr.**
**Wannenstrasse 14**
**CH-4415 Lausen(CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 303 573 B1

## Beschreibung

Aus der DE-A-3 013 162 und aus der US 4,431,822 sind herbizid wirksame N-Phenyl-phthalimide die im Phenylrest in para Stellung durch Halogen und in meta Stellung über eine Etherbrücke substituiert sind. Die EP-A-0 207 894 betrifft N-Phenyl-phthalimide die im Phenylrest in para Stellung durch Halogen und in meta Stellung durch Alkylcarbonyl oder durch eine Ketalstruktur substituiert sind.

Die vorliegende Erfindung betrifft neue 1-Phenyl-pyrrolidin-2,5-dione und 2-Phenyl-4,5,6,7-tetrahydroisoindol-1,3-dione der allgemeinen Formel I, welche in der 4-Position des Phenylsubstituenten obligatorisch durch eine Cyanogruppe substituiert sind; Verfahren zur Herstellung dieser neuen Verbindungen sowie neue Zwischenprodukte.

Die Verbindungen der Formel I sind herbizid wirksam und zeichnen sich insbesondere durch selektiv herbizide Wirkung aus. Die Erfindung betrifft somit auch herbizide Mittel, enthaltend Verbindungen der Formel I sowie deren Verwendung als Herbizide und Verfahren zur Bekämpfung unerwünschten Pflanzenwuchses.

Die neuen Verbindungen entsprechen der Formel I

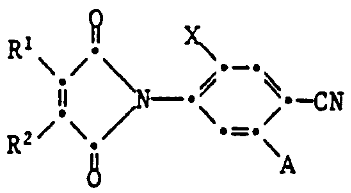

(I),

worin

$R^1$ Wasserstoff; oder $(C_1-C_4)$-Alkyl;

$R^2$ $(C_1-C_4)$-Alkyl; oder

$R^1$ und $R^2$ gemeinsam eine $-(CH_2)_4$-Gruppe bedeuten, die bis zu zweifach durch $(C_1-C_4)$-Alkyl substituiert sein kann;

X Wasserstoff; oder Halogen;

A

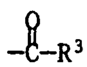

;

oder $O-R^4$

$R^3$ Hydroxy; $(C_1-C_8)$-Alkoxy; $(C_1-C_4)$-Alkoxy-$(C_1-C_4)$-alkoxy; $(C_1-C_4)$-Alkylthio-$(C_1-C_4)$-alkoxy; Mono- oder Di-$(C_1-C_4)$-Alkylamino-$(C_1-C_4)$-alkoxy; $(C_1-C_8)$-Cyanoalkoxy; $(C_3-C_8)$-Alkenyloxy; $(C_3-C_8)$-Halogenalkenyloxy; $(C_3-C_8)$-Alkinyloxy; $(C_3-C_7)$-Cycloakyloxy; $(C_3-C_7)$-Cycloalkyl-$(C_1-C_4)$-alkyloxy; Halogen-$(C_3-C_7)$-cycloalkoxy; Benzyloxy, das unsubstituiert oder am Phenylkern einfach durch Halogen, $(C_1-C_4)$-Alkyl, $(C_1-C_4)$-Alkoxy, $(C_1-C_4)$-Halogenalkyl, Cyan oder Nitro substituiert ist; Phenoxy; Halogenphenoxy; $(C_1-C_4)$-Alkylphenoxy; $(C_1-C_4)$-Alkyloxyphenoxy; $(C_1-C_4)$-Halogenalkylphenoxy; Cyanophenoxy; Nitrophenoxy; Phenylthio; Halogenphenylthio; $(C_1-C_4)$-Alkylphenylthio; $(C_1-C_4)$-Alkoxyphenylthio; $(C_1-C_4)$-Halogenalkylthio; Cyanophenylthio; Nitrophenylthio; die Salzgruppen -O-Na, -O-K, $-O-(Ca)_{0,5}$, $-O-(Mg)_{0,5}$ oder $-O-NH_4$; Amino; $(C_1-C_4)$-Alkylamino; Di-$(C_1-C_4)$-alkylamino; $(C_2-C_4)$-Halogenalkylamino; Di-$(C_2-C_4)$-halogenalkylamino; $(C_1-C_4)$-Hydroxylamino; Di-$(C_1-C_4)$-Hydroxyalkylamino; $(C_3-C_4)$-Alkenylamino; Diallylamino; N-Pyrrolidino; -N-Piperidino; N-Morpholino; -N-Thiomorpholino; N-Piperidazino; $(C_1-C_8)$-Alkylthio; $(C_3-C_8)$-Alkenylthio; Benzylthio; $(C_1-C_4)$-Alkylthio substituiert durch $(C_1-C_8)$-Alkoxycarbonyl, $(C_1-C_4)$-Alkoxy-$(C_1-C_4)$-alkoxycarbonyl, $(C_3-C_8)$-Alkenyloxycarbonyl, $(C_3-C_8)$-Alkinyloxycarbonyl, $(C_1-C_8)$-Alkylthiocarbonyl, $(C_3-C_8)$-Alkenylthiocarbonyl oder $(C_3-C_8)$-Alkinylthiocarbonyl; oder $(C_1-C_4)$-Alkoxy substituiert durch $(C_1-C_8)$-Alkoxycarbonyl, $(C_1-C_4)$-Alkoxy-$(C_1-C_4)$-alkoxycarbonyl, $(C_3-C_8)$-Alkenyloxycarbonyl, $(C_3-C_8)$-Alkinyloxycarbonyl, $(C_1-C_8)$-Alkylthiocarbonyl, $(C_3-C_8)$-Alkenylthiocarbonyl, $(C_3-C_8)$-Alkinylthiocarbonyl, $(C_1-C_4)$-Alkylaminocarbonyl, Di-$(C_1-C_4)$-Alkylaminocarbonyl oder Phenylaminocarbonyl, das unsubstituiert oder am Phenylkern einfach durch Halogen, $(C_1-C_4)$-Alkyl, $(C_1-C_4)$-Alkoxy, $(C_1-C_4)$-Halogenalkyl, Cyan oder

Nitro substituiert ist;

$R^4$ $(C_1-C_8)$-Alkyl; $(C_1-C_4)$-Akoxy-$(C_1-C_4)$-alkyl; $(C_1-C_4)$-Alkylthio$(C_1-C_4)$-alkyl, Mono- oder Di-$(C_1-C_4)$-Alkylamino-$(C_1-C_4)$-alkyl; $(C_1-C_8)$-Halogenalkyl; $(C_1-C_8)$-Cyanoalkyl; $(C_3-C_8)$-Alkenyl; $(C_3-C_8)$-Halogenalkenyl; $(C_3-C_8)$-Alkinyl; $(C_3-C_7)$-Cycloalkyl; $(C_3-C_7)$-Cycloalkyl-$(C_1-C_4)$-alkyl; $(C_3-C_7)$-Halogencycloalkyl; $(C_1-C_8)$-Alkylcarbonyl; Allylcarbonyl; Benzylcarbonyl, das unsubstituiert oder am Phenylring einfach durch Halogen, $(C_1-C_4)$-Alkyl, $(C_1-C_4)$-Alkoxy, $(C_1-C_4)$-Halogenalkyl, Cyan oder Nitro substituiert ist; $(C_3-C_7)$-Cycloalkylcarbonyl; Benzoyl, das unsubstituiert oder am Phenylring einfach durch Halogen, $(C_1-C_4)$-Alkyl, $(C_1-C_4)$-Alkoxy; $(C_1-C_4)$-Halogenalkyl, Cyan oder Nitro substituiert ist; Furoyl; Thienoyl; $(C_1-C_4)$-Alkyl substituiert durch Phenyl, Halogenphenyl, $(C_1-C_4)$-Alkylphenyl, $(C_1-C_4)$-Alkoxyphenyl, $(C_1-C_4)$-Haloalkylphenyl, $(C_1-C_4)$-Halogenalkoxyphenyl, Nintrophenyl, Cyanophenyl, $(C_1-C_8)$-Alkoxycarbonyl, $(C_1-C_4)$-Alkoxy-$(C_1-C_4)$-alkoxycarbonyl, $(C_3-C_8)$-Alkenyloxycarbonyl, $(C_3-C_8)$-Alkinyloxycarbonyl, $(C_1-C_8)$-Alkylthiocarbonyl, $(C_3-C_8)$-Alkenylthiocarbonyl, $(C_3-C_8)$-Alkinylthiocarbonyl, Carbamoyl, $(C_1-C_4)$-Alkylaminocarbonyl, Di-$(C_1-C_4)$-Alkylaminocarbonyl, Hydroxy, Phenylaminocarbonyl, das unsubstituiert oder am Phenylring einfach durch Halogen, $(C_1-C_4)$-Alkyl, $(C_1-C_4)$-Alkoxy, Cyan, Nitro oder $(C_1-C_4)$-Halogenalkyl substituiert ist; Dioxolan-2-yl, das unsubstituiert oder durch ein bis zwei $(C_1-C_4)$-Alkylreste substituiert ist oder 1,3-Dioxan-2-yl, das unsubstituiert oder durch ein bis zwei $(C_1-C_4)$-Alkylreste substituiert ist; bedeutet.

In der obigen Definition umfassen die angegebenen generischen Begriffe beispielsweise die folgenden spezifischen Einzelsubstituenten, wobei diese Aufzählung keine Einschränkung der Erfindung darstellt:

Alkyl: Methyl, Ethyl, n-Propyl, i-Propyl, n-Butyl, i-Butyl, s-Butyl und t-Butyl, vorzugsweise Methyl und Ethyl.

Halogen: Fluor, Chlor, Brom und Jod, vorzugsweise Fluor und Chlor.

Alkoxy: Methoxy, Ethoxy, Propyloxy, n-Butyloxy, i-Butyloxy, s-Butyloxy und t-Butyloxy, vorzugsweise Methoxy oder Ethoxy.

Halogenalkyl: Fluormethyl, Difluormethyl, Trifluormethyl, Chlormethyl, Dichlormethyl, Trichlormethyl, 2,2,2-Triflorethyl, 2-Fluorethyl, 2-Chlorethyl und 2,2,2-Trichlorethyl, vorzugsweise Chlormethyl, 2-Chlorethyl und Trifluormethyl.

Halogenalkoxy: Fluormethoxy, Difluormethoxy, Trifluormethoxy, 2,2,2-Trifluorethoxy, 1,1,2,2-Tetrafluorethoxy, 2-Fluorethoxy, 2-Chlorethoxy und 2,2,2-Trichlorethoxy, vorzugsweise Difluormethoxy, 2-Chlorethoxy und Trifluormethoxy.

Alkoxylcarbonyl: Methoxycarbonyl, Ethoxycarbonyl, 4-Propyloxycarbonyl, i-Propyloxycarbonyl und n-Butyloxycarbonyl, vorzugsweise Methoxycarbonyl und Ethoxycarbonyl.

Alkoxyalkyl: Methoxymethyl, Ethoxymethyl, Propyloxymethyl, Methoxyethyl, Ethoxyethyl, Propyloxyethyl, Methoxypropyl, Ethoxypropyl oder Propyloxypropyl.

Alkylthioalkyl: Methylthiomethyl, Ethylthiomethyl, Methylthioethyl, Aethylthioethyl, oder Isopropylthioethyl.

Alkylaminoalkyl: Methylaminoethyl, Dimethylaminoethyl, Ethylaminoethyl oder Diethylaminoethyl.

Cyanoalkyl: Cyanomethyl, Cyanoethyl oder Cyanopropyl.

Alkenyl: Allyl, 2-Butenyl, 3-Butenyl oder Methallyl, vorzugsweise aber Allyl.

Alkinyl: Propargyl, 2-Butinyl oder 3-Butinyl, vorzugsweise aber Propargyl.

Cycloalkyl: Cyclopropyl, Cyclobutyl, Cyclopentyl, Cyclohexyl oder Cycloheptyl. Bevorzugt sind Cyclopentyl oder Cyclohexyl.

Halogencycloalkyl: 2,2-Dichlorcyclopropyl oder Pentachlorcyclohexyl.

Alkylsulfonyl: Methylsulfonyl, Ethylsulfonyl, Propylsulfonyl oder Butylsulfonyl. Bevorzugt sind Methyl- und Ethylsulfonyl.

Cycloalkoxycarbonyl: Cyclopentyloxycarbonyl oder Cyclohexyloxycarbonyl.

Phenyl, auch als Teil eines grösseren Substituenten wie Phenoxy, Phenylthio, Phenoxycarbonyl, Phenylaminocarbonyl, Benzyl oder Benzoyl, kann im allgemeinen unsubstituiert oder durch einen weiteren Substituenten substituiert vorliegen. Die Substituenten können dann in ortho-, meta- oder para-Stellung stehen. Bevorzugte Substituentenstellungen sind die ortho- und para-Position zur Ringverknüpfungsstelle. Bevorzugte Substituenten sind Halogenatome.

In den weiteren Substituenten, welche aus mehreren Grundelementen zusammengesetzt sind, haben die Teilelemente die oben an Beispielen erläuterten Bedeutungen. Diese Aufzählungen bedeuten auch in diesen Fällen keine Einschrändung der Erfindung: sie haben illustrierenden Charakter.

Bevorzugt sind die Verbindungen der Formel I, worin

$R^1$ Wasserstoff; oder $(C_1-C_4)$-Alkyl;

$R^2$ $(C_1-C_4)$-Alkyl; oder

$R^1$ und $R^2$ gemeinsam eine $-(CH_2)_4$-Gruppe bedeuten, die gegebenenfalls einfach durch $(C_1-C_4)$-Alkyl

substituiert sein kann;

X Wasserstoff; Fluor; Chlor; oder Brom;

A

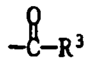 ;

oder O-$R^4$;

$R^3$ Hydroxy; ($C_1$-$C_5$)-Alkoxy; ($C_1$-$C_4$)-Alkoxy-($C_1$-$C_4$)-alkoxy; ($C_1$-$C_4$)-Alkylthio-($C_1$-$C_4$)-alkoxy; Mono- oder Di-($C_1$-$C_4$)-Alkylamino-($C_1$-$C_4$)-alkoxy; ($C_1$-$C_4$)-Cyanoalkoxy; ($C_3$-$C_5$)-Halogenalkenyloxy; ($C_3$-$C_5$)-Alkinyloxy; ($C_3$-$C_5$)-Alkenyloxy; ($C_3$-$C_6$)-Cycloalkylmethyloxy; die Salzgruppen -O-Na, O-K, -O-$(Ca)_{0,5}$, -O-$(mg)_{0,5}$ oder -O-$NH_4$; Amino; Di-($C_1$-$C_4$)-Alkylamino; Diallylamino; Benzyloxy; -N-Piperidino; -N-Morpholino; N-Thiomorpholino; ($C_1$-$C_4$)-Alkylthio; ($C_1$-$C_4$)-Alkylthio, substituiert durch ($C_1$-$C_4$)-Alkoxycarbonyl; ($C_1$-$C_4$)-Alkoxy, substituiert durch ($C_1$-$C_4$)-Alkoxycarbonyl, ($C_1$-$C_4$)-Alkylthiocarbonyl, ($C_1$-$C_4$)-Alkylaminocarbonyl oder Di-($C_1$-$C_4$)-Alkylaminocarbonyl;

$R^4$ ($C_1$-$C_5$)-Alkyl; ($C_1$-$C_4$)-Alkoxy-($C_1$-$C_4$)-alkyl; ($C_1$-$C_4$)-Alkylthio-($C_1$-$C_4$)-alkyl; ($C_1$-$C_4$)-Halogenalkyl; ($C_3$-$C_5$)-Alkenyl; ($C_3$-$C_5$)-Halogenalkenyl; ($C_3$-$C_5$)-Alkinyl; Cyclohexylmethyl; ($C_1$-$C_4$)-Alkylcarbonyl; Benzoyl, das unsubstituiert oder am Phenylring einfach durch Fluor, Chlor, ($C_1$-$C_4$)-Alkyl, ($C_1$-$C_4$)-Alkoxy, Cyan oder Nitro substituiert ist; ($C_1$-$C_4$)-Alkyl, einfach substituiert durch Phenyl, ($C_1$-$C_4$)-Alkoxycarbonyl, ($C_1$-$C_4$)-Alkylthiocarbonyl, Carbamoyl, Di-($C_1$-$C_4$)-Alkylaminocarbonyl oder Hydroxy;

bedeutet.

Besonders bevorzugt sind Verbindungen der Formel I worin

$R^1$ Wasserstoff; Methyl, oder Ethyl;

$R^2$ Methyl; oder Ethyl oder

$R^1$ und $R^2$ gemeinsam eine -$(CH_2)_4$-Gruppe bedeuten, die gegebenenfalls einfach durch Methyl substituiert sein kann;

X Wasserstoff; Fluor; oder Chlor;

A

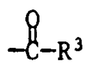 ;

oder -O-$R^4$;

$R^3$ Hydroxy; ($C_1$-$C_5$)-Alkoxy; ($C_1$-$C_2$)-Alkoxy-($C_1$-$C_2$)alkoxy; ($C_1$-$C_4$)-Alkylthio-($C_1$-$C_3$)-alkoxy; Dimethylamino-($C_1$-$C_3$)-alkoxy; Allyloxy; Chlorallyloxy; Propargyloxy; Cyclopropylmethyloxy; Cyclohexylmethyloxy; die Salzgruppen -O-Na, -O-K, -O-$(Ca)_{0,5}$, -O-$(Mg)_{0,5}$ oder -O-$NH_4$; Dimethylamino; Diallylamino; -N-Morpholino; Methylthio; ($C_1$-$C_2$)-Alkylthio, einfach substituiert durch ($C_1$-$C_3$)-Alkoxycarbonyl; ($C_1$-$C_2$)-Alkoxy, einfach substituiert durch ($C_1$-$C_4$)-Alkoxycarbonyl, ($C_1$-$C_3$)-Alkylthiocarbonyl oder N,N-Dimethylcarbamoyl;

$R^4$ ($C_1$-$C_5$)-Alkyl; Methoxyethyl; ($C_1$-$C_3$)-Alkylthio-($C_1$-$C_3$)-alkyl; Allyl; Chlorallyl; Propargyl; Cyclohexylmethyl; Acetyl; Benzoyl; ($C_1$-$C_3$)-Alkyl, einfach substituiert durch Phenyl, ($C_1$-$C_4$)-Alkoxycarbonyl, Methylthiocarbonyl, Carbamoyl, N,N-Dimethylcarbamoyl oder Hydroxy;

bedeutet.

Hervorzuheben sind die Verbindungen der Formel I, worin X für Fluor oder Wasserstoff steht.

Des weitern sind die Verbindungen der Formel I bevorzugt, in denen $R^4$ für Isopropyl steht und Verbindungen der Formel I, in denen $R^3$ für sek-Butyl steht.

Zu nennen sind insbesondere:

1-(4-Cyano-2-fluor-5-isopropyloxyphenyl)-3,4-dimethyl-pyrrol-(1H)2,5-dion, 2-(5-Carboxy-4-cyano-2-fluorphenyl)-4,5,6,7-tetrahydroisoindol-(2H)1,3-dion, 2-(4-Cyano-2-fluor-5-n-propyloxycarbonylphenyl)-4,5,6,7-tetrahydroisoindol-(2H)1,3-dion, 2-(5-n-Butyloxycarbonyl-4-cyano-2-fluorphenyl)-4,5,6,7-tetrahydroisoindol-(2H)1,3-dion, 2-(4-Cyano-2-fluor-5-isopropyloxyophenyl)-5-methyl-4,5,6,7-tetrahydroisoindol-(2H)1,3-dion, 1-(4-Cyano-2-fluor-5-isopropyloxyphenyl)-3-ethyl-4-methyl-pyrrol-(1H)2,5-dion, 2-(4-Cyano-2-fluor-5-isopropyloxyphenyl)-4,5,6,7-tetrahydroisoindol-(2H)1,3-dion, 2-(4-Cyano-2-fluor-5-isopropyloxycarbonylphenyl)-4,5,6,7-tetrahydroisoindol-(2H)1,3-dion, und 2-(5-sek-Butyloxycarbonyl-4-cyano-2-fluorphenyl)-4,5,6,7-tetrahydroiso-

indol-(2H)1,3-dion.

Analog zu literaturbekannten Verfahren können die Verbindungen der Formel I hergestellt werden

a) durch Umsetzung eines Furan-2,5-dione der Formel II mit einem Anilin III

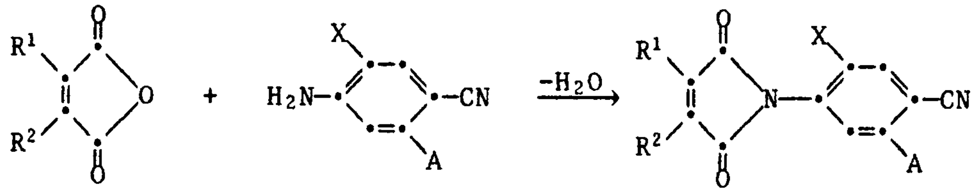


II III I,

worin die Reste $R^1$, $R^2$, X und A wie zuvor definiert sind oder

b) durch einen Halogen-Cyanid-Austausch ausgehend von einer Verbindung der Formel IV

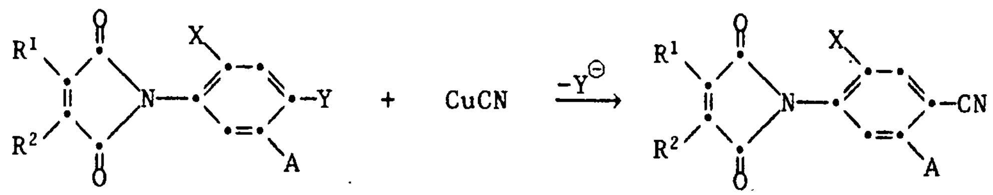


IV,

worin die Reste $R^1$, $R^2$, X und A wie zuvor definiert sind und Y für Chlor, Brom oder Jod steht, mit CuCN.

Des weitern können Verbindungen der Formel Ia, in denen A $COR^3$ bedeutet, hergestellt werden durch:

c) Kondensation einer Verbindung der Formel V, worin $R^1$, $R^2$ und X wie zuvor definiert sind,

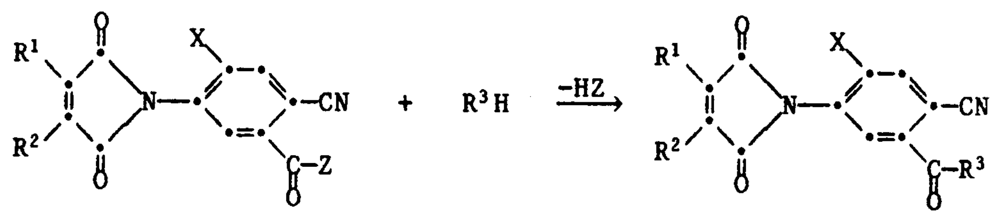


V VI I

und Z für eine unter den Reaktionsbedingungen austauschbare Gruppe, wie Halogen oder ($C_1$-$C_4$)-Alkylcarbonyloxy, steht, mit einer Verbindung der Formel VI, worin $R^3$ wie zuvor definiert ist, oder

d) durch Veresterung einer Carbonsäure der Formel Ia', worin die Reste $R^1$, $R^2$ und X wie zuvor definiert sind, mit einem Alkohol oder Thioalkohol der Formel VI

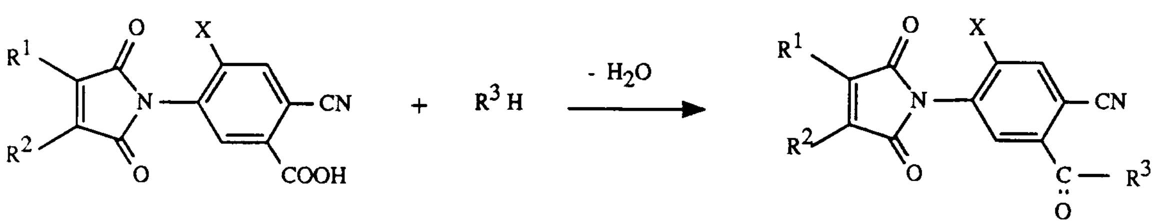


Ia' VI Ia

worin

$R^3$ ($C_1$-$C_8$)-Alkoxy; ($C_1$-$C_4$)-Alkoxy-($C_1$-$C_4$)-alkoxy; ($C_1$-$C_4$)-Alkylthio-($C_1$-$C_4$)alkoxy; Mono- oder Di-($C_1$-$C_4$)-Alkylamino-($C_1$-$C_4$)-alkoxy; ($C_1$-$C_8$)-Cyanoalkoxy; ($C_3$-$C_8$)-Alkenyloxy; ($C_3$-$C_8$)-Halogenalkenyloxy; ($C_3$-$C_8$)-Alkinyloxy; ($C_3$-$C_7$)-Cycloalkyloxy; ($C_3$-$C_7$)-Cycloalkyl-($C_1$-$C_4$)alkyloxy; Halogen-($C_3$-$C_7$)-cycloalkoxy; Benzyloxy, das unsubstituiert oder am Phenylkern einfach durch Halogen, ($C_1$-$C_4$)-Alkyl, ($C_1$-$C_4$)-Alkoxy, ($C_1$-$C_4$)Halogenalkyl, Cyan oder Nitro substituiert ist; Phenoxy, Halogenphenoxy; ($C_1$-$C_4$)-Alkylphenoxy; ($C_1$-$C_4$)-Alkyloxyphenoxy; Halogenalkylphenoxy; Cyanophenoxy; Nitrophenoxy; Phenylthio; Halogenphenylthio; ($C_1$-$C_4$)-Alkylphenylthio; ($C_1$-$C_4$)-Alkoxyphenylthio; ($C_1$-$C_4$)-Halogenalkylthio; Cyanophenylthio; Nitrophenylthio; ($C_1$-$C_8$)-Alkylthio; ($C_3$-$C_8$)-Alkenylthio; Benzylthio; ($C_1$-$C_4$)-Alkylthio substituiert durch ($C_1$-$C_8$)-Alkoxycarbonyl, ($C_1$-$C_4$)-Alkoxy-($C_1$-$C_4$)-alkoxycarbonyl, ($C_3$-$C_8$)-Alkenyloxycarbonyl, ($C_3$-$C_8$)-Alkinyloxycarbonyl, ($C_1$-$C_8$)-Alkylthiocarbonyl oder ($C_3$-$C_8$)-Alkinylthiocarbonyl; oder ($C_1$-$C_4$)-Alkoxy substituiert durch ($C_1$-$C_8$)-Alkoxycarbonyl, ($C_1$-$C_4$)-Alkoxy-($C_1$-$C_4$)-alkoxycarbonyl, ($C_3$-$C_8$)-Alkenyloxycarbonyl, ($C_3$-$C_8$)-Alkinyloxycarbonyl, ($C_1$-$C_8$)-Alkylthiocarbonyl, ($C_3$-$C_8$)-Alkenylthiocarbonyl, ($C_3$-$C_8$)-Alkinylthiocarbonyl, ($C_1$-$C_4$)-Alkylaminocarbonyl, Di-($C_1$-$C_4$)-Alkylaminocarbonyl oder Phenylaminocarbonyl, das unsubstituiert oder am Phenylkern einfach durch Halogen, ($C_1$-$C_4$)-Alkyl, ($C_1$-$C_4$)-Alkoxy, ($C_1$-$C_4$)-Halogen, Cyan oder Nitro substituiert ist bedeutet;

zu einem Ester der Formel Ia, worin die Reste $R^1$, $R^2$, $R^3$ und X wie zuvor definiert sind, vorzugsweise in Gegenwart wasserentziehender oder wasserbindender Mittel.

Verbindungen der Formel Ib, in denen A für $OR^4$ steht, können ausserdem nach an sich bekannten Verfahren hergestellt werden durch

e) Veretherung eines Phenols der Formel VII, worin $R^1$, $R^2$ und X wie zuvor definiert sind

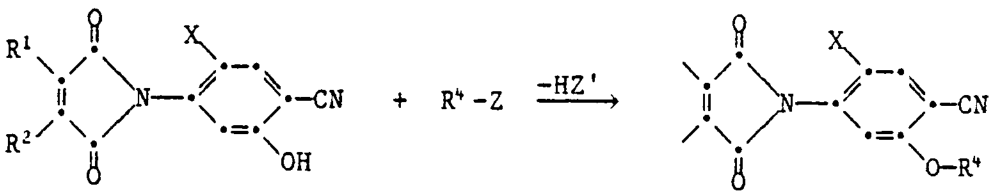


VII VIII Ib

mit einer Verbindung der Formel VIII, in der $R^4$ wie zuvor definiert ist und Z für eine unter den Reaktionsbedingungen abspaltbare Gruppe, wie Halogen oder gegebenenfalls alkyliertes Phenylsulfonyloxy, steht.

Mit Vorteil führt man die obigen Umsetzungen in einem reaktionsinerten Lösungsmittel aus. Dabei kommen als inerte Lösungsmittel Kohlenwasserstoffe, wie Benzol, Toluol oder Xylol; Ether, wie Diethylether, Methylisoproplyether, Glyme, Diglyme; cyclische Ether, wie Tetrahydrofuran und Dioxan; Ketone, wie Aceton, Methylethylketon; Amide, wie Dimethylformamid, N-Methylpyrrolidon; Sulfoxide, wie Dimethylsulfoxid; oder chlorierte Kohlenwasserstoffe, wie Dichlormethan, Trichlormethan, Tetrachlormethan oder Tetrachlorethan in Betracht.

Die Reaktionstemperatur kann in weiten Grenzen variiert werden. Geeignete Reaktionstemperaturen liegen beispielsweise zwischen -20 °C und der Rückflusstemperatur des Reaktionsgemische. Vorzugsweise wird die Umsetzung bei einer Temperatur zwischen 0 °C und 100 °C durchgeführt. Im falls des Halogen-Cyanid-Austausches (Reaktion b) sind höhere Temperaturen, vorzugsweise zwischen 100 °C und 180 °C, bevorzugt.

Die Veresterungsreaktion d) kann besonders schonend unter Verwendung von Dicyclohexylcarbodiimid oder des Systems Azodicarbonsäurediethylester/Triphenylphospin in Dichlormethan oder Tetrahydrofuran bei Temperaturen zwischen 0 °C und Raumtemperatur durchgeführt werden. Die Veresterungsreaktion d) kann auch gemäss üblicher Laboratoriumspraxis unter Verwendung wasserentziehender Mittel, wie etwa Schwefelsäure oder bei Verwendung katalytischer Mengen einer Protonensäure, unter Verwendung eines Wasserabscheiders durchgeführt werden.

Im allgemeinen die Kondensationsreaktionen a), c) und e) durch Zusatz von Kondensations-Katalysatoren und Entfernung des entstehenden Reaktionsproduktes ($H_2O$, HZ bzw. HZ') beschleunigt werden.

Als Katalysatoren kommen insbesondere dann in Betracht, wenn ein unprotisches Lösungsmittel verwendet wird: p-Toluolsulfonsäure, Benzoesäure, Schwefelsäure, Chlorwasserstoff oder Naphthalinsulfonsäure. Reaktionsführungen gemäss dem oben genannten Typ werden bei der Darstellung von Carbonsäure-Derivaten üblicherweise angewendet. Sie entsprechen der allgemeinen Laboratoriumspraxis.

Im Fall der Reaktion e) ist es vorteilhaft unter Basenzusatz zu arbeiten. Geeignete Basen sind u.a. Natrium-, Kalium- und Calciumhydroxid, Alkali- und Erdalkalicarbonate, Amine, wie etwa Triethylamin oder Heterocyclen, wie Pyridin, 4-Dialkylaminopyridine, DABCO sowie Alkalihydride.

Die Reaktionen b) und e) können auch vorteilhaft unter Phasentransfer-Bedingungen in Zweiphasensystemen durchgeführt werden. Derartige Reaktionen sind dem Fachmann geläufig (z.B. beschrieben in Dehmlow und Dehmlow, Phase Transfer Catalysis; Verlag Chemie; Weinheim 1983).

Obwohl die als Reaktion a) skizzierte Synthese des Endprodukts aus einem Furandion II und einem Anilin III in allen Fällen durchführbar ist, kann es aus ökonomischen oder verfahrenstechnischen Gründen sinnvoll sein, bestimmte Verbindungen der Formel I in andere, ebenfalls von Formel I umfasste, Derivate zu überführen. Beispiel für derartige Umwandlungen von bestimmten Wirkstoffen der Formel I in andere Wirkstoffe der Formel I ist die Umsetzung d). Darüber hinaus können aber auch Verbindungen der Formel I, in denen $R^3$ oder $R^4$ etwa für Halogenalkyl bzw. Halogenalkoxy stehen, durch Umsetzungen mit Alkoholen oder Aminen in Alkoxyalkyl-, Alkoxyalkoxy-, Aminoalkyl- oder Aminoalkoxyreste überführt werden. Derartige Derivatisierungsreaktionen sind dem Fachmann geläufig.

Die Verbindungen der Formeln III, V und VII sind wertvolle Zwischenprodukte zur Herstellung der erfindungsgemässen Wirkstoffe. Die Anmeldung betrifft somit auch diese Zwischenverbindungen, in denen die Reste $R^1$, $R^2$, X, Y, A und Z wie zuvor definiert sind, mit Ausnahme des aus Chem. Abstr. (1971) 74, 111 779 h bekannten 2-Methoxy-4-amino-5-chlorbenzonitrils der Formel III.

Die Herstellung der Zwischenprodukte III, V und VII geschieht analog zu literaturbekannten Verfahren: Die Aniline der Formel IIIb, worin A für $OR^4$ steht, sind ausgehend von den literaturbekannten Nitrobenzolen der Formel IX (bekannt z.B. aus EP-A 61741), Chem.Ber. 59, 1254) in einer dreistufigen Reaktionsfolge gemäss Schema 1 darstellbar:

Schema 1

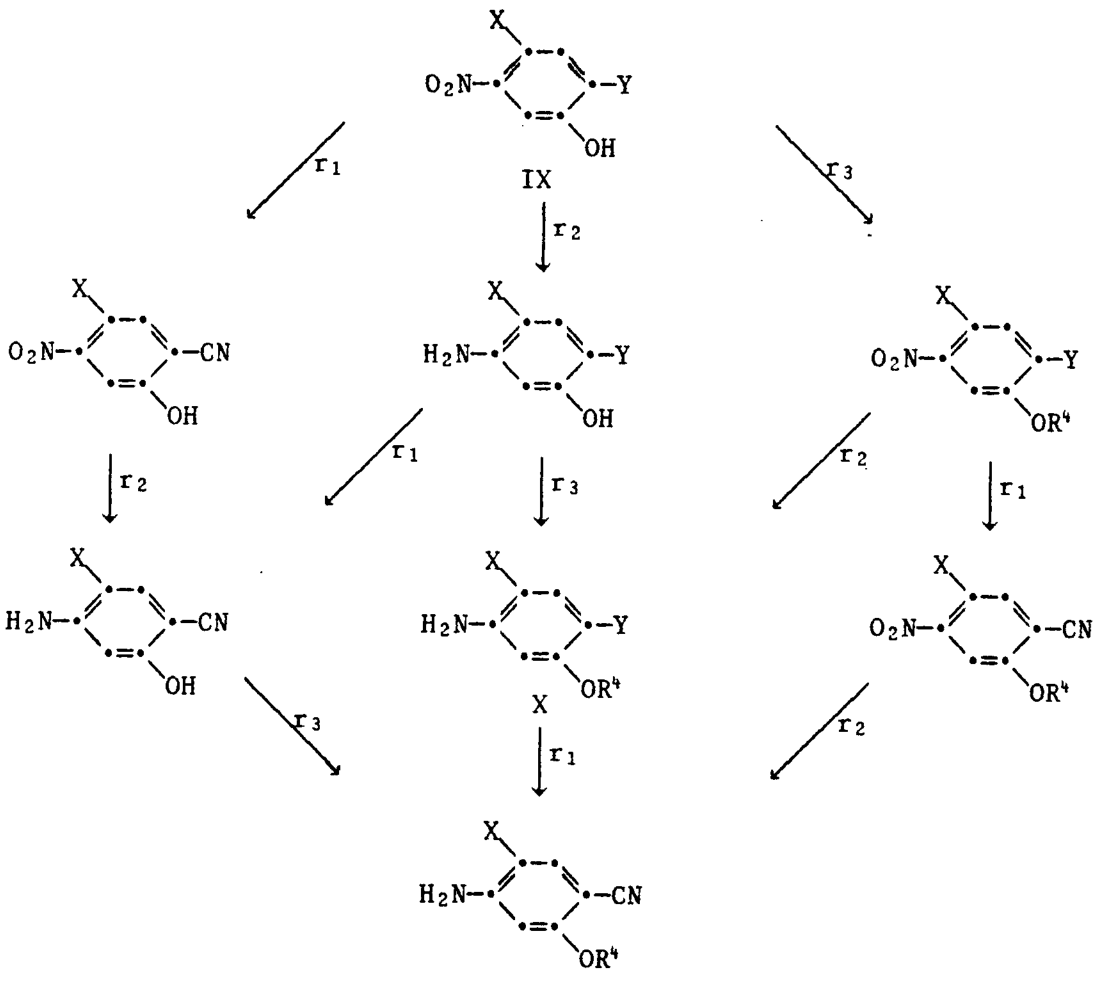

IIIb

Die hierzu notwendigen Reaktionsschritte $r_1$ (Halogen-Cyanid-Austausch), $r_2$ (Reduktion) und $r_3$ - (Veretherung) sind dem Fachmann bekannt und können analog zu literaturbekannten Verfahren durchgeführt werden.

In analoger Weise können die Verbindungen der Formel IIIa, in denen A für $-COR^3$ steht, gemäss Schema 2 aus den Nitrobenzolen XI durch

Schema 2

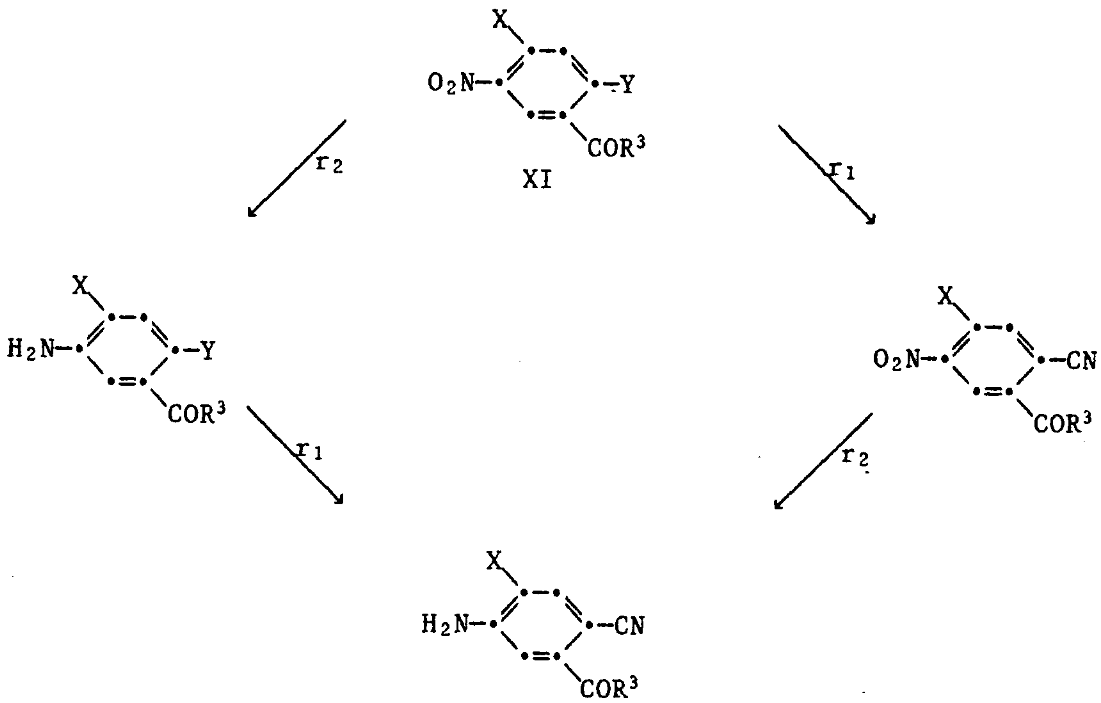

IIIc

Halogen-Cyanid-Austausch ($r_1$) und Reduktion ($r_2$) hergestellt werden.

Ein weiterer genereller Zugang zu den Verbindungen der Formel I wird anhand von Reaktionsschema 3 skizziert, in dem das aus dem

Schema 3

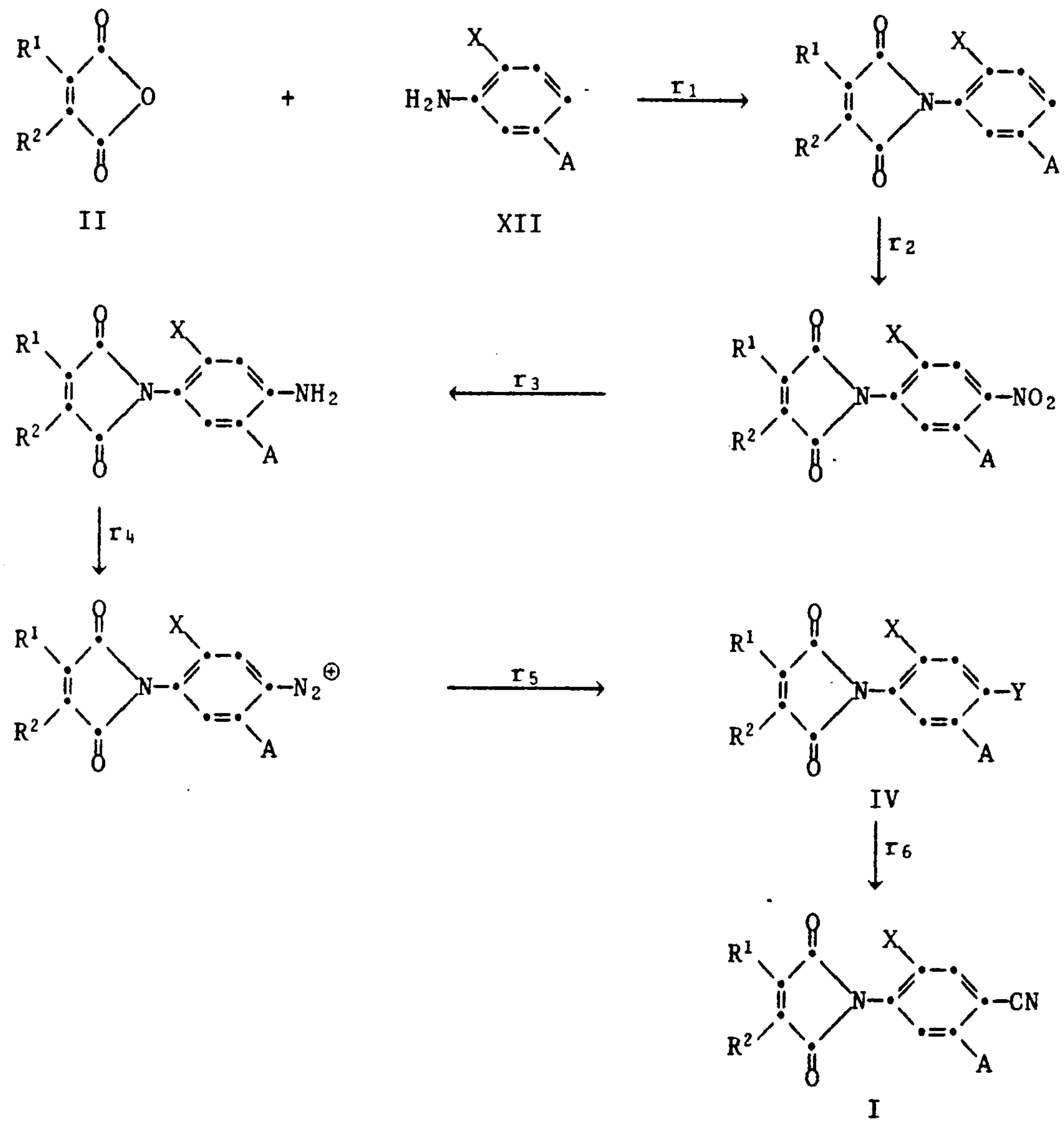


Furandion II und dem Anilin XII erhältlichen N-Phenylpyrrolidindion ($r_1$) zunächst in der p-Position am Phenylring nitriert ($r_2$), reduziert ($r_3$) und danach diazotiert ($r_4$) wird. Aus dem so zugänglichen Diazoniumsalz kann nach Sandmeyer ($r_4/r_5$) der Substituent Y eingeführt und dann durch Halogen/Cyan-Austausch ($r_6$) Verbindungen der Formel I hergestellt werden.

Ebenfalls breit anwendbar ist das in Schema 4 angegebene Verfahren zur Herstellung von Verbindungen der Formel Ia, in denen A für $COR^4$ steht:

Schema 4

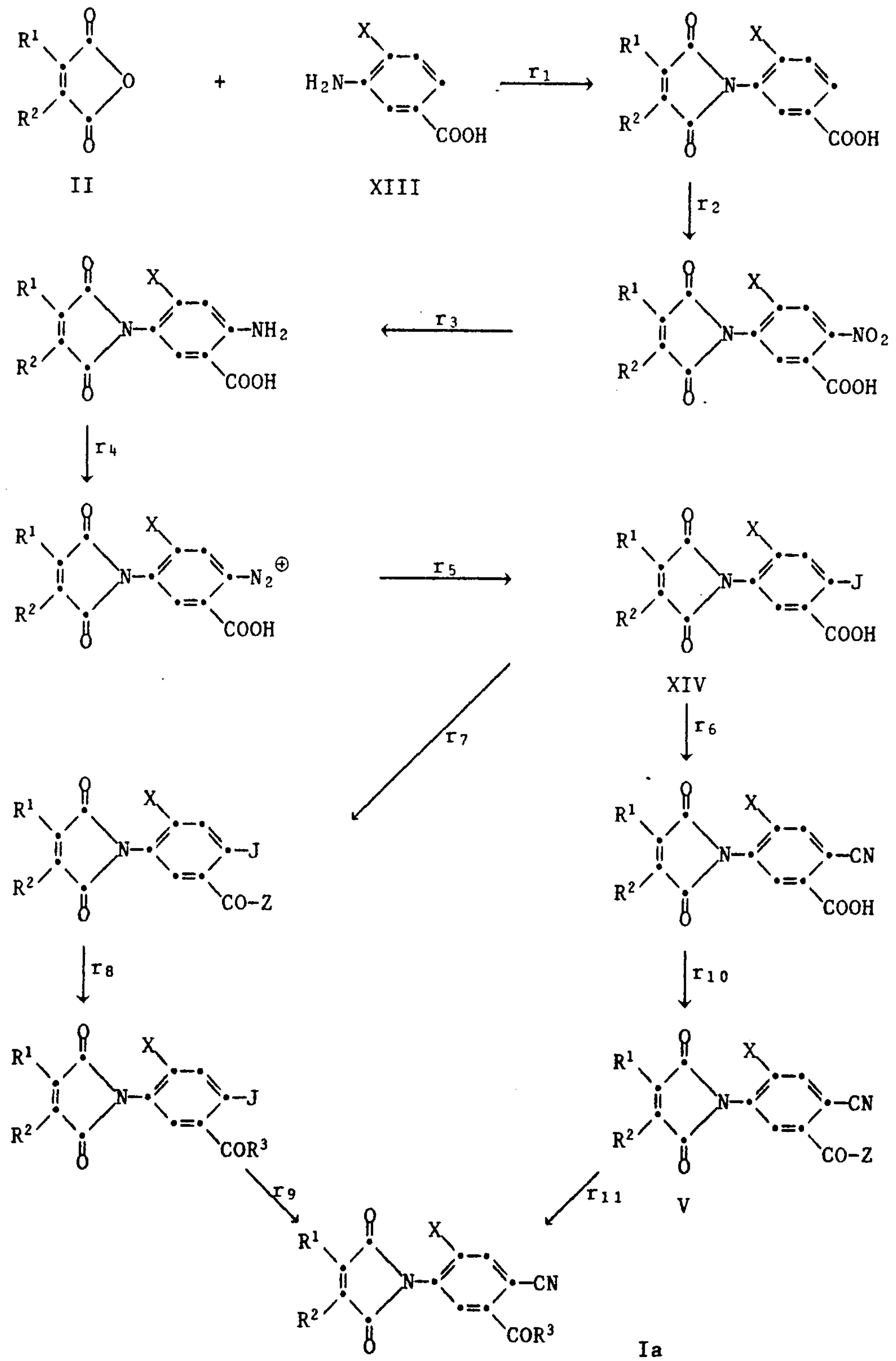


Die Verfahrensschritte $r_1$ bis $r_6$ entsprechen dabei den in Schema 3 erläuterten Reaktionen, mit dem Unterschied, dass hier ausgehend von der von Schema 3 mit umfassten m-Anthranilsäure XIII zunächst die Jodverbindung XIV hergestellt wird.

Verbindung XIV kann dann in ein aktiviertes Säurederivat ($r_7$) überführt, verestert ($r_8$) und dann zu Verbindung Ia ($r_9$) umgesetzt werden oder man stellt Verbindung Ia durch Umkehrung der Reaktionsstufen

durch Jod-Cyan-Austausch ($r_6$), Ueberführung in ein aktiviertes Säurederivat ($r_{10}$) und Veresterung ($r_{11}$) her.

Die vorstehenden Reaktionsschemata 1 bis 4 können sinngemäss auch für die Herstellung der Zwischenprodukte III, V und VII Anwendung finden (mit A bzw. $OR^4$ = CO-Z oder OH).

Die Verbindungen der Formel I sind hochaktive Herbizide, die sich bei entsprechenden Aufwandmengen in vorteilhafter Weise als Selektivherbizide zur Unkrautbekämpfung in Nutzpflanzenkulturen eignen. Kulturpflanzen wie Roggen, Gerste, Hafer, Weizen, Mais, Hirse, Reis, Baumwolle und Soja bleiben bei niedrigen Aufwandmengen praktisch ungeschädigt. Bei gesteigerten Aufwandmengen werden die Kulturpflanzen nur geringfügig in ihrem Wachstum beeinflusst. Werden sehr hohe Aufwandmengen appliziert, entfalten die Substanzen der Formel I totalherbizide Eigenschaften.

Die selektiv-herbizide Wirkung der erfindungsgemässen Verbindungen wird sowohl bei der preemergenten als auch der postemergenten Anwendung festgestellt. Diese Wirkstoffe können im Vorauflaufverfahren und im Nachauflaufverfahren zur selektiven Unkrautbekämpfung mit gutem Erfolg verwendet werden.

Die erfindungsgemässen Wirkstoffe greifen in den Metabolismus der Pflanzen ein und können deshalb auch als Wachstumsregulatoren eingesetzt werden.

Für die Wirkungsweise von Pflanzenwachstumsregulatoren gilt nach der bisherigen Erfahrung, dass ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf des Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluss von Wachstumsregulatoren kann der Blattbestand der Pflanzen so gesteuert werden, dass ein Entblättern der Pflanzen zu einem gewünschten Zeitpunkt erreicht wird (Defoliation). Eine derartige Entlaubung spielt bei der mechanischen Beerntung der Baumwolle eine grosse Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso lässt sich mit Wachstumsregulatoren der Fruchtfall steuern. Einerseits kann ein vorzeitiger Fruchtfall verhindert werden. Andererseits kann aber der Fruchtfall oder sogar das Abfallen der Blüten bis zu einem gewünschten Masse gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schliesslich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

In vorteilhafter Weise können die erfindungsgemässen Wirkstoffe oder Mittel auch auf das Vermehrungsgut der Kulturpflanze aufgebracht werden. Besonders zu erwähnen ist hier die Samenbeizung. Vermehrungsgut sind Samen, Stecklinge oder sonstige Teile der Pflanze, aus denen die Kulturpflanze gezogen werden kann. Das mit einer pflanzenwuchsregulatorisch oder herbizid wirksamen Menge einer Verbindung der Formel I behandelte Vermehrungsgut ist ebenfalls Gegenstand der Erfindung.

Die Erfindung betrifft auch herbizide und wuchsregulatorische Mittel, welche einen neuen Wirkstoff der Formel I enthalten, sowie Verfahren zu pre- und postemergenten Unkrautbekämpfung.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether Ketone wie Cyclohexanon, stark polare Lösungsmittel wie N-Methyl-2-pyrrolidon,Dimethylsulfoxid oder Dimethylformamid, sowie gegebenenfalls epoxidierte Pflanzenöle, wie epoxidiertes Kokosnussöl oder Sojaöl oder Wasser.

Als feste Trägerstoffe, z.B. Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nicht ionische Tenside in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Wietere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte. Tributylphenoxypolyethoxyelthanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ethylammoniumbromid.

Die in der Formulierungstechnik gebräuchlichen Tenside sind u.a. in folgenden Publikationen beschrieben:

"1986 International Mc Cutcheon's Emulsifiers & Detergents" Glen Rock, N.J. USA;

H. Stache, "Tensid-Taschenbuch", 2. Auflage., C. Hanser Verlag, München, Wien, 1981;

M. and J. Ash. "Encyclopedia of Surfactants", Vol. I-III, Chemical Publishing Co., New York, 1980-1981.

Die Wirkstoffzubereitungen enthalten in der Regel 0,1 bis 95 %, insbesondere 0,1 bis 80 % Wirkstoff der Formel I, 1 bis 99,9 % eines oder mehrerer fester oder flüssiger Zusatzstoffe und 0 bis 25 % eines Tensides.

Insbesondere setzen sich bevorzugte Formulierungen folgendermassen zusammen: (% = Gewichtsprozent).

| Emulgierbare Konzentrate: | |
|---|---|
| Aktiver Wirkstoff: | 1 bis 20 %, bevorzugt 5 bis 10 % |
| oberflächenaktive Mittel: | 5 bis 30 %, vorzugsweise 10 bis 20 % |
| flüssige Trägermittel: | 50 bis 94 %, vorzugsweise 70 bis 85 % |

| Stäube: | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

| Suspensions-Konzentrate: | |
|---|---|
| Aktiver Wirkstoff: | 5 bis 75 %, vorzugsweise 10 bis 50 % |
| Wasser: | 94 bis 25 %, vorzugsweise 88 bis 30 % |
| oberflächenaktives Mittel: | 1 bis 40 %, vorzugsweise 2 bis 30 % |

| Benetzbares Pulver: | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermittel: | 5 bis 95 %, vorzugsweise 15 bis 90 % |

| Granulate: | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 % |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,001 bis 4 kg AS/ha, vorzugsweise 0,005 bis 1 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachstehenden Beispiele erläutern die Erfindung.

Beispiel H 1.1: 4-Cyano-2-fluor-5-isopropyloxynitrobenzol

375 g 4-Brom-2-fluor-5-isopropoxy-nitrobenzol werden in 1 l Dimethyformamid vorgelegt. Unter Rühren gibt man 145 g Kuper(I)cyanid zu und heizt unter Argon langsam auf 155°C auf. Bei etwa 140°C Innentemperatur setzt eine leicht oxotherme Reaktion ein (die Innentemperatur steigt auf 153°C an und sinkt nach ca. 45 min langsam ab). Anschliessend wird das Reaktionsgemisch noch eine weitere h bei 155°C Badtemperatur gerührt. Man lässt auf Raumtemperatur abkühlen, dekantiert vom unlöslichen Bodensatz ab und dampft die Reaktionslösung am Wasserstrahlvakuum ein. Den braunen, öligen Rückstand versetzt man mit 1 l Essigester, rührt gut durch und dekantiert wieder. Die Essigesterlösung wird bis zur beginnenden Kristallisation eingeengt und in Eiswasser abgekühlt. Das ausgefallene Produkt wird abgenutscht und mit eiskaltem Essigester gewaschen und getrocknet. Mit der Mutterlauge wird nochmals wie oben verfahren.

Man isoliert 227 g der Titelverbindung der Formel

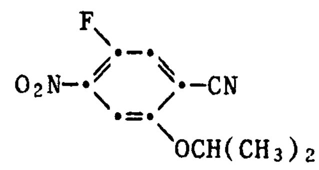

als beiges, flockiges Pulver mit einem Fp. von 116-118 °C.

Beispiel H 1.2: 4-Cyano-2-fluor-5-isopropyloxyanilin

160 g Zinn(II)chlorid•$2H_2O$ werden in 184 ml Salzsäure 37 %ig vorgelegt. In die farblose Lösung trägt man unter Rühren 44 g 4-Cyano-2-fluor-5-isopropoxynitrobenzol portionsweise ein, wobei eine orange gefärbte Suspension entsteht. Diese wird langsam zum Rückfluss erhitzt. Bei ca. 75 °C entsteht eine rote Lösung. Das Reaktionsgemisch wird noch 10 min bei 110 °C Badtemperatur ausgerührt, auf Raumtemperatur abgekühlt und dan auf ca. 1,5 l Eiswasser gegossen, mit conc. Natronlauge auf pH 9 gestellt. Die resultierende beige Suspension wird zweimal mit Essigester extrahiert. Die vereinigten Essigesterphasen werden über Magnesiumsulfat getrocknet und am Vakuum eingedampft und der Rückstand im Hochvakuum destilliert.

Man isoliert 32,2 g der Titelverbindung der Formel

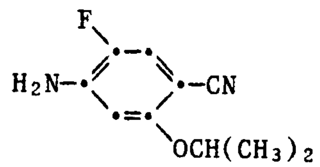

als hellgelbes Oel mit Kp 143-145 °C ($10^{-2}$ Torr)

Beispiel H 1.3: 2-(5-Carboxy-2-fluor-4-nitrophenyl)-4,5,6,7-tetrahydroisoindol-(2H)1,3-dion

95 g 2-(5-Carboxy-2-fluorphenyl)-4,5,6,7-tetrahydroisoindol-(2H)1,3-dion werden portionsweise bei 5 °C in 75 ml 96 %iger Schwefelsäure eingetragen. Dann wird zu diesem Gemisch unter starkem Rühren und bei der gleichen Temperatur 15 ml 100 %ige Salpetersäure zugetropft, 5 h bei Raumtemperatur weitergerührt und dann auf Eis gegossen. Der Niederschlag wird abgenutscht, mit kaltem Wasser gewaschen und getrocknet.

Man isoliert 98 g der Titelverbindung der Formel

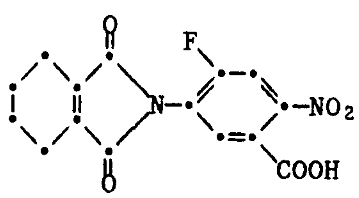

als Kristalle vom Fp. 225-227 °C.

Beispiel H 1.4: 2-(4-Amino-5-carboxy-2-fluorphenyl)-4,5,6,7-tetrahydroisoindol-(2H)1,3-dion

71 g 2-(5-Carboxy-2-fluor-4-nitrophenyl)-4,5,6,7-tetrahydroisoindol-(2H)1,3-dion werden in 720 ml Tetrahydrofuran bei einer Temperatur von 25-30 °C in Gegenwart von 14 g Raney-Nickel unter Normaldruck mit Wasserstoff hydriert. Nachdem die stöchiometrische Wasserstoffmenge verbraucht worden ist, wird der Katalysator abgetrennt und die Lösung eingedampft.

Man isoliert 54,6 g der Titelverbindung der Formel

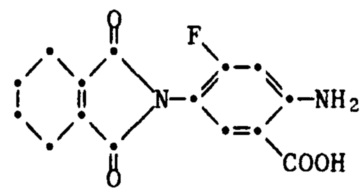

als Kristalle vom Fp 289 ° C.

Beispiel H 1.5: 2-(5-Carboxy-2-fluor-4-iodphenyl)-4,5,6,7-tetrahydroisoindol-(2H)1,3-dion

25 g 2-(4-Amino-5-carboxy-2-fluorphenyl)-4,5,6,7-tetrahydroisoindol-(2H)1,3-dion werden unter Rühren in 100 ml Eisessig und 100 ml 96 %ige Schwefelsäure portionsweise eingetragen, wobei die Temperatur bis auf 50 ° C steigt. Es wird bis zur vollständigen Lösung des Edukts weitergerührt. Anschliessend wird auf 5 ° C gekühlt und mit einer Lösung von 6,4 g Natriumnitrit in 40 ml Wasser diazotiert. Nach weiterem 6-stündigem Rühren bei Raumtemperatur wird das diazotierte Produkt in eine Lösung von 12,6 g Kaliumjodid in 80 ml Wasser getropft und dann noch 1 h bei 40 ° C weitergerührt. Das Reaktionsgemisch wird ins Eis/$H_2O$ gegossen, mit Essigester extrahiert, die organische Phase mit Natriumhydrogensulfitlösung gewaschen, getrocknet und eingedampft und aus Methanol umkristallisiert.

Man isoliert 25 g der Titelverbindung der Formel

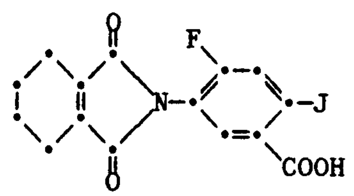

als Kristalle vom Fp. 205-207 ° C.

Beispiel H 2: 1-(4-Cyano-2-fluor-5-isopropyloxyphenyl)-3,4-dimethylpyrrol-(1H)2,5-dion

8,9 g 4-Cyano-2-fluor-5-isopropoxy-anilin und 6,3 g Dimethylmaleinsäureanhydrid werden in 50 ml Xylol in Gegenwart von 1 g 4-Dimethylamino-pyridin als Katalysator zum Rückfluss erhitzt. (Bei grösseren Ansätzen ist es vorteilhafter, die Reaktion in einer Wasserabscheidungsapparatur durchzuführen). Das Xylol wird am Wasserstrahlvakuum abdestilliert, der Rückstand in Essigester aufgenommen und auf Neutralkörper aufgearbeitet. Die organische Phase wird über Natriumsulfat getrocknet und das Lösungsmittel abdestilliert und der Rückstand aus Ethanol umkristallisiert.

Man isoliert 7,1 g der Titelverbindung der Formel

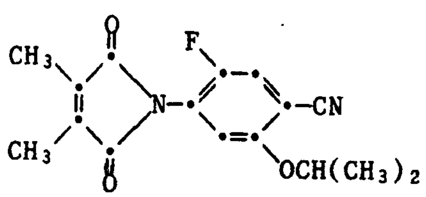

(Verb. No. 6.4)

als Kristalle vom Fp. 128-132 ° C.

Beispiel H 3: 2-(4-Cyano-2-fluor-5-isopropyloxyphenyl)-4,5,6,7-tetrahydroisoindol-(2H)1,3-dion

9,7 g 4-Cyano-2-fluor-5-isopropyloxy-anilin und 7,6 g 3,4,5,6-Tetrahydrophthalsäureanhydrid werden in 300 ml Propionsäure für insgesamt 30 h am Rückfluss erhitzt. Zur Vervollständigung der Reaktion gibt man nach 16 h nochmals 5 g Anhydrid zu. Das dunkel gefärbte Reaktionsgemisch lässt man 2 Tage bei Raumtemperatur stehen und nutscht dann den während dieser Zeit ausgefallenen gelben Niederschlag ab. Das so erhältliche Produkt wird aus Toluol umkristallisiert.

Man isoliert 6,7 g der Titelverbindung der Formel

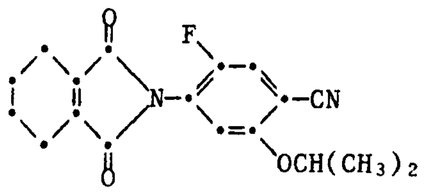

(Verb. No. 1.4)

als Kristalle vom Fp. 138-139 ° C, wobei die durch Umkristallisation aus Toluol erhältlichen Kristalle etwa 1/3 Mol Toloul im Kristall enthalten.

Beispiel H 4.1: 2-(5-Carboxy-4-cyano-2-fluorphenyl)-4,5,6,7-tetrahydroisoindol-(2H)1,3-dion

Eine Mischung aus 4,2 g 2-(5-Carboxy-2-fluor-4-iodphenyl)-4,5,6,7-tetrahydroisoindol-(2H)1,3-dion und 0,9 g Kupfer(I)cyanid werden in 50 ml Acetonitril für 12 h zum Rückfluss erhitzt. Nach dem Abkühlen wird von dem Ungelösten abgenutscht und das Filtrat zur Trockne eingeengt.

Man isoliert 2,7 g der Titelverbindung der Formel

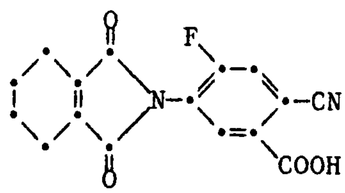

(Verb. No. 2.1)

als Kristalle vom Fp. 203-204 ° C.

Beispiel H 4.2: 2-(5-Chlorcarboxy-4-cyano-2-fluorphenyl)-4,5,6,7-tetrahydroisoindol-(2H)1,3-dion

6 g 2-(5-Carboxy-4-cyano-2-fluorphenyl)-4,5,6,7-tetrahydroisoindol-(2H)1,3-dion, 60 ml Thionylchlorid und 5 Tropfen Dimethylformamid werden für 1 h zum Rückfluss erhitzt. Nach dem Abkühlen wird im Vakuum eingedampft.

Man isoliert 6 g der Titelverbindung der Formel

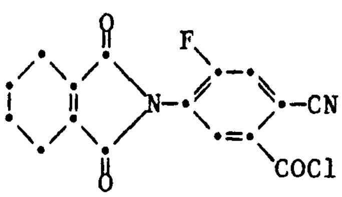

als dickflüssiges Oel, welches ohne wietere Reinigung für Folgereaktionen verwendet werden kann.

Beispiel H 4.3: 2-(4-Cyano-2-fluor-5-isopropyloxycarbonylphenyl)-4,5,6,7-tetrahydroisoindol-(2H)1,3-dion

Zu einer Mischung von 5 ml Isopropanol, 5 ml Triethylamin und 50 ml Toluol werden bei Raumtemperatur 7,1 g 2-(5-Chlorcarbonyl-4-cyano-2-fluorphenyl)-4,5,6,7-tetrahydroisoindol-(2H)1,3-dion gelöst in 50 ml Toluol, getropft, wobei die Temperatur auf 45 °C steigt. Nach 1-stündigem Rühren bei Raumtemperatur wird das Triethylaminhydrochlorid abgetrennt und das Filtrat eingedampft.

Man isoliert 5 g der Titelverbindung der Formel

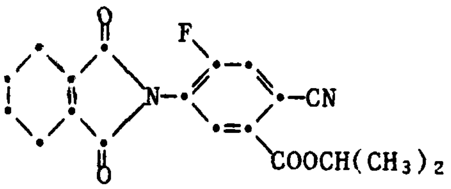

(Verb. No. 2.5)

als Kristalle vom Fp. 173-176 °C.

Beispiel H 5.1: 2-(5-Chlorcarbonyl-2-fluor-4-iodphenyl)-4,5,6,7-tetrahydroisoindol-(2H)1,3-dion

Ein Gemisch aus 16,4 g 2-(5-Carboxy-2-fluor-4-iodphenyl)-4,5,6,7-tetrahydroisoindol-(2H)1,3-dion, 6 ml Thionylchlorid und 200 ml Toluol werden für 12 h zum Rückfluss erhitzt. Nach dem Abkühlen wird im Vakuum eingedampft.

Man isoliert 17,6 g der Titelverbindung der Formel

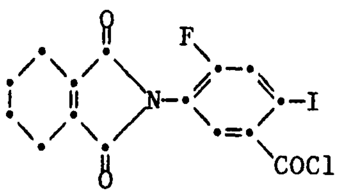

als Kristalle vom Fp. 114-117 °C.

Beispiel H 5.2: 2-(2-Fluor-4-iod-5-isopropyloxycarbonylphenyl)-4,5,6,7-tetrahydroisoindol-(2H)1,3-dion

Zu einer Mischung von 8 ml Isopropanol, 12 ml Triethylamin und 120 ml Toluol werden bei Raumtemperatur 17,1 g 2-(5-Chlorcarbonyl-2-fluor4-iodphenyl)-4,5,6,7-tetrahydroisoindol-(2H)1,3-dion getropft. Nach einer Reaktionszeit von 12 h wird von dem ausgefallenen Triethylaminhydrochlorid abgetrennt und das Filtrat eingedampft.

Man isoliert 7,2 der Titelverbindung der Formel

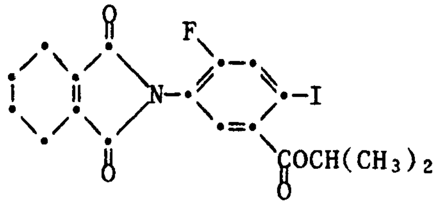

als Kristalle vom Fp. 127-132 °C.

Beispiel H 5.3: 2-(4-Cyano-2-fluor-5-isopropyloxycarbonyl)-4,5,6,7-tetrahydroisoindol-(2H)1,3-dion

Eine Mischung aus 2,3 g 2-(2-Fluor-4-iod-5-isopropyloxycarbonylphenyl)-4,5,6,7-tetrahydroisoindol-(2H)-1,3-dion und 0,5 g Kupfer(I)cyanid werden in 20 ml Dimethylformamid für 1 h unter Rühren auf 100 °C

erwärmt. Nach dem Erkalten wird von dem Rückstand abgenutscht und das Filtrat zur Trockne eingeengt.

Man isoliert 1,4 g der Titelverbindung der Formel

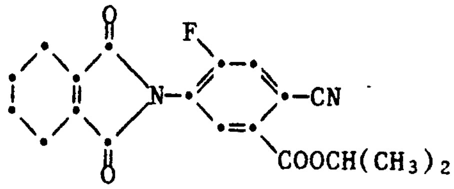

(Verb. No. 2.5)

als Kristalle vom Fp. 173-176 °C.

Analog zu vorstehenden Beispielen H 2 bis H 5 können die Verbindungen der Tabellen 1 bis 11 hergestellt werden.

Tabelle 1

Verbindungen der Formel

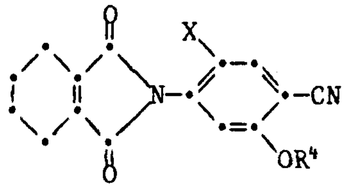

| Nr. | X | $R^4$ | Phys. Daten |
|---|---|---|---|
| 1.1 | F | $CH_3$ | |
| 1.2 | F | $C_2H_5$ | |
| 1.3 | F | $C_3H_7$ | |
| 1.4 | F | $C_3H_7$(i) | Fp:138-139°C * |
| 1.5 | F | $C_4H_9$ | |
| 1.6 | F | $C_4H_9$(s) | |
| 1.7 | F | $C_4H_9$(i) | |
| 1.8 | F | $C_4H_9$(t) | |
| 1.9 | F | $-C_5H_{11}$ | |
| 1.10 | F | $-C_5H_{11}$(s) | |
| 1.11 | F | $-CH_2-CH_2-OCH_3$ | |
| 1.12 | F | $-CH_2-CH=CH_2$ | |
| 1.13 | F | $-CH_2-CCl=CH_2$ | |
| 1.14 | F | $-CH_2-CH=CHCl$ | |
| 1.15 | F | $-CH_2-C\equiv CH$ | Fp: (196°) 200-202° |
| 1.16 | F | $-CH_2-C_6H_{11}$-(cycl.) | |

* Kristalle enthalten etwa 1/3 Mol Toluol.

Tabelle 1 (Fortsetzung)

| Nr. | X | $R^4$ | Phys. Daten |
|---|---|---|---|
| 1.17 | F | 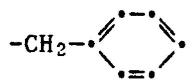 | |
| 1.18 | F | $-CH_2-COOCH_3$ | |
| 1.19 | F | $-CH(CH_3)COOCH_3$ | |
| 1.20 | F | $-CH(CH_3)COOC_3H_7(i)$ | |
| 1.21 | F | $-CH(CH_3)COOC_4H_9(n)$ | |
| 1.22 | F | $-CH(CH_3)COSCH_3$ | |
| 1.23 | F | $-CH(CH_3)CONH_2$ | |
| 1.24 | F | 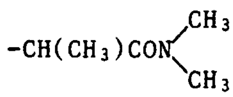 | |
| 1.25 | F | $-COCH_3$ | |
| 1.26 | F | $-COC_6H_5$ | |
| 1.27 | F | $-CH(CH_3)-CH_2-S-CH_3$ | |
| 1.28 | F | 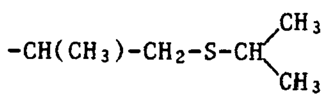 | |
| 1.29 | F | $-CH_2-CH_2-OH$ | |
| 1.30 | F | $-CH_2-CH_2-Cl$ | |
| 1.31 | F | $-CH_2-CN$ | |
| 1.32 | F | 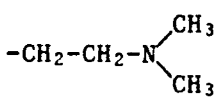 | |
| 1.33 | F | 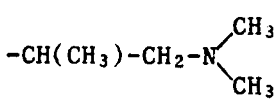 | |
| 1.34 | H | $-CH_3$ | |
| 1.35 | H | $-C_2H_5$ | |
| 1.36 | H | $-C_3H_7$ | |
| 1.37 | H | $-C_3H_7(i)$ | |
| 1.38 | H | $-C_4H_9(i)$ | |
| 1.39 | H | $-C_4H_9(s)$ | |
| 1.40 | H | $-CH_2-CH=CH_2$ | |

Tabelle 1 (Fortsetzung)

| Nr. | X | $R^4$ | Phys. Daten |
|---|---|---|---|
| 1.41 | H | $-CH_2-CCl=CH_2$ | |
| 1.42 | H | $-CH_2-CH=CHCl$ | |
| 1.43 | H | $-CH_2-C\equiv CH$ | |
| 1.44 | H | $-CH_2-COOCH_3$ | |
| 1.45 | H | $-CH(CH_3)COOCH_3$ | |
| 1.46 | H | $-CH(CH_3)-CH_2-S-CH_3$ | |
| 1.47 | H | $-CH(CH_3)-CH_2-S-C_2H_5$ | |
| 1.48 | H | $-CH(CH_3)-CH_2-S-C_3H_7(i)$ | |

Tabelle 2

Verbindungen der Formel

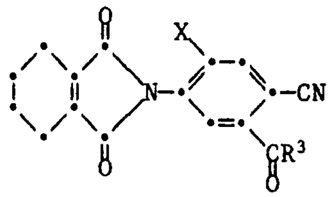

| Nr. | X | $R^3$ | Phys. Daten |
|---|---|---|---|
| 2.1 | F | OH | Fp: 203-204°C |
| 2.2 | F | $OCH_3$ | Fp: 136-141°C |
| 2.3 | F | $OC_2H_5$ | Fp: 155-157°C |
| 2.4 | F | $OC_3H_7$ | Fp: 86-90°C |
| 2.5 | F | $OC_3H_7$(i) | Fp: 173-176°C |
| 2.6 | F | $OC_4H_9$(n) | |
| 2.7. | F | $OC_4H_9$(i) | |
| 2.8 | F | $OC_4H_9$(s) | Fp: 80-82°C |
| 2.9 | F | $OC_4H_9$(t) | |
| 2.10 | F | $OC_5H_{11}$ | |
| 2.11 | F | $OC_5H_{11}$(s) | |
| 2.12 | F | $O-CH_2-CH_2-Cl$ | |
| 2.13 | F | $O-CH_2-CH_2-OCH_3$ | |
| 2.14 | F | $O-CH_2-CH_2-O-C_2H_5$ | |
| 2.15 | F | $O-CH_2-CH_2-S-CH_3$ | |
| 2.16 | F | $O-CH_2(CH_3)-CH_2-S-CH_3$ | |
| 2.17 | F | $O-CH_2(CH_3)-CH_2-S-C_2H_5$ | |
| 2.18 | F | $O-CH_2(CH_3)-CH_2-S-C_3H_7$(i) | |
| 2.19 | F | $O-CH(CH_3)-CH_2-S-C_4H_9$(n) | |
| 2.20 | F | $O-CH_2(CH_3)-CH_2-S-C_4H_9$ (i) | |
| 2.21 | F | $-O-CH_2-CH=CH_2$ | |
| 2.22 | F | $-O-CH_2-CCl=CH_2$ | |
| 2.23 | F | $-O-CH_2-CH=CHCl$ | |

Tabelle 2 (Fortsetzung)

| Nr. | X | $R^3$ | Phys. Daten |
|---|---|---|---|
| 2.24 | F | $-OCH_2-C{\equiv}CH$ | |
| 2.25 | F | $-O-CH_2-C_6H_{11}$-(cycl.) | |
| 2.26 | F | $-O-CH_2-C_3H_5$-(cycl.) | |
| 2.27 | F | $-O-CH_2-C_6H_5$ | |
| 2.28 | F | $-O-CH_2-COOCH_3$ | |
| 2.29 | F | $-O-CH(CH_3)COOCH_3$ | |
| 2.30 | F | $-O-CH(CH_3)COOC_2H_5$ | |
| 2.31 | F | $-O-CH(CH_3)COOC_3H_7i$ | |
| 2.32 | F | $-O-CH(CH_3)COOC_4H_9(n)$ | |
| 2.33 | F | $-O-CH(CH_3)COSCH_3$ | |
| 2.34 | F | $-O-CH(CH_3)CON(CH_3)_2$ | |
| 2.35 | F | $-S-CH_2-COOCH_3$ | |
| 2.36 | F | $-S-CH_2-COOC_2H_5$ | |
| 2.37 | F | $-S-CH(CH_3)-COOCH_3$ | |
| 2.38 | F | $-S-CH(CH_3)-COOC_2H_5$ | |
| 2.39 | F | $-S-CH(CH_3)-COOC_3H_7$ | |
| 2.40 | F | $-S-CH(CH_3)-COOC_3H_7(i)$ | |
| 2.41 | F | $-SCH_3$ | |
| 2.42 | F | $-N(CH_3)_2$ | |
| 2.43 | F | $-N(CH_2-CH{=}CH_2)_2$ | |
| 2.44 | F | 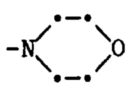 | |
| 2.45 | F | 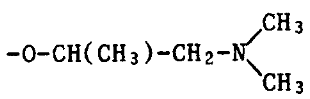 | |
| 2.46 | H | -OH | |
| 2.47 | H | $-OCH_3$ | Fp: (161°) 164-166° |
| 2.48 | H | $-OC_2H_5$ | |
| 2.49 | H | $OC_3H_7(n)$ | |
| 2.50 | H | $OC_3H_7(i)$ | |
| 2.51 | H | $OC_4H_9(i)$ | |
| 2.52 | H | $OC_4H_9(s)$ | |

Tabelle 2 (Fortsetzung)

| Nr. | X | $R^3$ | Phys. Daten |
|---|---|---|---|
| 2.53 | H | $OCH_2-CH=CH_2$ | |
| 2.54 | H | $OCH_2-CCl=CH_2$ | |
| 2.55 | H | $OCH_2-CH=CHCl$ | |
| 2.56 | H | $-OCH_2-C\equiv CH$ | |
| 2.57 | H | $-OCH_2-COOCH_3$ | |
| 2.58 | H | $-OCH(CH_3)COOCH_3$ | |
| 2.59 | H | $-OCH(CH_3)CH_2-S-CH_3$ | |
| 2.60 | H | $-OCH(CH_3)CH_2-S-C_2H_5$ | |
| 2.61 | H | $-OCH(CH_3)CH_2-S-C_3H_7i$ | |
| 2.62 | H | $-S-CH_2-COOCH_3$ | |
| 2.63 | H | $-S-CH_2-COOC_2H_5$ | |
| 2.64 | H | $-S-CH(CH_3)COOCH_3$ | |
| 2.65 | H | $-S-CH(CH_3)COOC_2H_5$ | |
| 2.66 | H | $-S-CH(CH_3)-COO-C_3H_7i$ | |

Tabelle 3

Verbindungen der Formel

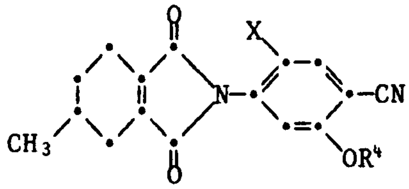

| Nr. | X | $R^4$ | Phys. Daten |
|---|---|---|---|
| 3.1 | F | $CH_3$ | |
| 3.2 | F | $C_3H_7i$ | Fp: 147-149°C |
| 3.3 | F | $CH_2-CH=CH_2$ | |
| 3.4 | F | $CH_2-CCl=CH_2$ | |
| 3.5 | F | $CH_2-CH=CHCl$ | |
| 3.6 | F | $CH_2-C\equiv CH$ | |
| 3.7 | F | $-CH_2-COOCH_3$ | |
| 3.8 | F | $-CH(CH_3)-COOCH_3$ | |

Tabelle 4

Verbindungen der Formel

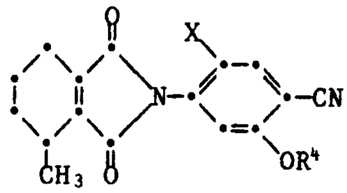

| Nr. | X | $R^4$ | Phys. Daten |
|---|---|---|---|
| 4.1 | F | $CH_3$ | |
| 4.2 | F | $C_3H_7i$ | |
| 4.3 | F | $-CH_2-CH=CH_2$ | |
| 4.4 | F | $-CH_2-CCl=CH_2$ | |
| 4.5 | F | $-CH_2-CH=CHCl$ | |
| 4.6 | F | $-CH_2-C\equiv CH$ | |
| 4.7 | F | $-CH_2-COOCH_3$ | |
| 4.8 | F | $-CH_2(CH_3)COOCH_3$ | |

Tabelle 5

Verbindungen der Formel

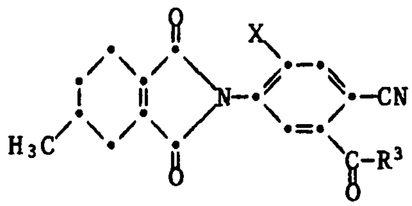

| Nr. | X | $R^3$ | Phys. Daten |
|---|---|---|---|
| 5.1 | F | $OCH_3$ | |
| 5.2 | F | $OC_2H_5$ | |
| 5.3 | F | $OC_3H_7(i)$ | |
| 5.4 | F | $OC_4H_9(i)$ | |
| 5.5 | F | $OCH_2-CH_2-Cl$ | |
| 5.6 | F | $OCH_2-COOCH_3$ | |
| 5.7 | F | $OCH_2-COOC_2H_5$ | |
| 5.8 | F | $OCH(CH_3)-COOCH_3$ | |
| 5.9 | F | $S-CH_2-COOCH_3$ | |
| 5.10 | F | $-S-CH_2-COOC_2H_5$ | |
| 5.11 | F | $-S-CH(CH_3)COOCH_3$ | |
| 5.12 | F | $-S-CH(CH_3)COOC_3H_7(i)$ | |
| 5.13 | F | $-O-CH(CH_3)-CH_2-S-CH_3$ | |

Tabelle 6

Verbindungen der Formel

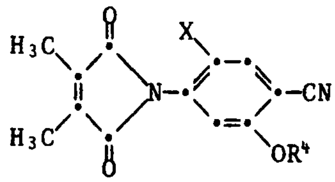

| Nr. | X | $R^4$ | Phys. Daten |
|---|---|---|---|
| 6.1 | F | $CH_3$ | |
| 6.2 | F | $C_2H_5$ | |
| 6.3 | F | $C_3H_7$ | |
| 6.4 | F | $C_3H_7$(i) | Fp:128-132°C |
| 6.5 | F | $C_4H_9$ | |
| 6.6 | F | $C_4H_9$(s) | |
| 6.7 | F | $C_4H_9$(i) | |
| 6.8 | F | $C_4H_9$(t) | |
| 6.9 | F | $-C_5H_{11}$ | |
| 6.10 | F | $-C_5H_{11}$(s) | |
| 6.11 | F | $-CH_2-CH_2-OCH_3$ | |
| 6.12 | F | $-CH_2-CH=CH_2$ | |
| 6.13 | F | $-CH_2-CCl=CH_2$ | |
| 6.14 | F | $-CH_2-CH=CHCl$ | |
| 6.15 | F | $-CH_2-C\equiv CH$ | |
| 6.16 | F | $-CH_2-C_6H_{11}$-(cycl.) | |
| 6.17 | F | $-CH_2-$ 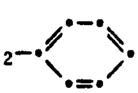 | |
| 6.18 | F | $-CH_2-COOCH_3$ | |
| 6.19 | F | $-CH(CH_3)COOCH_3$ | |
| 6.20 | F | $-CH(CH_3)COOC_3H_7$(i) | |
| 6.21 | F | $-CH(CH_3)COOC_4H_9$(n) | |
| 6.22 | F | $-CH(CH_3)COSCH_3$ | |

Tabelle 6 (Fortsetzung)

| Nr. | X | $R^4$ | Phys. Daten |
|---|---|---|---|
| 6.23 | F | $-CH(CH_3)CONH_2$ | |
| 6.24 | F | 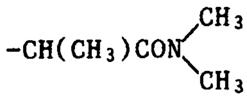 | |
| 6.25 | F | $-COCH_3$ | |
| 6.26 | F | $-COC_6H_5$ | |
| 6.27 | F | $-CH(CH_3)-CH_2-S-CH_3$ | |
| 6.28 | F | 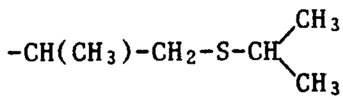 | |
| 6.29 | F | $-CH_2-CH_2-OH$ | |
| 6.30 | F | $-CH_2-CH_2-Cl$ | |
| 6.31 | F | $-CH_2-CN$ | |
| 6.32 | F | 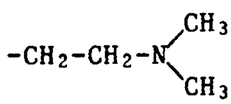 | |
| 6.33 | F | 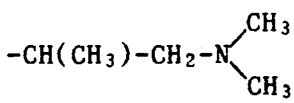 | |
| 6.34 | H | $-CH_3$ | |
| 6.35 | H | $-C_2H_5$ | |
| 6.36 | H | $-C_3H_7$ | |
| 6.37 | H | $-C_3H_7(i)$ | |
| 6.38 | H | $-C_4H_9(i)$ | |
| 6.39 | H | $-C_4H_9(s)$ | |
| 6.40 | H | $-CH_2-CH=CH_2$ | |
| 6.41 | H | $-CH_2-CCl=CH_2$ | |
| 6.42 | H | $-CH_2-CH=CHCl$ | |
| 6.43 | H | $-CH_2-C\equiv CH$ | |
| 6.44 | H | $-CH_2-COOCH_3$ | |
| 6.45 | H | $-CH(CH_3)COOCH_3$ | |
| 6.46 | H | $-CH(CH_3)-CH_2-S-CH_3$ | |
| 6.47 | H | $-CH(CH_3)-CH_2-S-C_2H_5$ | |

Tabelle 7

Verbindungen der Formel

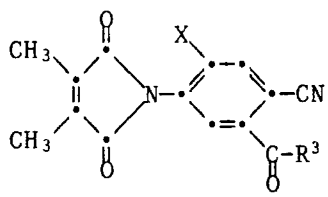

| Nr. | X | $R^3$ | Phys. Daten |
|---|---|---|---|
| 7.1 | F | OH | |
| 7.2 | F | $OCH_3$ | |
| 7.3 | F | $OC_2H_5$ | |
| 7.4 | F | $OC_3H_7$ | |
| 7.5 | F | $OC_3H_7(i)$ | |
| 7.6 | F | $OC_4H_9(n)$ | |
| 7.7. | F | $OC_4H_9(i)$ | |
| 7.8 | F | $OC_4H_9(s)$ | |
| 7.9 | F | $OC_4H_9(t)$ | |
| 7.10 | F | $OC_5H_{11}$ | |
| 7.11 | F | $OC_5H_{11}(s)$ | |
| 7.12 | F | $O-CH_2-CH_2-Cl$ | |
| 7.13 | F | $O-CH_2-CH_2-OCH_3$ | |
| 7.14 | F | $O-CH_2-CH_2-O-C_2H_5$ | |
| 7.15 | F | $O-CH_2-CH_2-S-CH_3$ | |
| 7.16 | F | $O-CH_2(CH_3)-CH_2-S-CH_3$ | |
| 7.17 | F | $O-CH_2(CH_3)-CH_2-S-C_2H_5$ | |
| 7.18 | F | $O-CH_2(CH_3)-CH_2-S-C_3H_7(i)$ | |
| 7.19 | F | $O-CH(CH_3)-CH_2-S-C_4H_9(n)$ | |
| 7.20 | F | $O-CH_2(CH_3)-CH_2-S-C_4H_9$ (i) | |
| 7.21 | F | $-O-CH_2-CH=CH_2$ | |
| 7.22 | F | $-O-CH_2-CCl=CH_2$ | |
| 7.23 | F | $-O-CH_2-CH=CHCl$ | |

Tabelle 7 (Fortsetzung)

| Nr. | X | $R^3$ | Phys. Daten |
|---|---|---|---|
| 7.24 | F | $-OCH_2-C\equiv CH$ | |
| 7.25 | F | $-O-CH_2-C_6H_{11}$-(cycl.) | |
| 7.26 | F | $-O-CH_2-C_3H_5$-(cycl.) | |
| 7.27 | F | $-O-CH_2-C_6H_5$ | |
| 7.28 | F | $-O-CH_2-COOCH_3$ | |
| 7.29 | F | $-O-CH(CH_3)COOCH_3$ | |
| 7.30 | F | $-O-CH(CH_3)COOC_2H_5$ | |
| 7.31 | F | $-O-CH(CH_3)COOC_3H_7$(i) | |
| 7.32 | F | $-O-CH(CH_3)COOC_4H_9$(n) | |
| 7.33 | F | $-O-CH(CH_3)COSCH_3$ | |
| 7.34 | F | $-O-CH(CH_3)CON(CH_3)_2$ | |
| 7.35 | F | $-S-CH_2-COOCH_3$ | |
| 7.36 | F | $-S-CH_2-COOC_2H_5$ | |
| 7.37 | F | $-S-CH(CH_3)-COOCH_3$ | |
| 7.38 | F | $-S-CH(CH_3)-COOC_2H_5$ | |
| 7.39 | F | $-S-CH(CH_3)-COOC_3H_7$ | |
| 7.40 | F | $-S-CH(CH_3)-COOC_3H_7$(i) | |
| 7.41 | F | $-SCH_3$ | |
| 7.42 | F | $-N(CH_3)_2$ | |
| 7.43 | F | $-N(CH_2-CH=CH_2)_2$ | |
| 7.44 | F | 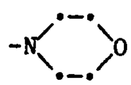 | |
| 7.45 | F | 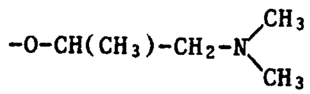 | |
| 7.46 | H | -OH | |
| 7.47 | H | $-OCH_3$ | |
| 7.48 | H | $-OC_2H_5$ | |
| 7.49 | H | $OC_3H_7$(n) | |
| 7.50 | H | $OC_3H_7$(i) | |
| 7.51 | H | $OC_4H_9$(i) | |
| 7.52 | H | $OC_4H_9$(s) | |

Tabelle 7 (Fortsetzung)

| Nr. | X | $R^3$ | Phys. Daten |
|---|---|---|---|
| 7.53 | H | $OCH_2-CH=CH_2$ | |
| 7.54 | H | $OCH_2-CCl=CH_2$ | |
| 7.55 | H | $OCH_2-CH=CHCl$ | |
| 7.56 | H | $-OCH_2-C\equiv CH$ | |
| 7.57 | H | $-OCH_2-COOCH_3$ | |
| 7.58 | H | $-OCH(CH_3)COOCH_3$ | |
| 7.59 | H | $-OCH(CH_3)CH_2-S-CH_3$ | |
| 7.60 | H | $-OCH(CH_3)CH_2-S-C_2H_5$ | |
| 7.61 | H | $-OCH(CH_3)CH_2-S-C_3H_7(i)$ | |
| 7.62 | H | $-S-CH_2-COOCH_3$ | |
| 7.63 | H | $-S-CH_2-COOC_2H_5$ | |
| 7.64 | H | $-S-CH(CH_3)COOCH_3$ | |
| 7.65 | H | $-S-CH(CH_3)COOC_2H_5$ | |
| 7.66 | H | $-S-CH(CH_3)-COO-C_3H_7(i)$ | |

Tabelle 8

Verbindungen der Formel

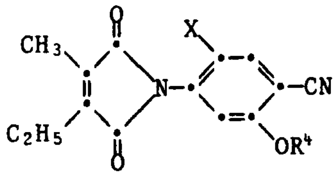

| Nr. | X | $R^4$ | Phys. Daten |
|---|---|---|---|
| 8.1 | F | $CH_3$ | |
| 8.2 | F | $C_2H_5$ | |
| 8.3 | F | $C_3H_7$ | |
| 8.4 | F | $C_3H_7(i)$ | Fp: 98-100°C |
| 8.5 | F | $C_4H_9$ | |
| 8.6 | F | $C_4H_9(s)$ | |
| 8.7 | F | $C_4H_9(i)$ | |
| 8.8 | F | $C_4H_9(t)$ | |
| 8.9 | F | $-C_5H_{11}$ | |
| 8.10 | F | $-C_5H_{11}(s)$ | |
| 8.11 | F | $-CH_2-CH_2-OCH_3$ | |
| 8.12 | F | $-CH_2-CH=CH_2$ | |
| 8.13 | F | $-CH_2-CCl=CH_2$ | |
| 8.14 | F | $-CH_2-CH=CHCl$ | |
| 8.15 | F | $-CH_2-C\equiv CH$ | |
| 8.16 | F | $-CH_2-C_6H_{11}-(cycl.)$ | |
| 8.17 | F | $-CH_2-$ 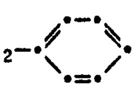 | |
| 8.18 | F | $-CH_2-COOCH_3$ | |
| 8.19 | F | $-CH(CH_3)COOCH_3$ | |
| 8.20 | F | $-CH(CH_3)COOC_3H_7(i)$ | |
| 8.21 | F | $-CH(CH_3)COOC_4H_9(n)$ | |
| 8.22 | F | $-CH(CH_3)COSCH_3$ | |

Tabelle 8 (Fortsetzung)

| Nr. | X | $R^4$ | Phys. Daten |
|---|---|---|---|
| 8.23 | F | $-CH(CH_3)CONH_2$ | |
| 8.24 | F | 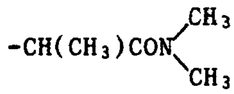 | |
| 8.25 | F | $-COCH_3$ | |
| 8.26 | F | $-COC_6H_5$ | |
| 8.27 | F | $-CH(CH_3)-CH_2-S-CH_3$ | |
| 8.28 | F | 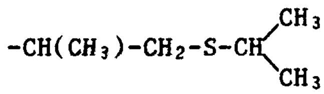 | |
| 8.29 | F | $-CH_2-CH_2-OH$ | |
| 8.30 | F | $-CH_2-CH_2-Cl$ | |
| 8.31 | F | $-CH_2-CN$ | |
| 8.32 | F | 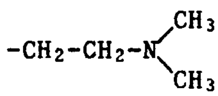 | |
| 8.33 | F | 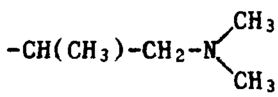 | |
| 8.34 | H | $-CH_3$ | |
| 8.35 | H | $-C_2H_5$ | |
| 8.36 | H | $-C_3H_7$ | |
| 8.37 | H | $-C_3H_7(i)$ | |
| 8.38 | H | $-C_4H_9(i)$ | |
| 8.39 | H | $-C_4H_9(s)$ | |
| 8.40 | H | $-CH_2-CH=CH_2$ | |
| 8.41 | H | $-CH_2-CCl=CH_2$ | |
| 8.42 | H | $-CH_2-CH=CHCl$ | |
| 8.43 | H | $-CH_2-C\equiv CH$ | |
| 8.44 | H | $-CH_2-COOCH_3$ | |
| 8.45 | H | $-CH(CH_3)COOCH_3$ | |
| 8.46 | H | $-CH(CH_3)-CH_2-S-CH_3$ | |
| 8.47 | H | $-CH(CH_3)-CH_2-S-C_2H_5$ | |
| 8.48 | H | $-CH(CH_3)-CH_2-S-C_3H_7(i)$ | |

Tabelle 9

Verbindungen der Formel

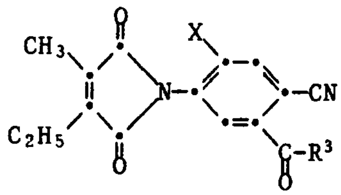

| Nr. | X | $R^3$ | Phys. Daten |
|---|---|---|---|
| 9.1 | F | OH | |
| 9.2 | F | $OCH_3$ | |
| 9.3 | F | $OC_2H_5$ | |
| 9.4 | F | $OC_3H_7$ | |
| 9.5 | F | $OC_3H_7$(i) | |
| 9.6 | F | $OC_4H_9$(n) | |
| 9.7. | F | $OC_4H_9$(i) | |
| 9.8 | F | $OC_4H_9$(s) | |
| 9.9 | F | $OC_4H_9$(t) | |
| 9.10 | F | $OC_5H_{11}$ | |
| 9.11 | F | $OC_5H_{11}$(s) | |
| 9.12 | F | $O-CH_2-CH_2-Cl$ | |
| 9.13 | F | $O-CH_2-CH_2-OCH_3$ | |
| 9.14 | F | $O-CH_2-CH_2-O-C_2H_5$ | |
| 9.15 | F | $O-CH_2-CH_2-S-CH_3$ | |
| 9.16 | F | $O-CH_2(CH_3)-CH_2-S-CH_3$ | |
| 9.17 | F | $O-CH_2(CH_3)-CH_2-S-C_2H_5$ | |
| 9.18 | F | $O-CH_2(CH_3)-CH_2-S-C_3H_7$(i) | |
| 9.19 | F | $O-CH(CH_3)-CH_2-S-C_4H_9$(n) | |
| 9.20 | F | $O-CH_2(CH_3)-CH_2-S-C_4H_9$ (i) | |
| 9.21 | F | $-O-CH_2-CH=CH_2$ | |
| 9.22 | F | $-O-CH_2-CCl=CH_2$ | |
| 9.23 | F | $-O-CH_2-CH=CHCl$ | |

Tabelle 9 (Fortsetzung)

| Nr. | X | $R^3$ | Phys. Daten |
|---|---|---|---|
| 9.24 | F | $-OCH_2-C\equiv CH$ | |
| 9.25 | F | $-O-CH_2-C_6H_{11}$-(cycl.) | |
| 9.26 | F | $-O-CH_2-C_3H_5$-(cycl.) | |
| 9.27 | F | $-O-CH_2-C_6H_5$ | |
| 9.28 | F | $-O-CH_2-COOCH_3$ | |
| 9.29 | F | $-O-CH(CH_3)COOCH_3$ | |
| 9.30 | F | $-O-CH(CH_3)COOC_2H_5$ | |
| 9.31 | F | $-O-CH(CH_3)COOC_3H_7$(i) | |
| 9.32 | F | $-O-CH(CH_3)COOC_4H_9$(n) | |
| 9.33 | F | $-O-CH(CH_3)COSCH_3$ | |
| 9.34 | F | $-O-CH(CH_3)CON(CH_3)_2$ | |
| 9.35 | F | $-S-CH_2-COOCH_3$ | |
| 9.36 | F | $-S-CH_2-COOC_2H_5$ | |
| 9.37 | F | $-S-CH(CH_3)-COOCH_3$ | |
| 9.38 | F | $-S-CH(CH_3)-COOC_2H_5$ | |
| 9.39 | F | $-S-CH(CH_3)-COOC_3H_7$ | |
| 9.40 | F | $-S-CH(CH_3)-COOC_3H_7$(i) | |
| 9.41 | F | $-SCH_3$ | |
| 9.42 | F | $-N(CH_3)_2$ | |
| 9.43 | F | $-N(CH_2-CH=CH_2)_2$ | |
| 9.44 | F | 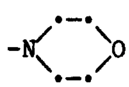 | |
| 9.45 | F | 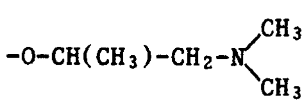 | |
| 9.46 | H | -OH | |
| 9.47 | H | $-OCH_3$ | |
| 9.48 | H | $-OC_2H_5$ | |
| 9.49 | H | $OC_3H_7$(n) | |
| 9.50 | H | $OC_3H_7$(i) | |
| 9.51 | H | $OC_4H_9$(i) | |
| 9.52 | H | $OC_4H_9$(s) | |

Tabelle 9 (Fortsetzung)

| Nr. | X | $R^3$ | Phys. Daten |
|---|---|---|---|
| 9.53 | H | $OCH_2-CH=CH_2$ | |
| 9.54 | H | $OCH_2-CCl=CH_2$ | |
| 9.55 | H | $OCH_2-CH=CHCl$ | |
| 9.56 | H | $-OCH_2-C\equiv CH$ | |
| 9.57 | H | $-OCH_2-COOCH_3$ | |
| 9.58 | H | $-OCH(CH_3)COOCH_3$ | |
| 9.59 | H | $-OCH(CH_3)CH_2-S-CH_3$ | |
| 9.60 | H | $-OCH(CH_3)CH_2-S-C_2H_5$ | |
| 9.61 | H | $-OCH(CH_3)CH_2-S-C_3H_7(i)$ | |
| 9.62 | H | $-S-CH_2-COOCH_3$ | |
| 9.63 | H | $-S-CH_2-COOC_2H_5$ | |
| 9.64 | H | $-S-CH(CH_3)COOCH_3$ | |
| 9.65 | H | $-S-CH(CH_3)COOC_2H_5$ | |
| 9.66 | H | $-S-CH(CH_3)-COO-C_3H_7(i)$ | |

Tabelle 10

Verbindungen der Formel

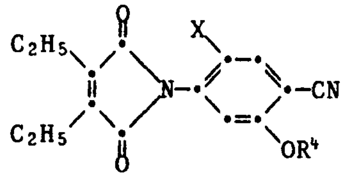

| Nr. | X | $R^4$ | Phys. Daten |
|---|---|---|---|
| 10.1 | F | $CH_3$ | |
| 10.2 | F | $C_2H_5$ | |
| 10.3 | F | $C_3H_7$ | |
| 10.4 | F | $C_3H_7(i)$ | |
| 10.5 | F | $C_4H_9$ | |
| 10.6 | F | $C_4H_9(s)$ | |
| 10.7 | F | $C_4H_9(i)$ | |
| 10.8 | F | $C_4H_9(t)$ | |
| 10.9 | F | $-C_5H_{11}$ | |
| 10.10 | F | $-C_5H_{11}(s)$ | |
| 10.11 | F | $-CH_2-CH_2-OCH_3$ | |
| 10.12 | F | $-CH_2-CH=CH_2$ | |
| 10.13 | F | $-CH_2-CCl=CH_2$ | |
| 10.14 | F | $-CH_2-CH=CHCl$ | |
| 10.15 | F | $-CH_2-C\equiv CH$ | |
| 10.16 | F | $-CH_2-C_6H_{11}$-(cycl.) | |
| 10.17 | F | $-CH_2-$ 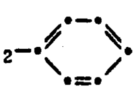 | |
| 10.18 | F | $-CH_2-COOCH_3$ | |
| 10.19 | F | $-CH(CH_3)COOCH_3$ | |
| 10.20 | F | $-CH(CH_3)COOC_3H_7(i)$ | |
| 10.21 | F | $-CH(CH_3)COOC_4H_9(n)$ | |
| 10.22 | F | $-CH(CH_3)COSCH_3$ | |

Tabelle 10 (Fortsetzung)

| Nr. | X | $R^4$ | Phys. Daten |
|---|---|---|---|
| 10.23 | F | $-CH(CH_3)CONH_2$ | |
| 10.24 | F | 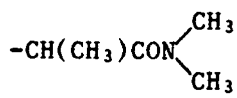 | |
| 10.25 | F | $-COCH_3$ | |
| 10.26 | F | $-COC_6H_5$ | |
| 10.27 | F | $-CH(CH_3)-CH_2-S-CH_3$ | |
| 10.28 | F | 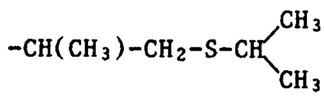 | |
| 10.29 | F | $-CH_2-CH_2-OH$ | |
| 10.30 | F | $-CH_2-CH_2-Cl$ | |
| 10.31 | F | $-CH_2-CN$ | |
| 10.32 | F | 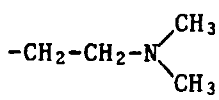 | |
| 10.33 | F | 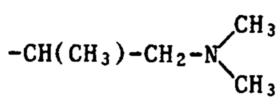 | |
| 10.34 | H | $-CH_3$ | |
| 10.35 | H | $-C_2H_5$ | |
| 10.36 | H | $-C_3H_7$ | |
| 10.37 | H | $-C_3H_7(i)$ | |
| 10.38 | H | $-C_4H_9(i)$ | |
| 10.39 | H | $-C_4H_9(s)$ | |
| 10.40 | H | $-CH_2-CH=CH_2$ | |
| 10.41 | H | $-CH_2-CCl=CH_2$ | |
| 10.42 | H | $-CH_2-CH=CHCl$ | |
| 10.43 | H | $-CH_2-C\equiv CH$ | |
| 10.44 | H | $-CH_2-COOCH_3$ | |
| 10.45 | H | $-CH(CH_3)COOCH_3$ | |
| 10.46 | H | $-CH(CH_3)-CH_2-S-CH_3$ | |
| 10.47 | H | $-CH(CH_3)-CH_2-S-C_2H_5$ | |
| 10.48 | H | $-CH(CH_3)-CH_2-S-C_3H_7(i)$ | |

Tabelle 11

Verbindungen der Formel

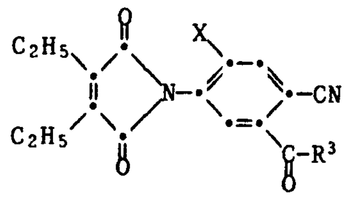

| Nr. | X | $R^3$ | Phys. Daten |
|---|---|---|---|
| 11.1 | F | OH | |
| 11.2 | F | $OCH_3$ | |
| 11.3 | F | $OC_2H_5$ | |
| 11.4 | F | $OC_3H_7$ | |
| 11.5 | F | $OC_3H_7(i)$ | |
| 11.6 | F | $OC_4H_9(n)$ | |
| 11.7 | F | $OC_4H_9(i)$ | |
| 11.8 | F | $OC_4H_9(s)$ | |
| 11.9 | F | $OC_4H_9(t)$ | |
| 11.10 | F | $OC_5H_{11}$ | |
| 11.11 | F | $OC_5H_{11}(s)$ | |
| 11.12 | F | $O-CH_2-CH_2-Cl$ | |
| 11.13 | F | $O-CH_2-CH_2-OCH_3$ | |
| 11.14 | F | $O-CH_2-CH_2-O-C_2H_5$ | |
| 11.15 | F | $O-CH_2-CH_2-S-CH_3$ | |
| 11.16 | F | $O-CH_2(CH_3)-CH_2-S-CH_3$ | |
| 11.17 | F | $O-CH_2(CH_3)-CH_2-S-C_2H_5$ | |
| 11.18 | F | $O-CH_2(CH_3)-CH_2-S-C_3H_7(i)$ | |
| 11.19 | F | $O-CH(CH_3)-CH_2-S-C_4H_9(n)$ | |
| 11.20 | F | $O-CH_2(CH_3)-CH_2-S-C_4H_9$ (i) | |
| 11.21 | F | $-O-CH_2-CH{=}CH_2$ | |
| 11.22 | F | $-O-CH_2-CCl{=}CH_2$ | |
| 11.23 | F | $-O-CH_2-CH{=}CHCl$ | |

Tabelle 11 (Fortsetzung)

| Nr. | X | $R^3$ | Phys. Daten |
|---|---|---|---|
| 11.24 | F | $-OCH_2-C{\equiv}CH$ | |
| 11.25 | F | $-O-CH_2-C_6H_{11}$-(cycl.) | |
| 11.26 | F | $-O-CH_2-C_3H_5$-(cycl.) | |
| 11.27 | F | $-O-CH_2-C_6H_5$ | |
| 11.28 | F | $-O-CH_2-COOCH_3$ | |
| 11.29 | F | $-O-CH(CH_3)COOCH_3$ | |
| 11.30 | F | $-O-CH(CH_3)COOC_2H_5$ | |
| 11.31 | F | $-O-CH(CH_3)COOC_3H_7(i)$ | |
| 11.32 | F | $-O-CH(CH_3)COOC_4H_9(n)$ | |
| 11.33 | F | $-O-CH(CH_3)COSCH_3$ | |
| 11.34 | F | $-O-CH(CH_3)CON(CH_3)_2$ | |
| 11.35 | F | $-S-CH_2-COOCH_3$ | |
| 11.36 | F | $-S-CH_2-COOC_2H_5$ | |
| 11.37 | F | $-S-CH(CH_3)-COOCH_3$ | |
| 11.38 | F | $-S-CH(CH_3)-COOC_2H_5$ | |
| 11.39 | F | $-S-CH(CH_3)-COOC_3H_7$ | |
| 11.40 | F | $-S-CH(CH_3)-COOC_3H_7(i)$ | |
| 11.41 | F | $-SCH_3$ | |
| 11.42 | F | $-N(CH_3)_2$ | |
| 11.43 | F | $-N(CH_2-CH{=}CH_2)_2$ | |
| 11.44 | F | 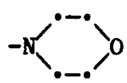 | |
| 11.45 | F | 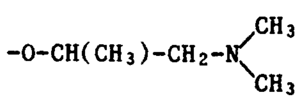 | |
| 11.46 | H | -OH | |
| 11.47 | H | $-OCH_3$ | |
| 11.48 | H | $-OC_2H_5$ | |
| 11.49 | H | $OC_3H_7(n)$ | |
| 11.50 | H | $OC_3H_7(i)$ | |
| 11.51 | H | $OC_4H_9(i)$ | |
| 11.52 | H | $OC_4H_9(s)$ | |

Tabelle 11 (Fortsetzung)

| Nr. | X | $R^3$ | Phys. Daten |
|---|---|---|---|
| 11.53 | H | $OCH_2-CH=CH_2$ | |
| 11.54 | H | $OCH_2-CCl=CH_2$ | |
| 11.55 | H | $OCH_2-CH=CHCl$ | |
| 11.56 | H | $-OCH_2-C\equiv CH$ | |
| 11.57 | H | $-OCH_2-COOCH_3$ | |
| 11.58 | H | $-OCH(CH_3)COOCH_3$ | |
| 11.59 | H | $-OCH(CH_3)CH_2-S-CH_3$ | |
| 11.60 | H | $-OCH(CH_3)CH_2-S-C_2H_5$ | |
| 11.61 | H | $-OCH(CH_3)CH_2-S-C_3H_7(i)$ | |
| 11.62 | H | $-S-CH_2-COOCH_3$ | |
| 11.63 | H | $-S-CH_2-COOC_2H_5$ | |
| 11.64 | H | $-S-CH(CH_3)COOCH_3$ | |
| 11.65 | H | $-S-CH(CH_3)COOC_2H_5$ | |
| 11.66 | H | $-S-CH(CH_3)-COO-C_3H_7(i)$ | |

Formulierungsbeispiele

Beispiel F 1.1: Formulierungsbeispiele für Wirkstoffe der Formel I (% = Gewichtsprozent)

| a) Emulsionskonzentrate | | | |
|---|---|---|---|
| | a) | b) | c) |
| Wirkstoff gemäss Tabelle 1 bis 11 | 20 % | 40 % | 50 % |
| Ca-Dodecylbenzolsulfonat | 5 % | 8 % | 5,8 % |
| Ricinusöl-polyethylenglykolether (36 Mol EO) | 5 % | - | - |
| Tributylphenoyl-polyethylenglykolether (30 Mol EO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| b) Lösungen | | | |
|---|---|---|---|
| | a) | b) | c) |
| Wirkstoff gemäss Tabelle 1 bis 11 | 80 % | 10 % | 5 % |
| Ethylenglykol-monomethylether | 20 % | - | - |
| Polyethylenglykol MG 400 | - | 70 % | - |
| N-Methyl-2-pyrrolidon | - | 20 % | 5 % |
| Epoxidiertes Kokosnussöl | - | - | 90 % |

Die Lösungen sind zur Anwendung in Form kleinster Tropfen geeignet.

| c) Granulate | | |
|---|---|---|
| | a) | b) |
| Wirkstoff gemäss Tabelle 1 bis 11 | 5 % | 10 % |
| Kaolin | 94% | - |
| Hochdisperse Kieselsäure | 1 % | |
| Attapulgit | - | 90% |

Der Wirkstoff wird gelöst, auf den Träger aufgesprüht und das Lösungsmittel anschliessend im Vakuum abgedampft.

| d) Stäubemittel | | | |
|---|---|---|---|
| | a) | b) | c) |
| Wirkstoff gemäss Tabelle 1 bis 11 | 2 % | 5 % | 8 % |
| Hochdisperse Kieselsäure | 1 % | 5 % | 5 % |
| Talkum | 97 % | - | 10 % |
| Kaolin | - | 90 % | 77 % |

Durch inniges Vermischen der Trägerstoffe mit dem Wirkstoff erhält man gebrauchsfertiges Stäubemittel.

| e) Spritzpulver | | |
|---|---|---|
| | a) | b) |
| Wirkstoff gemäss Tabelle 1 bis 11 | 20 % | 60 % |
| Na-Ligninsulfonat | 5 % | 5 % |
| Na-Laurylsulfat | 3 % | 6 % |
| Octylphenolpolyethylenglykolether (7-8 Mol EO) | - | 2% |
| Hochdisperse Kieselsäure | 5 % | 27 % |
| Kaolin | 67 % | - |

Der Wirkstoff wird mit den Zusatzstoffen gut vermischt und in einer geeigneten Mühle gut vermahlen. Man erhält Spritzpulver, die sich mit Wasser zur Suspension jeder gewünschten Konzentration verdünnen lassen.

| f) Extruder Granulat | |
|---|---|
| Wirkstoff gemäss Tabelle 1 bis 11 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

| g) Umhüllungs-Granulat | |
|---|---|
| Wirkstoff gemäss Tabelle 1 bis 11 | 3 % |
| Polyethylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| h) Suspensions-Konzentrat | |
|---|---|
| Wirkstoff gemäss Tabelle 1 bis 11 | 40 % |
| Ethylenglykol | 10 % |
| Nonylphenolpolyethylenglykolether (15 Mol EO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

## Biologische Beispiele

### Beispiel B 1: Herbizidwirkung im Vorauflauf

Unmittelbar nach der Einsaat der Samen in Blumentöpfe von 12-15 cm Durchmesser wird die Oberfläche mit einer wässrigen Spritzbrühe, entsprechend einer Aufwandmenge von 500, 250 oder 125[g] AS/[ha], behandelt.

Die Töpfe im Gewächshaus bei einer Temperatur von 22-25 °C und 50-70 % relativer Luftfeuchtigkeit belassen.

Nach 3 Wochen wird die Herbizidwirkung mit einem neunstufigen (1 = vollständige Schädigung, 9 = keine Wirkung) Boniturschema im Vergleich in einer unbehandelten Kontrollgruppe bewertet.

Boniturnoten von 1 bis 4 (insbesondere 1 bis 3) weisen auf eine gute bis sehr gute Herbizidwirkung hin. Boniturnoten von 6 bis 9 (insbesondere von 7 bis 9) weisen aauf eine gute Toleranz (insbesondere bei Kulturpflanzen) hin.

In diesem Versuch zeigen die Verbindungen gemäss Tabelle 1 bis 11 gute bis sehr Herbizidwirkung bei guter Kulturpflanzenverträglichkeit.

Die Ergebnisse für Verbindung Nr. 2.5 sind in Tabelle 12 zusammengestellt:

Tabelle 12

| Versuchspflanze | Aufwandmenge [g/ha] | | |
|---|---|---|---|
| | 500 | 250 | 125 |
| Gerste | 8 | 9 | 9 |
| Mais | 8 | 9 | 9 |
| Reis (Saatreis) | 9 | 9 | 9 |
| Soja | 9 | 9 | 9 |
| Baumwolle | 9 | 9 | 9 |
| Sonnenblume | 9 | 9 | 9 |
| Amaranthus ret. | 1 | 1 | 1 |
| Abutilon | 1 | 1 | 1 |
| Solanum nigrum | 1 | 1 | 1 |
| Viola tricolor | 1 | 1 | 1 |
| Veronica Sp. | 1 | 1 | 1 |

Beispiel B 2: Post-emergente Herbizid-Wirkung (Kontaktherbizid)

Eine Anzahl Unkräuter, sowohl monokotyle wie dikotyle, werden nach dem Auflaufen (im 4- bis 6-Blattstadium) mit einer wässrigen Wirkstoffdispersion in einer Dosierung von 500, 250, 125 und 60 g Wirksubstanz pro Hektar auf die Pflanzen gespritzt und diese bei 24°-26°C und 45-60 % relativer Luftfeuchtigkeit gehalten. 15 Tage nach der Behandlung wird der Versuch nach der obigen Notenskala ausgewertet. Auch in diesem Versuch zeigen die Verbindungen der Tabelle 1 bis 11 starke bis sehr starke Herbizidwirkung.

Beispiel B 3: Herbizidwirkung für Wasserreis (paddy)

Die Wasserunkräuter Echinochloa crus galli und Monocharia vag. werden in Plastikbechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Emulsion der Prüfsubstanzen durch eine Spritzung auf die Gefässe mit einer Aufwandmenge von 0,5 bis 4 kg AS pro Hektar. Die Pflanzenbecher werden dann im Gewächshaus unter optimalen Wachstumsbedingungen für die Reisunkräuter aufgestellt, d.h. bei 25°-30°C und hoher Luftfeuchtigkeit. Die Auswertung der Versuche findet 3 Wochen nach Applikation statt. Die Verbindungen gemäss Herstellungsbeispiel 1 schädigen dabei die Unkräuter, nicht aber den Reis.

**Patentansprüche**
**Patentansprüche für folgende Vertragsstaaten : AT, BE, CH, DE, FR, GB, GR, IT, LI, LU, NL, SE**

**1.** Verbindungen der Formel I

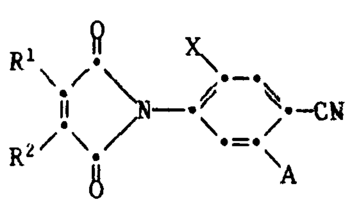

(I),

worin

- $R^1$ Wasserstoff; oder $(C_1\text{-}C_4)$-Alkyl;
- $R^2$ $(C_1\text{-}C_4)$-Alkyl; oder
- $R^1$ und $R^2$ gemeinsam eine $-(CH_2)_4$-Gruppe bedeuten, die bis zu zweifach durch $(C_1\text{-}C_4)$-Alkyl

substituiert sein kann;

X Wasserstoff; oder Halogen;

A 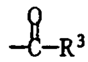 ;

oder O-$R^4$

$R^3$ Hydroxy; ($C_1$-$C_8$)-Alkoxy; ($C_1$-$C_4$)-Alkoxy-($C_1$-$C_4$)-alkoxy; ($C_1$-$C_4$)-Alkylthio-($C_1$-$C_4$)-alkoxy; Mono- oder Di-($C_1$-$C_4$)-Alkylamino-($C_1$-$C_4$)-alkoxy; ($C_1$-$C_8$)-Cyanoalkoxy; ($C_3$-$C_8$)-Alkenyloxy; ($C_3$-$C_8$)-Halogenalkenyloxy; ($C_3$-$C_8$)-Alkinyloxy; ($C_3$-$C_7$)-Cycloalkyloxy; ($C_3$-$C_7$)-Cycloalkyl-($C_1$-$C_4$)-alkyloxy; Halogen-($C_3$-$C_7$)-cycloalkoxy; Benzyloxy, das unsubstituiert oder am Phenylkern einfach durch Halogen, ($C_1$-$C_4$)-Alkyl, ($C_1$-$C_4$)-Alkoxy, ($C_1$-$C_4$)-Halogenalkyl, Cyan oder Nitro substituiert ist; Phenoxy; Halogenphenoxy; ($C_1$-$C_4$)-Alkylphenoxy; ($C_1$-$C_4$)-Alkyloxyphenoxy; ($C_1$-$C_4$)-Halogenalkylphenoxy; Cyanophenoxy; Nitrophenoxy; Phenylthio; Halogenphenylthio; ($C_1$-$C_4$)-Alkylphenylthio; ($C_1$-$C_4$)-Alkoxyphenylthio; ($C_1$-$C_4$)-Halogenalkylthio; Cyanophenylthio; Nitrophenylthio; die Salzgruppen -O-Na, -O-K, -O-$(Ca)_{0,5}$, -O-$(Mg)_{0,5}$ oder -O-$NH_4$; Amino; ($C_1$-$C_4$)-Alkylamino; Di-($C_1$-$C_4$)-alkylamino; ($C_2$-$C_4$)-Halogenalkylamino; Di-($C_2$-$C_4$)-halogenalkylamino; ($C_1$-$C_4$)-Hydroxyalkylamino; Di-($C_1$-$C_4$)-Hydroxyalkylamino; ($C_3$-$C_4$)-Alkenylamino; Diallylamino; -N-Pyrrolidino; -N-Piperidino; -N-Morpholino; -N-Thiomorpholino; -N-Piperidazino; ($C_1$-$C_8$)-Alkylthio; ($C_3$-$C_8$)-Alkenylthio; Benzylthio; ($C_1$-$C_4$)-Alkylthio substituiert durch ($C_1$-$C_8$)-Alkoxycarbonyl, ($C_1$-$C_4$)-Alkoxy-($C_1$-$C_4$)-alkoxycarbonyl, ($C_3$-$C_8$)-Alkenyloxycarbonyl, ($C_3$-$C_8$)-Alkinyloxycarbonyl, ($C_1$-$C_8$)-Alkylthiocarbonyl, ($C_3$-$C_8$)-Alkenylthiocarbonyl oder ($C_3$-$C_8$)-Alkinylthiocarbonyl; oder ($C_1$-$C_4$)-Alkoxy substituiert durch ($C_1$-$C_8$)-Alkoxycarbonyl, ($C_1$-$C_4$)-Alkoxy-($C_1$-$C_4$)-alkoxycarbonyl, ($C_3$-$C_8$)-Alkenyloxycarbonyl, ($C_3$-$C_8$)-Alkinyloxycarbonyl, ($C_1$-$C_8$)-Alkylthiocarbonyl, ($C_3$-$C_8$)-Alkenylthiocarbonyl, ($C_3$-$C_8$)-Alkinylthiocarbonyl, ($C_1$-$C_4$)-Alkylaminocarbonyl, Di-($C_1$-$C_4$)-Alkylaminocarbonyl oder Phenylaminocarbonyl, das unsubstituiert oder am Phenylkern einfach durch Halogen, ($C_1$-$C_4$)-Alkyl, ($C_1$-$C_4$)-Alkoxy, ($C_1$-$C_4$)-Halogenalkyl, Cyan oder Nitro substituiert ist;

$R^4$ ($C_1$-$C_8$)-Alkyl; ($C_1$-$C_4$)-Alkoxy-($C_1$-$C_4$)-Alkyl; ($C_1$-$C_4$)-Alkylthio-($C_1$-$C_4$)-alkyl, Mono- oder Di-($C_1$-$C_4$)-Alkylamino-($C_1$-$C_4$)-alkyl; ($C_1$-$C_8$)-Halogenalkyl; ($C_1$-$C_8$)-Cyanoalkyl; ($C_3$-$C_8$)-Alkenyl; ($C_3$-$C_8$)-Halogenalkenyl; ($C_3$-$C_8$)-Alkinyl; ($C_3$-$C_7$)-Cycloalkyl; ($C_3$-$C_7$)-Cycloalkyl-($C_1$-$C_4$)-Alkyl; ($C_3$-$C_7$)-Halogencycloalkyl; ($C_1$-$C_8$)-Alkylcarbonyl; Allylcarbonyl; Benzylcarbonyl, das unsubstituiert oder am Phenylring einfach durch Halogen, ($C_1$-$C_4$)-Alkyl, ($C_1$-$C_4$)-Alkoxy, ($C_1$-$C_4$)-Halogenalkyl, Cyan oder Nitro substituiert ist; ($C_3$-$C_7$)-Cycloalkylcarbonyl; Benzoyl, das unsubstituiert oder am Phenylring einfach durch Halogen, ($C_1$-$C_4$)-Alkyl, ($C_1$-$C_4$)-Alkoxy, ($C_1$-$C_4$)-Halogenalkyl, Cyan oder Nitro substituiert ist; Furoyl; Thienoyl; ($C_1$-$C_4$)-Alkyl substituiert durch Phenyl, Halogenphenyl, ($C_1$-$C_4$)-Alkylphenyl, ($C_1$-$C_4$)-Alkoxyphenyl, ($C_1$-$C_4$)-Haloalkylphenyl, ($C_1$-$C_4$)-Halogenalkoxyphenyl, Nitrophenyl, Cyanophenyl, ($C_1$-$C_8$)-Alkoxycarbonyl, ($C_1$-$C_4$)-Alkoxy-($C_1$-$C_4$)-alkoxycarbonyl, ($C_3$-$C_8$)-Alkenyloxycarbonyl, ($C_3$-$C_8$)-Alkinyloxycarbonyl, ($C_1$-$C_8$)-Alkylthiocarbonyl, ($C_3$-$C_8$)-Alkenylthiocarbonyl, ($C_3$-$C_8$)-Alkinylthiocarbonyl, Carbamoyl, ($C_1$-$C_4$)-Alkylaminocarbonyl, Di-($C_1$-$C_4$)-Alkylaminocarbonyl, Hydroxy, Phenylaminocarbonyl, das unsubstituiert oder am Phenylring einfach durch Halogen, ($C_1$-$C_4$)-Alkyl, ($C_1$-$C_4$)-Alkoxy, Cyan, Nitro oder ($C_1$-$C_4$)-Halogenalkyl substituiert ist; Dioxolan-2-yl, das unsubstituiert oder durch ein bis zwei ($C_1$-$C_4$)-Alkylreste substituiert ist oder 1,3-Dioxan-2-yl, das unsubstituiert oder durch ein bis zwei ($C_1$-$C_4$)-Alkylreste substituiert ist; bedeutet.

**2.** Verbindungen der Formel I gemäss Anspruch 1, worin X für Fluor oder Wasserstoff steht.

**3.** Verbindungen der Formel I gemäss Anspruch 1 oder 2, worin A für $OR^4$ und $R^4$ für Isopropyl steht oder A für $COOR^3$ und $R^3$ für sek-Butyl steht.

4. 1-(4-Cyano-2-fluor-5-isopropyloxyphenyl)-3,4-dimethyl-pyrrol-(1H)2,5-dion, 2-(5-Carboxy-4-cyano-2-fluorphenyl)-4,5,6,7-tetrahydroisoindol-(2H)1,3-dion, 2-(4-Cyano-2-fluor-5-n-propyloxycarbonylphenyl)-4,5,6,7-tetrahydroisoindol-(2H)1,3-dion, 2-(5-n-Butyloxycarbonyl-4-cyano-2-fluorphenyl)-4,5,6,7-tetrahydroisoindol-(2H)1,3-dion, 2-(4-Cyano-2-fluor-5-isopropyloxyphenyl)-5-methyl-4,5,6,7-tetrahydroisoindol-(2H)1,3-dion, 1-(4-Cyano-2-fluor-5-isopropyloxyphenyl)-3-ethyl-4-methyl-pyrrol-(1H)2,5-dion, 2-(4-Cyano-2-fluor-5-isopropyloxyphenyl-4,5,6,7-tetrahydroisoindol-(2H)1,3-dion, 2-(4-Cyano-2-fluor-5-isopropyloxycarbonylphenyl)-4,5,6,7-tetrahydroisoindol-(2H)1,3-dion oder 2-(5-sek-Butyloxycarbonyl-4-cyano-2-fluorphenyl)-4,5,6,7-tetrahydro-isoindol-(2H)1,3-dion als Verbindung der Formel I gemäss Anspruch 1.

5. Verfahren zur Herstellung von Verbindungen der Formel I gemäss Anspruch 1, gekennzeichnet,
a) durch die Umsetzung eines Furan-2,5-dions der Formel II mit einem Anilin III

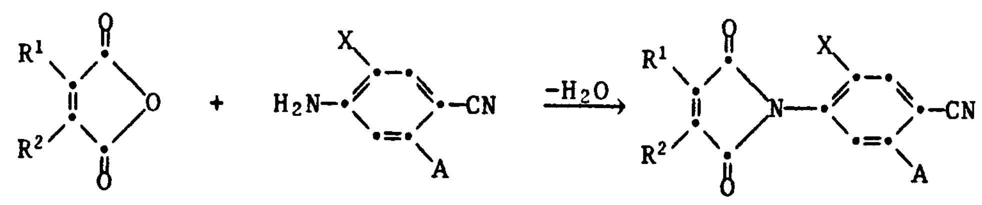


II III I,

worin die Reste $R^1$, $R^2$, X und A wie zuvor definiert sind
oder
b) durch einen Halogen-Cyanid-Austausch ausgehend von einer Verbindung der Formel IV

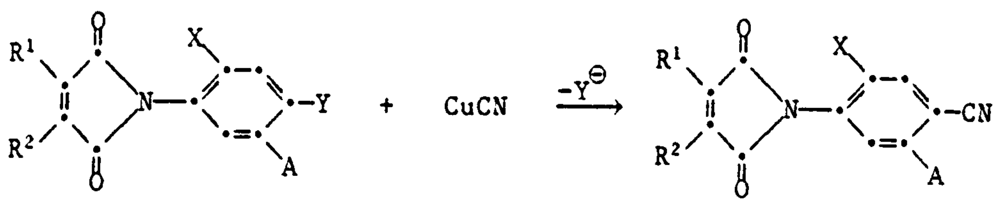


IV,

worin die Reste $R^1$, $R^2$, X und A wie zuvor definiert sind und Y für Chlor, Brom oder Jod steht, mit CuCN.

6. Verfahren zur Herstellung von Verbindungen der Formel I gemäss Anspruch 1, bei denen A für $COR^3$ steht, gekennzeichnet durch
a) die Kondensation einer Verbindung der Formel V, worin $R^1$, $R^2$ und X wie zuvor definiert sind,

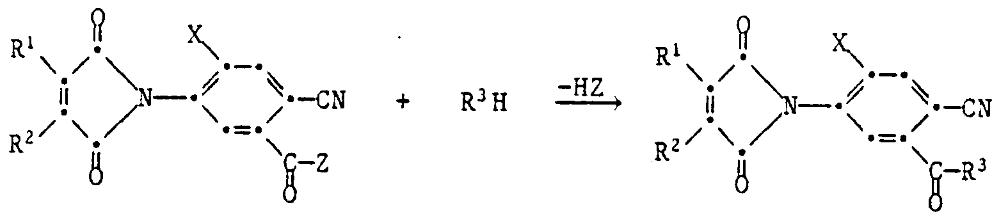


V VI I

und Z für eine unter den Reaktionsbedingungen austauschbare Gruppe, wie Halogen oder ($C_1$-$C_4$)-Alkylcarbonyloxy, steht, mit einer Verbindung der Formel VI, worin $R^3$ wie zuvor definiert ist
oder

b) die Veresterung einer Carbonsäure der Formel Ia', worin die Reste $R^1$, $R^2$ und X wie zuvor definiert sind, mit einem Alkohol oder Thioalkohol der Formel VI

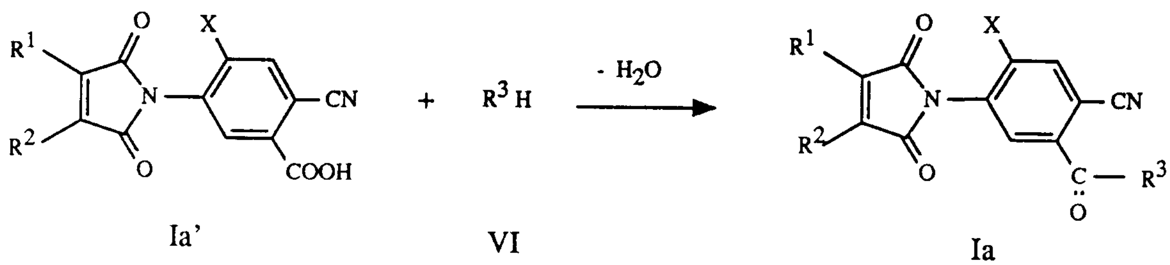


Ia' VI Ia

worin

$R^3$ ($C_1$-$C_8$)-Alkoxy; ($C_1$-$C_4$)-Alkoxy-($C_1$-$C_4$)-alkoxy; ($C_1$-$C_4$)-Alkylthio-($C_1$-$C_4$)-alkoxy; Mono- oder Di-($C_1$-$C_4$)-Alkylamino-($C_1$-$C_4$)-alkoxy; ($C_1$-$C_8$)-Cyanoalkoxy; ($C_3$-$C_8$)-Alkenyloxy; ($C_3$-$C_8$)-Halogenalkenyloxy; ($C_3$-$C_8$)-Alkinyloxy; ($C_3$-$C_7$)-Cycloalkyloxy; ($C_3$-$C_7$)-Cycloalkyl-($C_1$-$C_4$)-alkyloxy; Halogen-($C_3$-$C_7$)-cycloalkoxy; Benzyloxy, das unsubstituiert oder am Phenylkern einfach durch Halogen, ($C_1$-$C_4$)-Alkyl, ($C_1$-$C_4$)-Alkoxy, ($C_1$-$C_4$)-Halogenalkyl, Cyan oder Nitro substituiert ist; Phenoxy, Halogenphenoxy; ($C_1$-$C_4$)-Alkylphenoxy; ($C_1$-$C_4$)-Alkyloxyphenoxy; Halogenalkylphenoxy; Cyanophenoxy; Nitrophenoxy; Phenylthio; Halogenphenylthio; ($C_1$-$C_4$)-Alkylphenylthio; ($C_1$-$C_4$)-Alkoxyphenylthio; ($C_1$-$C_4$)-Halogenalkylthio; Cyanophenylthio; Nitrophenylthio; ($C_1$-$C_8$)-Alkylthio; ($C_3$-$C_8$)-Alkenylthio; Benzylthio; ($C_1$-$C_4$)-Alkylthio substituiert durch ($C_1$-$C_8$)-Alkoxycarbonyl, ($C_1$-$C_4$)-Alkoxy-($C_1$-$C_4$)-alkoxycarbonyl, ($C_3$-$C_8$)-Alkenyloxycarbonyl, ($C_3$-$C_8$)-Alkinyloxycarbonyl, ($C_1$-$C_8$)-Alkylthiocarbonyl oder ($C_3$-$C_8$)-Alkinylthiocarbonyl; oder ($C_1$-$C_4$)-Alkoxy substituiert durch ($C_1$-$C_8$)-Alkoxycarbonyl, ($C_1$-$C_4$)-Alkoxy-($C_1$-$C_4$)-alkoxycarbonyl, ($C_3$-$C_8$)-Alkenyloxycarbonyl, ($C_3$-$C_8$)-Alkinyloxycarbonyl, ($C_1$-$C_8$)-Alkylthiocarbonyl, ($C_3$-$C_8$)-Alkenylthiocarbonyl, ($C_3$-$C_8$)-Alkinylthiocarbonyl, ($C_1$-$C_4$)-Alkylaminocarbonyl, Di-($C_1$-$C_4$)-Alkylaminocarbonyl oder Phenylaminocarbonyl, das unsubstituiert oder am Phenylkern einfach durch Halogen, ($C_1$-$C_4$)-Alkyl, ($C_1$-$C_4$)-Alkoxy, ($C_1$-$C_4$)-Halogen, Cyan oder Nitro substituiert ist bedeutet;

zu einem Ester der Formel Ia, worin die Reste $R^1$, $R^2$, $R^3$ und X wie zuvor definiert sind.

**7.** Verfahren zur Herstellung von Verbindungen der Formel I gemäss Anspruch 1, in denen A für $OR^4$ steht, gekennzeichnet durch die Veretherung eines Phenols der Formel VII, worin $R^1$, $R^2$ und X wie zuvor definiert sind

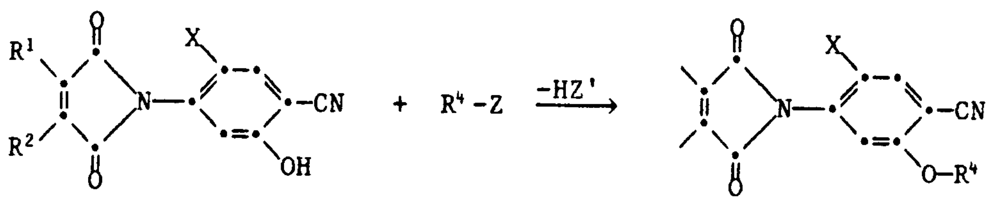


VII VIII Ib

mit einer Verbindung der Formel VIII, in der $R^4$ wie zuvor definiert ist und Z für eine unter den Reaktionsbedingungen abspaltbare Gruppe, wie Halogen oder gegebenenfalls alkyliertes Phenylsulfonyloxy, steht.

**8.** Aniline der Formel IIIb

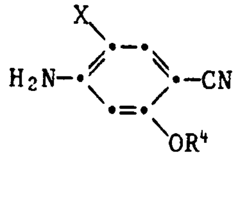

IIIb

worin
X Wasserstoff; oder Halogen; und
$R^4$ $(C_1-C_8)$-Alkyl; $(C_1-C_4)$-Alkoxy-$(C_1-C_4)$-alkyl; $(C_1-C_4)$-Alkylthio-$(C_1-C_4)$-alkyl, Mono- oder Di-$(C_1-C_4)$-Alkylamino-$(C_1-C_4)$-alkyl; $(C_1-C_8)$-Halogenalkyl; $(C_1-C_8)$-Cyanoalkyl; $(C_3-C_8)$-Alkenyl; $(C_3-C_8)$-Halogenalkenyl; $(C_3-C_8)$-Alkinyl; $(C_3-C_7)$-Cycloalkyl; $(C_3-C_7)$-Cycloalkyl-$(C_1-C_4)$-alkyl; $(C_3-C_7)$-Halogencycloalkyl; $(C_1-C_8)$-Alkylcarbonyl; Allylcarbonyl; Benzylcarbonyl, das unsubstituiert oder am Phenylring einfach durch Halogen, $(C_1-C_4)$-Alkyl, $(C_1-C_4)$-Alkoxy, $(C_1-C_4)$-Halogenalkyl, Cyan oder Nitro substituiert ist; $(C_3-C_7)$-Cycloalkylcarbonyl; Benzoyl, das unsubstituiert oder am Phenylring einfach durch Halogen, $(C_1-C_4)$-Alkyl, $(C_1-C_4)$-Alkoxy, $(C_1-C_4)$-Halogenalkyl, Cyan oder Nitro substituiert ist; Furoyl; Thienoyl; $(C_1-C_4)$-Alkyl substituiert durch Phenyl, Halogenphenyl, $(C_1-C_4)$-Alkylphenyl, $(C_1-C_4)$-Alkoxyphenyl, $(C_1-C_4)$-Haloalkylphenyl, $(C_1-C_4)$-Halogenalkoxyphenyl, Nitrophenyl, Cyanophenyl, $(C_1-C_8)$-Alkoxycarbonyl, $(C_1-C_4)$-Alkoxy-$(C_1-C_4)$-alkoxycarbonyl, $(C_3-C_8)$-Alkenyloxycarbonyl, $(C_3-C_8)$-Alkinyloxycarbonyl, $(C_1-C_8)$-Alkylthiocarbonyl, $(C_3-C_8)$-Alkenylthiocarbonyl, $(C_3-C_8)$-Alkinylthiocarbonyl, Carbamoyl, $(C_1-C_4)$-Alkylaminocarbonyl, Di-$(C_1-C_4)$-Alkylaminocarbonyl, Hydroxy, Phenylaminocarbonyl, das unsubstitiert oder am Phenylring einfach durch Halogen, $(C_1-C_4)$-Alkyl, $(C_1-C_4)$-Alkoxy, Cyan, Nitro oder $(C_1-C_4)$-Halogenalkyl substituiert ist, Dioxolan-2-yl, das unsubstituiert oder durch ein bis zwei $(C_1-C_4)$-Alkylreste substituiert ist oder 1,3-Dioxan-2-yl, das unsubstituiert oder durch ein bis zwei $(C_1-C_4)$-Alkylreste substituiert ist; bedeutet, mit Ausnahme des 2-Methoxy-4-amino-5-chlorbenzonitrils.

**9.** Aniline der Formel IIIc

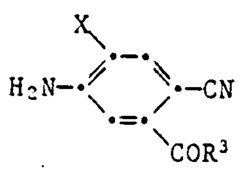

IIIc

worin
X Wasserstoff; oder Halogen; und
$R^3$ Hydroxy; $(C_1-C_8)$-Alkoxy; $(C_1-C_4)$-Alkoxy-$(C_1-C_4)$-alkoxy; $(C_1-C_4)$-Alkylthio-$(C_1-C_4)$-alkoxy; Mono- oder Di-$(C_1-C_4)$-Alkylamino-$(C_1-C_4)$-alkoxy; $(C_1-C_8)$-Cyanoalkoxy; $(C_3-C_8)$-Alkenyloxy; $(C_3-C_8)$-Halogenalkenyloxy; $(C_3-C_8)$-Alkinyloxy; $(C_3-C_7)$-Cycloalkyloxy; $(C_3-C_7)$-Cycloalkyl-$(C_1-C_4)$-alkyloxy; Halogen-$(C_3-C_7)$-cycloalkoxy; Benzyloxy, das unsubstituiert oder am Phenylkern einfach durch Halogen, $(C_1-C_4)$-Alkyl, $(C_1-C_4)$-Alkoxy, $(C_1-C_4)$-Halogenalkyl, Cyan oder Nitro substituiert ist; Phenoxy; Halogenphenoxy; $(C_1-C_4)$-Alkylphenoxy; $(C_1-C_4)$-Alkyloxyphenoxy; $(C_1-C_4)$-Halogenalkylphenoxy; Cyanophenoxy; Nitrophenoxy; Phenylthio; Halogenphenylthio; $(C_1-C_4)$-Alkylphenylthio; $(C_1-C_4)$-Alkoxyphenylthio; $(C_1-C_4)$-Halogenalkylthio; Cyanophenylthio; Nitrophenylthio; die Salzgruppen -O-Na, -O-K, $-O-(Ca)_{0,5}$, $-O-(Mg)_{0,5}$ oder $-O-NH_4$; Amino; $(C_1-C_4)$-Alkylamino; Di-$(C_1-C_4)$-alkylamino; $(C_2-C_4)$-Halogenalkylamino; Di-$(C_2-C_4)$-halogenalkylamino; $(C_1-C_4)$-Hydroxyalkylamino; Di-$(C_1-C_4)$-Hydroxyalkylamino; $(C_3-C_4)$-Alkenylamino; Diallylamino; -N-Pyrrolidino; -N-Piperidino; -N-Morpholino; -N-Thiomorpholino; -N-Piperidazino; $(C_1-C_8)$-Alkylthio; $(C_3-C_8)$-Alkenylthio; Benzylthio; $(C_1-C_4)$-Alkylthio substituiert durch $(C_1-C_8)$-Alkoxycarbonyl, $(C_1-C_4)$-Alkoxy-$(C_1-C_4)$-alkoxycarbonyl, $(C_3-C_8)$-Alkenyloxycarbonyl, $(C_3-C_8)$-Alkinyloxycarbonyl, $(C_1-C_8)$-Alkylthiocarbonyl, $(C_3-C_8)$-Alkenylthiocarbonyl oder $(C_3-C_8)$-Alkinylthiocarbonyl; oder $(C_1-C_4)$-Alkoxy substituiert durch $(C_1-C_8)$-Alkoxycarbonyl, $(C_1-C_4)$-Alkoxy-$(C_1-C_4)$-alkoxycarbonyl, $(C_3-C_8)$-Alkenylcarbonyl, $(C_3-C_8)$-Alkinyloxycarbonyl, $(C_1-C_8)$-Alkylthiocarbonyl, $(C_3-C_8)$-Alkenylthiocarbonyl, $(C_3-C_8)$-Alkinylthiocarbonyl, $(C_1-C_4)$-Alkylaminocarbonyl, Di-$(C_1-C_4)$-Alkylaminocarbonyl oder Phenylaminocarbonyl, das unsubstituiert oder am Phenylkern einfach durch Halogen, $(C_1-C_4)$-Alkyl, $(C_1-C_4)$-Alkoxy, $(C_1-C_4)$-Halogenalkyl, Cyan oder Nitro substituiert ist;
bedeutet.

**10.** Verbindungen der Formel VII

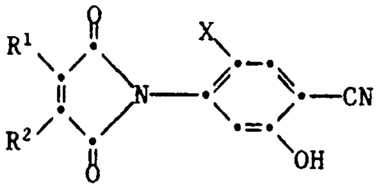

(VII),

worin

| | |
|---|---|
| $R^1$ | Wasserstoff; oder $(C_1-C_4)$-Alkyl; |
| $R^2$ | $(C_1-C_4)$-Alkyl; oder |
| $R^1$ und $R^2$ | gemeinsam eine $-(CH_2)_4$-Gruppe bedeuten, die bis zu zweifach durch $(C_1-C_4)$-Alkyl substituiert sein kann; |
| X | Wasserstoff; oder Halogen bedeutet. |

**11.** Herbizides oder wuchsregulatorisches Mittel enthaltend eine Verbindung der Formel I gemäss einem der Ansprüche 1 bis 4 neben weiteren Hilfs- und/oder Trägerstoffen.

**12.** Verfahren zur Bekämpfung unerwünschten Pflanzenwuchses, dadurch gekennzeichnet, dass man eine Verbindung der Formel I gemäss einem der Ansprüche 1 bis 4 oder ein herbizide Mittel gemäss Anspruch 11 auf die zu bekämpfende Pflanze oder deren Lebensraum einwirken lässt.

**13.** Saatgut gekennzeichnet durch einen herbizid wirksamen Gehalt an einer Verbindung der Formel I gemäss einem der Ansprüche 1 bis 4.

**14.** Verfahren zur Defoliation von Nutzpflanzen, dadurch gekennzeichnet, dass man eine Verbindung der Formel I gemäss einem der Ansprüche 1 bis 4 oder ein wuchsregulatorisches Mittel gemäss Anspruch 11 auf die zu entlaubende Pflanze einwirken lässt.

**Patentansprüche für folgenden Vertragsstaat : ES**

**1.** Verfahren zur Herstellung von Verbindungen der Formel I

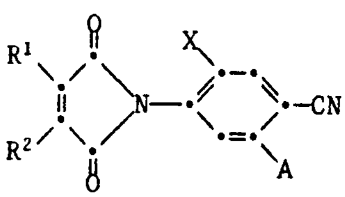

(I),

worin

| | |
|---|---|
| $R^1$ | Wasserstoff; oder $(C_1-C_4)$-Alkyl; |
| $R^2$ | $(C_1-C_4)$-Alkyl; oder |
| $R^1$ und $R^2$ | gemeinsam eine $-(CH_2)_4$-Gruppe bedeuten, die bis zu zweifach durch $(C_1-C_4)$-Alkyl substituiert sein kann; |
| X | Wasserstoff; oder Halogen; |
| A | |

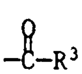

;

oder $O-R^4$

$R^3$ Hydroxy; $(C_1-C_8)$-Alkoxy; $(C_1-C_4)$-Alkoxy-$(C_1-C_4)$-alkoxy; $(C_1-C_4)$-Alkylthio-$(C_1-C_4)$-alkoxy; Mono- oder Di-$(C_1-C_4)$-Alkylamino-$(C_1-C_4)$-alkoxy; $(C_1-C_8)$-Cyanoalkoxy; $(C_3-C_8)$-Alkenyloxy; $(C_3-C_8)$-Halogenalkenyloxy; $(C_3-C_8)$-Alkinyloxy; $(C_3-C_7)$-Cycloalkoxy; $(C_3-C_7)$-Cycloalkyl-$(C_1-C_4)$-alkyloxy; Halogen-$(C_3-C_7)$-cycloalkoxy; Benzyloxy, das unsubstituiert oder am Phenylkern einfach durch Halogen, $(C_1-C_4)$-Alkyl, $(C_1-C_4)$-Alkoxy, $(C_1-C_4)$-Halogenalkyl, Cyan oder Nitro substituiert ist; Phenoxy; Halogenphenoxy; $(C_1-C_4)$-Alkylphenoxy; $(C_1-C_4)$-Alkyloxyphenoxy; $(C_1-C_4)$-Halogenalkylphenoxy; Cyanophenoxy; Nitrophenoxy; Phenylthio; Halogenphenylthio; $(C_1-C_4)$-Alkylphenylthio; $(C_1-C_4)$-Alkoxyphenylthio; $(C_1-C_4)$-Halogenalkylthio; Cyanophenylthio; Nitrophenylthio; die Salzgruppen -O-Na, -O-K, -O-$(Ca)_{0,5}$, -O-$(Mg)_{0,5}$ oder -O-$NH_4$; Amino; $(C_1-C_4)$-Alkylamino; Di-$(C_1-C_4)$-alkylamino; $(C_2-C_4)$-Halogenalkylamino; Di-$(C_2-C_4)$-halogenalkylamino; $(C_1-C_4)$-Hydroxyalkylamino; Di-$(C_1-C_4)$-Hydroxyalkylamino; $(C_3-C_4)$-Alkenylamino; Diallylamino; -N-Pyrrolidino; -N-Piperidino; -N-Morpholino; N-Thiomorpholino; -N-Piperidazino; $(C_1-C_8)$-Alkylthio; $(C_3-C_8)$-Alkenylthio; Benzylthio; $(C_1-C_4)$-Alkylthio substituiert durch $(C_1-C_8)$-Alkoxycarbonyl, $(C_1-C_4)$-Alkoxy-$(C_1-C_4)$alkoxycarbonyl, $(C_3-C_8)$-Alkenyloxycarbonyl, $(C_3-C_8)$-Alkinyloxycarbonyl, $(C_1-C_8)$-Alkylthiocarbonyl, $(C_3-C_8)$-Alkenylthiocarbonyl oder $(C_3-C_8)$-Alkinylthiocarbonyl; oder $(C_1-C_4)$-Alkoxy substituiert durch $(C_1-C_8)$-Alkoxycarbonyl, $(C_1-C_4)$-Alkoxy-$(C_1-C_4)$-alkoxycarbonyl, $(C_3-C_8)$-Alkenyloxycarbonyl, $(C_3-C_8)$-Alkinyloxycarbonyl, $(C_1-C_8)$-Alkylthiocarbonyl, $(C_3-C_8)$-Alkenylthiocarbonyl, $(C_3-C_8)$-Alkinylthiocarbonyl, $(C_1-C_4)$-Alkylaminocarbonyl, Di-$(C_1-C_4)$-Alkylaminocarbonyl oder Phenylaminocarbonyl, das unsubstituiert oder am Phenylkern einfach durch Halogen, $(C_1-C_4)$-Alkyl, $(C_1-C_4)$-Alkoxy, $(C_1-C_4)$-Halogenalkyl, Cyan oder Nitro substituiert ist;

$R^4$ $(C_1-C_8)$-Alkyl; $(C_1-C_4)$-Alkoxy-$(C_1-C_4)$-alkyl; $(C_1-C_4)$-Alkylthio-$(C_1-C_4)$-alkyl, Mono- oder Di-$(C_1-C_4)$-Alkylamino-$(C_1-C_4)$-alkyl; $(C_1-C_8)$-Halogenalkyl; $(C_1-C_8)$-Cyanoalkyl; $(C_3-C_8)$-Alkenyl; $(C_3-C_8)$-Halogenalkenyl; $(C_3-C_8)$-Alkinyl; $(C_3-C_7)$-Cycloalkyl; $(C_3-C_7)$-Cycloalkyl-$(C_1-C_4)$-alkyl; $(C_3-C_7)$-Halogencycloalkyl; $(C_1-C_8)$-Alkylcarbonyl; Allylcarbonyl; Benzylcarbonyl, das unsubstituiert oder am Phenylring einfach durch Halogen, $(C_1-C_4)$-Alkyl, $(C_1-C_4)$-Alkoxy, $(C_1-C_4)$-Halogenalkyl, Cyan oder Nitro substituiert ist; $(C_3-C_7)$-Cycloalkylcarbonyl; Benzoyl, das unsubstituiert oder am Phenylring einfach durch Halogen, $(C_1-C_4)$-Alkyl, $(C_1-C_4)$-Alkoxy, $(C_1-C_4)$-Halogenalkyl, Cyan oder Nitro substituiert ist; Furoyl; Thienoyl; $(C_1-C_4)$-Alkyl substituiert durch Phenyl, Halogenphenyl, $(C_1-C_4)$-Alkylphenyl, $(C_1-C_4)$-Alkoxyphenyl, $(C_1-C_4)$-Haloalkylphenyl, $(C_1-C_4)$-Halogenalkoxyphenyl, Nitrophenyl, Cyanophenyl, $(C_1-C_8)$-Alkoxycarbonyl, $(C_1-C_4)$-Alkoxy-$(C_1-C_4)$-alkoxycarbonyl, $(C_3-C_8)$-Alkenyloxycarbonyl, $(C_3-C_8)$-Alkinyloxycarbonyl, $(C_1-C_8)$-Alkylthiocarbonyl, $(C_3-C_8)$-Alkenylthiocarbonyl, $(C_3-C_8)$-Alkinylthiocarbonyl, Carbamoyl, $(C_1-C_4)$-Alkylaminocarbonyl, Di-$(C_1-C_4)$-Alkylaminocarbonyl, Hydroxy, Phenylaminocarbonyl, das unsubstituiert oder am Phenylring einfach durch Halogen, $(C_1-C_4)$-Alkyl, $(C_1-C_4)$-Alkoxy, Cyan, Nitro oder $(C_1-C_4)$-Halogenalkyl substituiert ist; Dioxolan-2-yl, das unsubstituiert oder durch ein bis zwei $(C_1-C_4)$-Alkylreste substituiert ist oder 1,3-Dioxan-2-yl, das unsubstituiert oder durch ein bis zwei $(C_1-C_4)$-Alkylreste substituiert ist;

bedeutet, gekennzeichnet

a) durch die Umsetzung eines Furan-2,5-dions der Formel II mit einem Anilin III

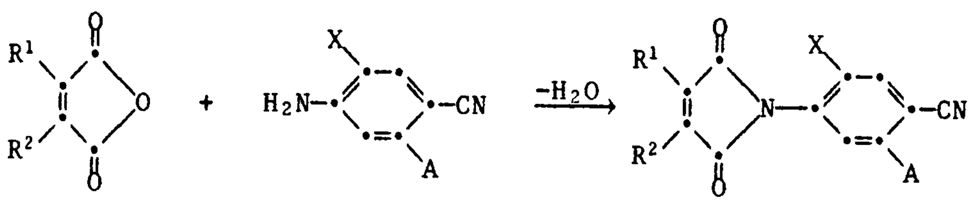


II III I,

worin die Reste $R^1$, $R^2$, X und A wie zuvor definiert sind
oder

b) durch einen Halogen-Cyanid-Austausch ausgehend von einer Verbindung der Formel IV

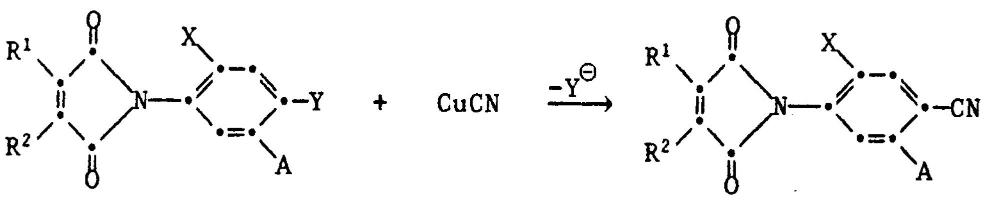


IV,

worin die Reste $R^1$, $R^2$, X und A wie zuvor definiert sind und Y für Chlor, Brom oder Jod steht, mit CuCN.

**2.** Verfahren nach Anspruch 1 zur Herstellung von Verbindungen der Formel I

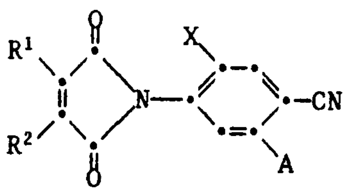

(I),

worin

| | |
|---|---|
| $R^1$ | Wasserstoff; oder $(C_1\text{-}C_4)$-Alkyl; |
| $R^2$ | $(C_1\text{-}C_4)$-Alkyl; oder |
| $R^1$ und $R^2$ | gemeinsam eine $-(CH_2)_4$-Gruppe bedeuten, die gegebenenfalls einfach durch $(C_1\text{-}C_4)$-Alkyl substituiert sein kann; |
| X | Wasserstoff; Fluor; Chlor; oder Brom; |
| A | 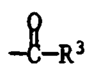 ; oder $O\text{-}R^4$; |
| $R^3$ | Hydroxy; $(C_1\text{-}C_5)$-Alkoxy; $(C_1\text{-}C_4)$-Alkoxy-$(C_1\text{-}C_4)$-alkoxy; $(C_1\text{-}C_4)$-Alkylthio-$(C_1\text{-}C_4)$-alkoxy; Mono- oder Di-$(C_1\text{-}C_4)$-Alkylamino-$(C_1\text{-}C_4)$-alkoxy; $(C_1\text{-}C_4)$-Cyanoalkoxy; $(C_3\text{-}C_5)$-Halogenalkenyloxy; $(C_3\text{-}C_5)$-Alkinyloxy; $(C_3\text{-}C_5)$-Alkenyloxy; $(C_3\text{-}C_6)$-Cycloalkylmenthyloxy; die Salzgruppen -O-Na, -O-K, $-O-(Ca)_{0,5}$, $-O-(Mg)_{0,5}$ oder $-O-NH_4$; Amino; Di-$(C_1\text{-}C_4)$-Alkylamino; Diallylamino; Benzyloxy; N-Piperidino; -N-Morpholino; -N-Thiomorpholino; $(C_1\text{-}C_4)$-Alkylthio; $(C_1\text{-}C_4)$-Alkylthio, substituiert durch $(C_1\text{-}C_4)$-Alkoxycarbonyl; $(C_1\text{-}C_4)$-Alkoxy, substituiert durch $(C_1\text{-}C_4)$-Alkoxycarbonyl, $(C_1\text{-}C_4)$-Alkylthiocarbonyl, $(C_1\text{-}C_4)$-Alkylaminocarbonyl oder Di-$(C_1\text{-}C_4)$-Alkylaminocarbonyl; |
| $R^4$ | $(C_1\text{-}C_5)$-Alkyl; $(C_1\text{-}C_4)$-Alkoxy-$(C_1\text{-}C_4)$-alkyl; $(C_1\text{-}C_4)$-Alkylthio-$(C_1\text{-}C_4)$-alkyl; $(C_1\text{-}C_4)$-Halogenalkyl; $(C_3\text{-}C_5)$-Alkenyl; $(C_3\text{-}C_5)$-Halogenalkenyl; $(C_3\text{-}C_5)$-Alkinyl; Cyclohexylmethyl; $(C_1\text{-}C_4)$-Alkylcarbonyl; Benzoyl, das unsubstituiert oder am Phenylring einfach durch Fluor, Chlor, $(C_1\text{-}C_4)$-Alkyl, $(C_1\text{-}C_4)$-Alkoxy, Cyan oder Nitro substituiert ist; $(C_1\text{-}C_4)$-Alkyl, einfach substituiert durch Phenyl, $(C_1\text{-}C_4)$-Alkoxycarbonyl, $(C_1\text{-}C_4)$-Alkylthiocarbonyl, Carbamoyl, Di-$(C_1\text{-}C_4)$-Alkylaminocarbonyl oder Hydroxy; |

bedeutet.

3. Verfahren nach Anspruch 1 zur Herstellung von Verbindungen der Formel I

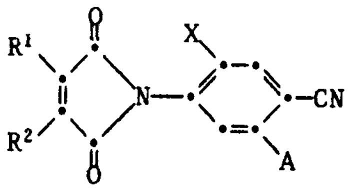

(I),

worin X für Fluor oder Wasserstoff steht und die Reste $R^1$, $R^2$ und A die in Anspruch 1 oder 2 gegebene Bedeutung haben.

4. Verfahren nach Anspruch 1 zur Herstellung von Verbindungen der Formel I

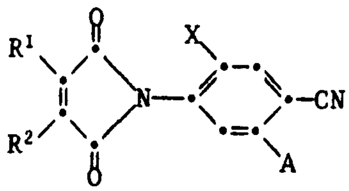

(I),

worin
A für $OR^4$ und $R^4$ für Isopropyl steht oder A für $COOR^3$ und $R^3$ für sec-Butyl steht und die Reste $R^1$, $R^2$ und X die in einem der Ansprüche 1 bis 3 gegebene Bedeutung haben.

5. Verfahren zur Herstellung von Verbindungen der Formel I

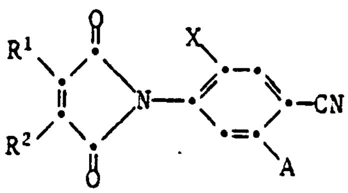

(I),

worin
A für $COR^3$ steht und die Reste $R^1$, $R^2$, $R^3$ und X die in einem der Ansprüche 1 bis 4 gegebene Bedeutung haben gekennzeichnet durch

a) die Kondensation einer Verbindung der Formel V, worin $R^1$, $R^2$ und X wie zuvor definiert sind,

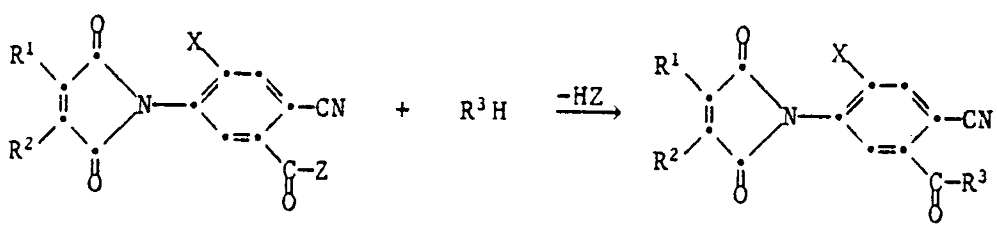


+ $R^3H$

V VI I

und Z für eine unter den Reaktionsbedingungen austauschbare Gruppe, wie Halogen oder $(C_1$-$C_4)$-Alkylcarbonyloxy, steht, mit einer Verbindung der Formel VI, worin $R^3$ wie zuvor definiert ist
oder

b) die Veresterung einer Carbonsäure der Formel Ia', worin die Reste $R^1$, $R^2$ und X wie zuvor definiert sind, mit einem Alkohol oder Thioalkohol der Formel VI

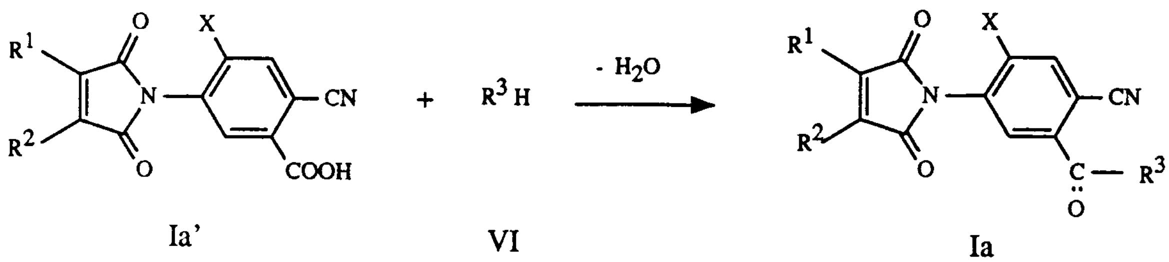


Ia' VI Ia

worin

$R^3$ ($C_1$-$C_8$)-Alkoxy; ($C_1$-$C_4$)-Alkoxy-($C_1$-$C_4$)-alkoxy; ($C_1$-$C_4$)-Alkylthio-($C_1$-$C_4$)-alkoxy; Mono- oder Di-($C_1$-$C_4$)-Alkylamino-($C_1$-$C_4$)-alkoxy; ($C_1$-$C_8$)-Cyanoalkoxy; ($C_3$-$C_8$)-Alkenyloxy; ($C_3$-$C_8$)-Halogenalkenyloxy; ($C_3$-$C_8$)-Alkinyloxy; ($C_3$-$C_7$)-Cycloalkyloxy; ($C_3$-$C_7$)-Cycloalkyl-($C_1$-$C_4$)-alkyloxy; Halogen-($C_3$-$C_7$)-cycloalkoxy; Benzyloxy, das unsubstituiert oder am Phenylkern einfach durch Halogen, ($C_1$-$C_4$)-Alkyl, ($C_1$-$C_4$)-Alkoxy, ($C_1$-$C_4$)-Halogenalkyl, Cyan oder Nitro substituiert ist; Phenoxy, Halogenphenoxy; ($C_1$-$C_4$)-Alkylphenoxy; ($C_1$-$C_4$)-Alkyloxyphenoxy; Halogenalkylphenoxy; Cyanophenoxy; Nitrophenoxy; Phenylthio; Halogenphenylthio; ($C_1$-$C_4$)-Alkylphenylthio; ($C_1$-$C_4$)-Alkoxyphenylthio; ($C_1$-$C_4$)-Halogenalkylthio; Cyanophenylthio; Nitrophenylthio; ($C_1$-$C_8$)-Alkylthio; ($C_3$-$C_8$)-Alkenylthio; Benzylthio; ($C_1$-$C_4$)-Alkylthio substituiert durch ($C_1$-$C_8$)-Alkoxycarbonyl, ($C_1$-$C_4$)-Alkoxy-($C_1$-$C_4$)-alkoxycarbonyl, ($C_3$-$C_8$)-Alkenyloxycarbonyl, ($C_3$-$C_8$)-Alkinyloxycarbonyl, ($C_1$-$C_8$)-Alkylthiocarbonyl oder ($C_3$-$C_8$)-Alkinylthiocarbonyl; oder ($C_1$-$C_4$)-Alkoxy substituiert durch ($C_1$-$C_8$)-Alkoxycarbonyl, ($C_1$-$C_4$)-Alkoxy-($C_1$-$C_4$)-alkoxycarbonyl, ($C_3$-$C_8$)-Alkenyloxycarbonyl, ($C_3$-$C_8$)-Alkinyloxycarbonyl, ($C_1$-$C_8$)-Alkylthiocarbonyl, ($C_3$-$C_8$)-Alkenylthiocarbonyl, ($C_3$-$C_8$)-Alkinylthiocarbonyl, ($C_1$-$C_4$)-Alkylaminocarbonyl, Di-($C_1$-$C_4$)-Alkylaminocarbonyl oder Phenylaminocarbonyl, das unsubstituiert oder am Phenylkern einfach durch Halogen, ($C_1$-$C_4$)-Alkyl, ($C_1$-$C_4$)-Alkoxy, ($C_1$-$C_4$)-Halogen, Cyan oder Nitro substituiert ist bedeutet;

zu einem Ester der Formel Ia, worin die Reste $R^1$, $R^2$, $R^3$ und X wie zuvor definiert sind.

**6.** Verfahren zur Herstellung von Verbindungen der Formel I

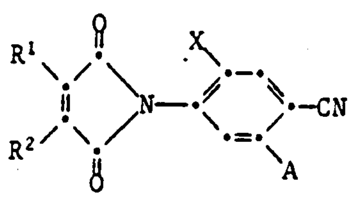

(I),

worin A für $OR^4$ steht und die Reste $R^1$, $R^2$, $R^4$ und X die in einem der Ansprüche 1 bis 4 gegebene Bedeutung haben, gekennzeichnet durch die Veretherung eines Phenols der Formel VII, worin $R^1$, $R^2$ und X wie zuvor definiert sind

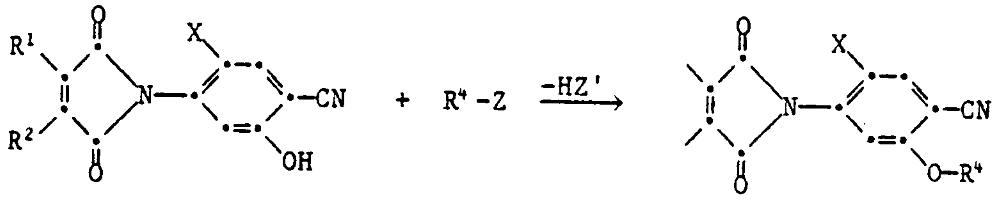


VII VIII Ib

mit einer Verbindung der Formel VIII, in der $R^4$ wie zuvor definiert ist und Z für eine unter den Reaktionsbedingungen abspaltbare Gruppe, wie Halogen oder gegebenenfalls alkyliertes Phenylsulfonyloxy, steht.

**7.** Verfahren zur Herstellung von Anilinen der Formel IIIb

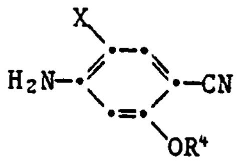

IIIb

worin

X Wasserstoff; oder Halogen; und

$R^4$ ($C_1$-$C_8$)-Alkyl; ($C_1$-$C_4$)-Alkoxy-($C_1$-$C_4$)-alkyl; ($C_1$-$C_4$)-Alkylthio-($C_1$-$C_4$)-alkyl, Mono- oder Di-($C_1$-$C_4$)-Alkylamino-($C_1$-$C_4$)-alkyl; ($C_1$-$C_8$)-Halogenalkyl; ($C_1$-$C_8$)-Cyanoalkyl; ($C_3$-$C_8$)-Alkenyl; ($C_3$-$C_8$)-Halogenalkenyl; ($C_3$-$C_8$)-Alkinyl; ($C_3$-$C_7$)-Cycloalkyl; ($C_3$-$C_7$)-Cycloalkyl-($C_1$-$C_4$)-alkyl; ($C_3$-$C_7$)-Halogencycloalkyl; ($C_1$-$C_8$)-Alkylcarbonyl; Allylcarbonyl; Benzylcarbonyl, das unsubstituiert oder am Phenylring einfach durch Halogen, ($C_1$-$C_4$)-Alkyl, ($C_1$-$C_4$)-Alkoxy, ($C_1$-$C_4$)-Halogenalkyl, Cyan oder Nitro substituiert ist; ($C_3$-$C_7$)-Cycloalkylcarbonyl; Benzoyl, das unsubstituiert oder am Phenylring einfach durch Halogen, ($C_1$-$C_4$)-Alkyl, ($C_1$-$C_4$)-Alkoxy, ($C_1$-$C_4$)-Halogenalkyl, Cyan oder Nitro substituiert ist; Furoyl; Thienoyl; ($C_1$-$C_4$)-Alkyl substituiert durch Phenyl, Halogenphenyl, ($C_1$-$C_4$)-Alkylphenyl, ($C_1$-$C_4$)-Alkoxyphenyl, ($C_1$-$C_4$)-Haloalkylphenyl, ($C_1$-$C_4$)-Halogenalkoxyphenyl, Nitrophenyl, Cyanophenyl, ($C_1$-$C_8$)-Alkoxycarbonyl, ($C_1$-$C_4$)-Alkoxy-($C_1$-$C_4$)-alkoxycarbonyl, ($C_3$-$C_8$)-Alkenyloxycarbonyl, ($C_3$-$C_8$)-Alkinyloxycarbonyl, ($C_1$-$C_8$)-Alkylthiocarbonyl, ($C_3$-$C_8$)-Alkenylthiocarbonyl, ($C_3$-$C_8$)-Alkinylthiocarbonyl, Carbamoyl, ($C_1$-$C_4$)-Alkylaminocarbonyl, Di-($C_1$-$C_4$)-Alkylaminocarbonyl, Hydroxy, Phenylaminocarbonyl, das unsubstitiert oder am Phenylring einfach durch Halogen, ($C_1$-$C_4$)-Alkyl, ($C_1$-$C_4$)-Alkoxy, Cyan, Nitro oder ($C_1$-$C_4$)-Halogenalkyl substituiert ist, Dioxolan-2-yl, das unsubstituiert oder durch ein bis zwei ($C_1$-$C_4$)-Alkylreste substituiert ist oder 1,3-Dioxan-2-yl, das unsubstituiert oder durch ein bis zwei ($C_1$-$C_4$)-Alkylreste substituiert ist; bedeutet, mit Ausnahme des 2-Methoxy-4-amino-5-chlorbenzonitrils,

gekennzeichnet durch

a) die Veretherung eines Phenols der Formel

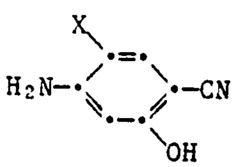

worin X wie zuvor definiert ist mit einer Verbindung der Formel VIII

$R^4$-Z (VIII),

worin $R^4$ wie zuvor definiert ist und Z für eine unter den Reaktionsbedingungen abspaltbare Gruppe, wie Halogen oder Phenylsulfonyloxy steht, oder

b) den Halogen-Cyanid-Austausch an einer Verbindung der Formel

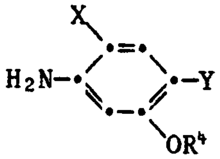

worin X und $R^4$ wie zuvor definiert sind und Y für Chlor, Brom oder Jod steht, mit CuCN, oder
c) die Reduktion eines Nitrobenzols der Formel

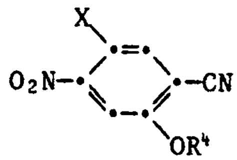

worin X und $R^4$ wie zuvor definiert sind.

**8.** Verfahren zur Herstellung von Anilinen der Formel

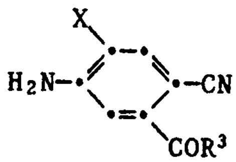

IIIc

worin
X Wasserstoff; oder Halogen; und
$R^3$ Hydroxy; ($C_1$-$C_8$)-Alkoxy; ($C_1$-$C_4$)-Alkoxy-($C_1$-$C_4$)-alkoxy; ($C_1$-$C_4$)-Alkylthio-($C_1$-$C_4$)-alkoxy; Mono- oder Di-($C_1$-$C_4$)-Alkylamino-($C_1$-$C_4$)-alkoxy; ($C_1$-$C_8$)-Cyanoalkoxy; ($C_3$-$C_8$)-Alkenyloxy; ($C_3$-$C_8$)-Halogenalkenyloxy; ($C_3$-$C_8$)-Alkinyloxy; ($C_3$-$C_7$)-Cycloalkyloxy; ($C_3$-$C_7$)-Cycloalkyl-($C_1$-$C_4$)-alkoxy; Halogen-($C_3$-$C_7$)-cycloalkoxy; Benzyloxy, das unsubstituiert oder am Phenylkern einfach durch Halogen, ($C_1$-$C_4$)-Alkyl, ($C_1$-$C_4$)-Alkoxy, ($C_1$-$C_4$)-Halogenalkyl, Cyan oder Nitro substituiert ist; Phenoxy; Halogenphenoxy; ($C_1$-$C_4$)-Alkylphenoxy; ($C_1$-$C_4$)-Alkyloxyphenoxy; ($C_1$-$C_4$)-Halogenalkylphenoxy, Cyanophenoxy; Nitrophenoxy; Phenylthio; Halogenphenylthio, ($C_1$-$C_4$)-Alkylphenylthio; ($C_1$-$C_4$)-Alkoxyphenylthio; ($C_1$-$C_4$)-Halogenalkylthio; Cyanophenylthio; Nitrophenylthio; die Salzgruppen -O-Na, -O-K, -O-$(Ca)_{0,5}$, -O-$(Mg)_{0,5}$ oder -O-$NH_4$; Amino; ($C_1$-$C_4$)-Alkylamino; Di-($C_1$-$C_4$)-alkylamino; ($C_2$-$C_4$)-Halogenalkylamino; Di-($C_2$-$C_4$)-halogenalkylamino; ($C_1$-$C_4$)-Hydroxyalkylamino; Di-($C_1$-$C_4$)-Hydroxyalkylamino; ($C_3$-$C_4$)-Alkenylamino; Diallylamino; -N-Pyrrolidino; -N-Piperidino; N-Morpholino; -N-Thiomorpholino; -N-Piperidazino; ($C_1$-$C_8$)-Alkylthio; ($C_3$-$C_8$)-Alkenylthio; Benzylthio; ($C_1$-$C_4$)-Alkylthio substituiert durch ($C_1$-$C_8$)-Alkoxycarbonyl, ($C_1$-$C_4$)-Alkoxy-($C_1$-$C_4$)alkoxycarbonyl, ($C_3$-$C_8$)-Alkenyloxycarbonyl, ($C_3$-$C_8$)-Alkinyloxycarbonyl, ($C_1$-$C_8$)-Alkylthiocarbonyl, ($C_3$-$C_8$)-Alkenylthiocarbonyl oder ($C_3$-$C_8$)-Alkinylthiocarbonyl; oder ($C_1$-$C_4$)-Alkoxy substituiert durch ($C_1$-$C_8$)-Alkoxycarbonyl, ($C_1$-$C_4$)-Alkoxy-($C_1$-$C_4$)-alkoxycarbonyl, ($C_3$-$C_8$)-Alkenyloxycarbonyl, ($C_3$-$C_8$)-Alkinyloxycarbonyl, ($C_1$-$C_8$)-Alkylthiocarbonyl, ($C_3$-$C_8$)-Alkenylthiocarbonyl, ($C_3$-$C_8$)-Alkinylthiocarbonyl, ($C_1$-$C_4$)-Alkylaminocarbonyl, Di-($C_1$-$C_4$)-Alkylaminocarbonyl oder Phenylaminocarbonyl, das unsubstituiert oder am Phenylkern einfach durch Halogen, ($C_1$-$C_4$)-Alkyl, ($C_1$-$C_4$)-Alkoxy, ($C_1$-$C_4$)-Halogenalkyl, Cyan oder Nitro substituiert ist:
bedeutet,
gekennzeichnet durch
a) den Halogen-Cyanid-Austausch an einer Verbindung der Formel

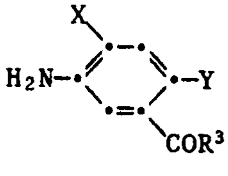

worin X und $R^3$ wie zuvor definiert sind und Y für Chlor, Brom oder Jod steht, mit CuCN oder

b) die Reduktion eines Nitrobenzols der Formel

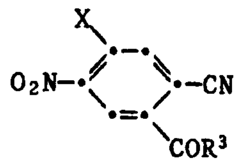

worin X und $R^3$ wie zuvor definiert sind.

**9.** Verfahren zur Herstellung von Verbindungen der Formel VII

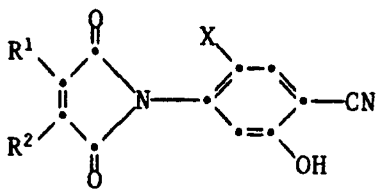

(VII),

worin

| | |
|---|---|
| $R^1$ | Wasserstoff; oder $(C_1$-$C_4)$-Alkyl; |
| $R^2$ | $(C_1$-$C_4)$-Alkyl; oder |
| $R^1$ und $R^2$ | gemeinsam eine $-(CH_2)_4$-Gruppe bedeuten, die bis zu zweifach durch $(C_1$-$C_4)$-Alkyl substituiert sein kann; |
| X | Wasserstoff; oder Halogen bedeutet, |

gekennzeichnet

a) durch die Umsetzung eines Furan-2,5-dions der Formel III mit einem 5-Hydroxyanilin

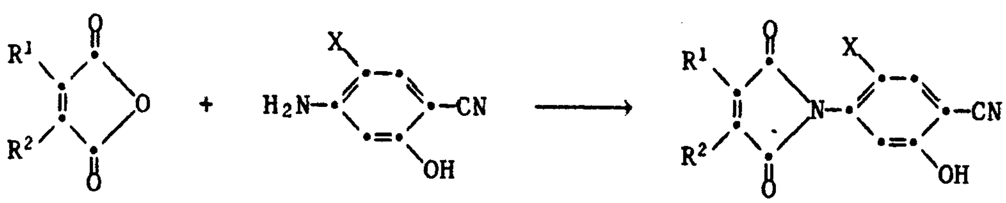

II

VII

wobei die Reste $R^1$, $R^2$ und X wie zuvor definiert sind oder

b) durch einen Halogen-Cyanid-Austausch, gemäss nachstehender Gleichung:

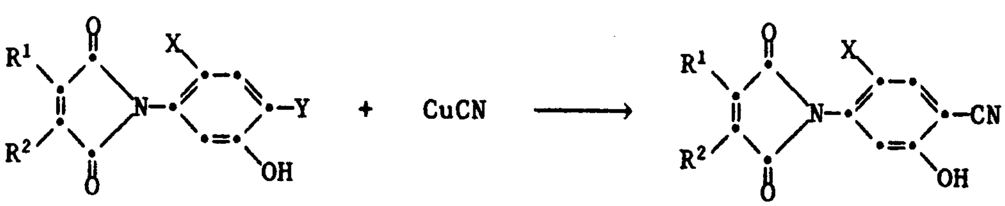

VII

worin $R^1$, $R^2$ und X wie zuvor definiert sind und Y Chlor, Brom oder Jod bedeutet.

**10.** Verfahren zur Herstellung eines herbiziden oder pflanzenwuchsregulatorischen Mittels, dadurch gekennzeichnet, dass man eine Verbindung der Formel I

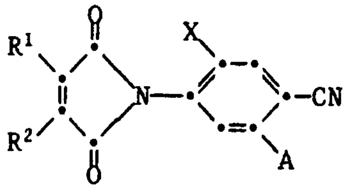

(I),

worin die Reste $R^1$, $R^2$, X und A die in den Ansprüchen 1 bis 4 gegebene Bedeutung haben können, mit den Hilfs- und/oder Trägerstoffen mischt.

**11.** Verfahren zur Bekämpfung unerwünschten Pflanzenwuchses, dadurch gekennzeichnet, dass man eine herbizid wirksame Menge einer Verbindung der Formel I

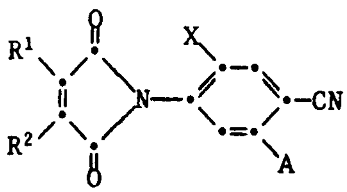

(I),

worin die Reste $R^1$, $R^2$, X und A wie in einem der Ansprüche 1 bis 4 definiert sind, auf die zu bekämpfende Pflanze oder deren Lebensraum einwirken lässt.

**12.** Verfahren zur Beeinflussung des Wachstums von Kulturpflanzen, dadurch gekennzeichnet, dass man eine pflanzenwuchsregulatorisch wirksame Menge einer Verbindung der Formel I

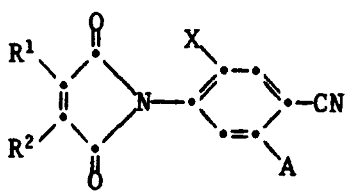

(I),

worin die Reste $R^1$, $R^2$, X und A wie in einem der Ansprüche 1 bis 4 definiert sind, auf die Kulturpflanze oder deren Lebensraum einwirken lässt.

**13.** Verfahren zur Saatgutbeizung, dadurch gekennzeichnet, dass man auf das Saatgut der Kulturpflanze eine herbizid wirksame Menge einer Verbindung der Formel I

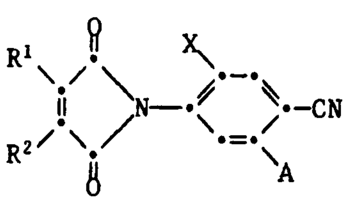

(I),

worin die Reste $R^1$, $R^2$, X und A wie in einem der Ansprüche 1 bis 4 definiert sind, aufbringt.

## Claims

**Claims for the following Contracting States : AT, BE, CH, DE, FR, GB, GR, IT, LI, LU, NL, SE**

**1.** A compound of formula I

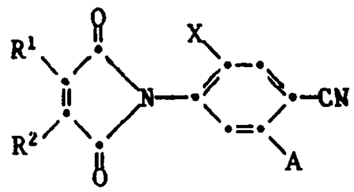

(I)

in which

$R^1$ is hydrogen; or $(C_1\text{-}C_4)$-alkyl;

$R^2$ is $(C_1\text{-}C_4)$-alkyl;

or $R^1$ and $R^2$, when taken together, are a $(CH_2)_4$ group which may be substituted by one or two $(C_1\text{-}C_4)$-alkyl groups;

X is hydrogen; or halogen;

A is

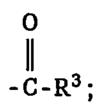

or $O\text{-}R^4$;

$R^3$ is hydroxyl; $(C_1\text{-}C_8)$-alkoxy; $(C_1\text{-}C_4)$-alkoxy-$(C_1\text{-}C_4)$-alkoxy; $(C_1\text{-}C_4)$-alkylthio-$(C_1\text{-}C_4)$-alkoxy; mono- or di-$(C_1\text{-}C_4)$-alkylamino-$(C_1\text{-}C_4)$-alkoxy; $(C_1\text{-}C_8)$-cyanoalkoxy; $(C_3\text{-}C_8)$-alkenyloxy; $(C_3\text{-}C_8)$-haloalkenyloxy; $(C_3\text{-}C_8)$-alkynyloxy; $(C_3\text{-}C_7)$-cycloalkoxy; $(C_3\text{-}C_7)$-cycloalkyl-$(C_1\text{-}C_4)$-alkoxy; halo-$(C_3\text{-}C_7)$-cycloalkoxy; benzyloxy which is unsubstituted or monosubstituted in the phenyl nucleus by halogen, $(C_1\text{-}C_4)$-alkyl,
$(C_1\text{-}C_4)$-alkoxy, $(C_1\text{-}C_4)$-haloalkyl, cyano or nitro; phenoxy; halophenoxy; $(C_1\text{-}C_4)$-alkylphenoxy; $(C_1\text{-}C_4)$-alkoxyphenoxy; $(C_1\text{-}C_4)$-haloalkylphenoxy; cyanophenoxy; nitrophenoxy; phenylthio; halophenylthio; $(C_1\text{-}C_4)$-alkylphenylthio; $(C_1\text{-}C_4)$-alkoxyphenylthio; $(C_1\text{-}C_4)$-haloalkylthio; cyanophenylthio; nitrophenylthio; the salt groups -O-Na, -O-K, $\text{-O-(Ca)}_{0.5}$, $\text{-O-(Mg)}_{0.5}$ or $\text{-O-NH}_4$; amino; $(C_1\text{-}C_4)$-alkylamino; di-$(C_1\text{-}C_4)$-alkylamino; $(C_2\text{-}C_4)$-haloalkylamino; di-$(C_2\text{-}C_4)$-haloalkylamino; $(C_1\text{-}C_4)$-hydroxyalkylamino; di-$(C_1\text{-}C_4)$-hydroxyalkylamino; $(C_3\text{-}C_4)$-alkenylamino; diallylamino; N-pyrrolidino; N-piperidino; N-morpholino; N-thiomorpholino; N-piperidazino; $(C_1\text{-}C_8)$-alkylthio; $(C_3\text{-}C_8)$-alkenylthio; benzylthio; $(C_1\text{-}C_4)$-alkylthio substituted by $(C_1\text{-}C_8)$-alkoxycarbonyl, $(C_1\text{-}C_4)$-alkoxy-$(C_1\text{-}C_4)$-alkoxycarbonyl, $(C_3\text{-}C_8)$-alkenyloxycarbonyl, $(C_3\text{-}C_8)$-alkynyloxycarbonyl, $(C_1\text{-}C_8)$-alkylthiocarbonyl, $(C_3\text{-}C_8)$-alkenylthiocarbonyl or $(C_3\text{-}C_8)$-alkynylthiocarbonyl; or
$(C_1\text{-}C_4)$-alkoxy substituted by $(C_1\text{-}C_8)$-alkoxycarbonyl, $(C_1\text{-}C_4)$-alkoxy-$(C_1\text{-}C_4)$-alkoxycarbonyl, $(C_3\text{-}C_8)$-alkenyloxycarbonyl, $(C_3\text{-}C_8)$-alkynyloxycarbonyl, $(C_1\text{-}C_8)$-alkylthiocarbonyl, $(C_3\text{-}C_8)$-alkenylthiocarbonyl, $(C_3\text{-}C_8)$alkynylthiocarbonyl, $(C_1\text{-}C_4)$-alkylcarbamoyl, di-$(C_1\text{-}C_4)$-alkylcarbamoyl or phenylcarbamoyl which is unsubstituted or monosubstituted in the phenyl nucleus by halogen, $(C_1\text{-}C_4)$-alkyl, $(C_1\text{-}C_4)$-alkoxy, $(C_1\text{-}C_4)$-haloalkyl cyano or nitro;

$R^4$ is $(C_1\text{-}C_8)$-alkyl; $(C_1\text{-}C_4)$-alkoxy-$(C_1\text{-}C_4)$-alkyl; $(C_1\text{-}C_4)$-alkylthio-$(C_1\text{-}C_4)$-alkyl; mono- or di-$(C_1\text{-}C_4)$-alkylamino-$(C_1\text{-}C_4)$-alkyl; $(C_1\text{-}C_8)$-haloalkyl; $(C_1\text{-}C_8)$-cyanoalkyl; $(C_3\text{-}C_8)$-alkenyl; $(C_3\text{-}C_8)$-haloalkenyl; $(C_3\text{-}C_8)$-alkynyl; $(C_3\text{-}C_7)$-cycloalkyl; $(C_3\text{-}C_7)$-cycloalkyl-$(C_1\text{-}C_4)$-alkyl; $(C_3\text{-}C_7)$-halocycloalkyl; $(C_1\text{-}C_8)$-alkylcarbonyl; allylcarbonyl; benzylcarbonyl which is unsubstituted or monosubstituted in the phenyl ring by halogen, $(C_1\text{-}C_4)$-alkyl, $(C_1\text{-}C_4)$-alkoxy, $(C_1\text{-}C_4)$-haloalkyl, cyano or nitro; $(C_3\text{-}C_7)$-cycloalkylcarbonyl; benzoyl which is unsubstituted or monosubstituted in the phenyl ring by halogen, $(C_1\text{-}C_4)$-alkyl, $(C_1\text{-}C_4)$-alkoxy, $(C_1\text{-}C_4)$-haloalkyl, cyano or nitro; furoyl; thienoyl; $(C_1\text{-}C_4)$-alkyl substituted by phenyl, halophenyl, $(C_1\text{-}C_4)$-alkylphenyl, $(C_1\text{-}C_4)$-alkoxyphenyl, $(C_1\text{-}C_4)$-haloalkylphenyl, $(C_1\text{-}C_4)$-haloalkoxyphenyl, nitrophenyl, cyanophenyl, $(C_1\text{-}C_8)$-alkoxycarbonyl, $(C_1\text{-}C_4)$-alkoxy-$(C_1\text{-}C_4)$-alkoxycarbonyl, $(C_3\text{-}C_8)$-al-

kenyloxycarbonyl, ($C_3$-$C_8$)-alkynyloxycarbonyl, ($C_1$-$C_8$)-alkylthiocarbonyl, ($C_3$-$C_8$)-alkenylthiocarbonyl, ($C_3$-$C_8$)-alkynylthiocarbonyl, carbamoyl, ($C_1$-$C_4$)-alkylcarbamoyl, di-($C_1$-$C_4$)-alkylcarbamoyl, hydroxyl, phenylcarbamoyl which is unsubstituted or monosubstituted in the phenyl ring by halogen, ($C_1$-$C_4$)-alkyl, ($C_1$-$C_4$)-alkoxy, cyano, nitro or ($C_1$-$C_4$)-haloalkyl; dioxolan-2-yl which is unsubstituted or substituted by one or two ($C_1$-$C_4$)-alkyl radicals; or 1,3-dioxan-2-yl which is unsubstituted or substituted by one or two ($C_1$-$C_4$)-alkyl radicals.

**2.** A compound of formula I according to claim 1 in which X is fluorine or hydrogen.

**3.** A compound of formula I according to claim 1 or 2 in which A is $OR^4$, and $R^4$ is isopropyl, or A is $COOR^3$ and $R^3$ is sec-butyl.

**4.** A compound of formula I according to claim 1 selected from the group consisting of 1-(4-cyano-2-fluoro-5-isopropoxyphenyl)-3,4-dimethyl-pyrrole-(1H)2,5-dione, 2-(5-carboxy-4-cyano-2-fluorophenyl)-4,5,6,7-tetrahydroisoindole-(2H)1,3-dione, 2-(4-cyano-2-fluoro-5-n-propoxycarbonylphenyl)-4,5,6,7-tetrahydroisoindole-(2H)1,3-dione, 2-(5-n-butoxycarbonyl-4-cyano-2-fluorophenyl)-4,5,6,7-tetrahy droisoindole-(2H)1,3-dione, 2-(4-cyano-2-fluoro-5-isopropoxyphenyl)-5-methyl-4,5,6,7-tetrahydroisoindole-(2H)1,3-dione, 1-(4-cyano-2-fluoro-5-isopropoxyphenyl)-3-ethyl-4-methyl-pyrrole-(1H)2,5-dione, 2-(4-cyano-2-fluoro-5-isopropoxyphenyl)-4,5,6,7-tetrahydroisoindole-(2H)1,3-dione, 2-(4-cyano-2-fluoro-5-isopropoxycarbonylphenyl)-4,5,6,7-tetrahydroisoindole-(2H)1,3-dione or 2-(5-sec-butoxycarbonyl-4-cyano-2-fluorophenyl)-4,5,6,7-tetrahydroisoindole-(2H)1,3-dione.

**5.** A process for the preparation of a compound of formula I according to claim 1, which comprises

a) reacting a furan-2,5-dione of formula II with an aniline III

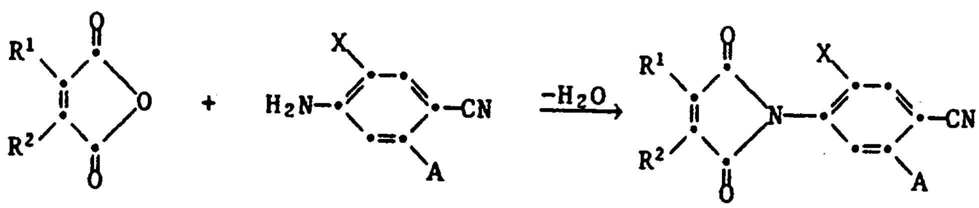


II III I

in which $R^1$, $R^2$, X and A are as previously defined; or

b) starting from a compound of formula IV

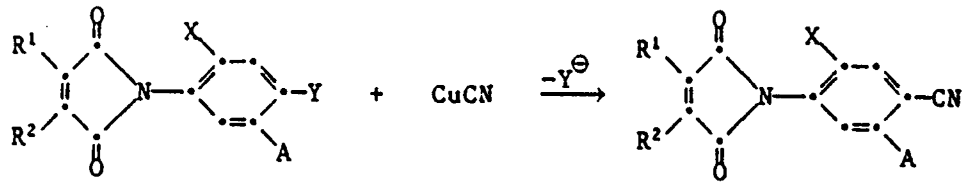


IV

in which $R^1$, $R^2$, X and A are as previously defined and Y is chlorine, bromine or iodine, replacing halogen with cyanide by reaction with CuCN.

**6.** A process for the preparation of a compound of formula I according to claim 1 in which A is $COR^3$, which comprises

a) condensing a compound of formula V, in which $R^1$, $R^2$ and X are as previously defined

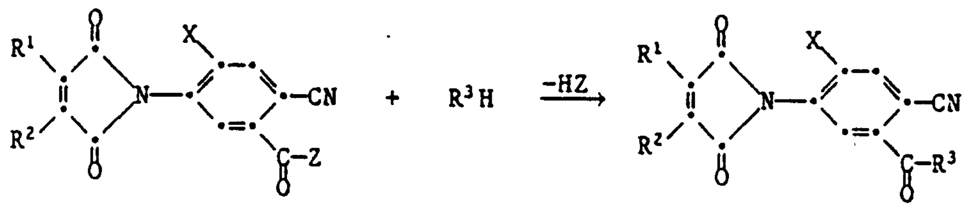


V VI I

and Z is a group which can be replaced under the reaction conditions, such as halogen or $(C_1$-$C_4)$-alkylcarbonyloxy, with a compound of formula VI, in which $R^3$ is as previously defined; or
b) esterifying a carboxylic acid of formula Ia', in which $R^1$, $R^2$ and X are as previously defined, with an alcohol or thioalcohol of formula VI

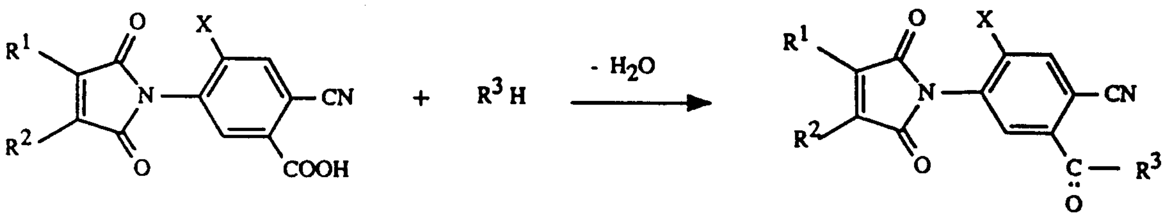


Ia' VI Ia

in which
$R^3$ is $(C_1$-$C_8)$-alkoxy; $(C_1$-$C_4)$-alkoxy-$(C_1$-$C_4)$-alkoxy; $(C_1$-$C_4)$-alkylthio-$(C_1$-$C_4)$-alkoxy; mono- or di-$(C_1$-$C_4)$-alkylamino-$(C_1$-$C_4)$-alkoxy; $(C_1$-$C_8)$-cyanoalkoxy; $(C_3$-$C_8)$-alkenyloxy; $(C_3$-$C_8)$-haloalkenyloxy; $(C_3$-$C_8)$-alkynyloxy; $(C_3$-$C_7)$-cycloalkoxy; $(C_3$-$C_7)$-cycloalkyl-$(C_1$-$C_4)$-alkoxy; halo-$(C_3$-$C_7)$-cycloalkoxy; benzyloxy which is unsubstituted or monosubstituted in the phenyl nucleus by halogen, $(C_1$-$C_4)$-alkyl, $(C_1$-$C_4)$-alkoxy, $(C_1$-$C_4)$-haloalkyl, cyano or nitro; phenoxy; halophenoxy; $(C_1$-$C_4)$-alkylphenoxy; $(C_1$-$C_4)$-alkoxyphenoxy; haloalkylphenoxy; cyanophenoxy; nitrophenoxy; phenylthio; halophenylthio; $(C_1$-$C_4)$-alkylphenylthio; $(C_1$-$C_4)$-alkoxyphenylthio; $(C_1$-$C_4)$-haloalkylthio; cyanophenylthio; nitrophenylthio; $(C_1$-$C_8)$-alkylthio; $(C_3$-$C_8)$-alkenylthio; benzylthio; $(C_1$-$C_4)$-alkylthio substituted by $(C_1$-$C_8)$-alkoxycarbonyl, $(C_1$-$C_4)$-alkoxy-$(C_1$-$C_4)$-alkoxycarbonyl, $(C_3$-$C_8)$-alkenyloxycarbonyl, $(C_3$-$C_8)$-alkynyloxycarbonyl, $(C_1$-$C_8)$-alkylthiocarbonylor $(C_3$-$C_8)$-alkylthiocarbonyl; or $(C_1$-$C_4)$-alkoxy substituted by $(C_1$-$C_8)$-alkoxycarbonyl, $(C_1$-$C_4)$-alkoxy-$(C_1$-$C_4)$-alkoxycarbonyl, $(C_3$-$C_8)$-alkenyloxycarbonyl, $(C_3$-$C_8)$-alkynyloxycarbonyl, $(C_1$-$C_8)$-alkylthiocarbonyl, $(C_3$-$C_8)$-alkenylthiocarbonyl, $(C_3$-$C_8)$-alkynylthiocarbonyl, $(C_1$-$C_4)$-alkylcarbamoyl, di-$(C_1$-$C_4)$-alkylcarbamoyl or phenylcarbamoyl which is unsubstituted or monosubstituted in the phenyl nucleus by halogen, $(C_1$-$C_4)$-alkyl, $(C_1$-$C_4)$-alkoxy, $(C_1$-$C_4)$-halogen, cyano or nitro,

to give an ester of formula Ia, in which $R^1$, $R^2$, $R^3$ and X are as previously defined.

**7.** A process for the preparation of a compound of formula I according to claim 1, in which A is $OR^4$, which process comprises etherifying a phenol of formula VII, in which $R^1$, $R^2$ and X are as previously defined

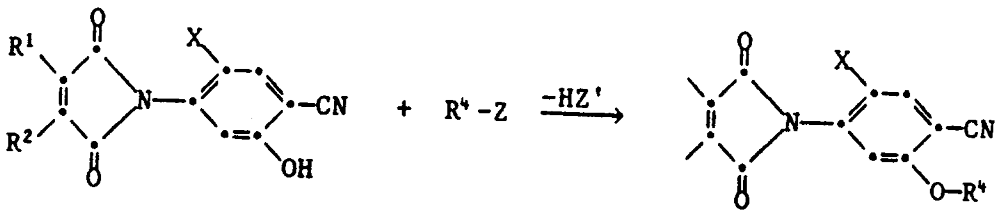

VII VIII Ib

with a compound of formula VIII, in which $R^4$ is as previously defined and Z is a group which can be removed under the reaction conditions, such as halogen or phenylsulfonyloxy or alkylated phenylsulfonyloxy.

**8.** An aniline of formula IIIb

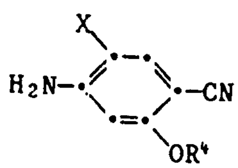

IIIb

in which
X is hydrogen; or halogen; and
$R^4$ is $(C_1-C_8)$-alkyl; $(C_1-C_4)$-alkoxy-$(C_1-C_4)$-alkyl; $(C_1-C_4)$-alkylthio-$(C_1-C_4)$-alkyl; mono- or di-$(C_1-C_4)$-alkylamino-$(C_1-C_4)$-alkyl; $(C_1-C_8)$-haloalkyl; $(C_1-C_8)$-cyanoalkyl; $(C_3-C_8)$-alkenyl; $(C_3-C_8)$-haloalkenyl; $(C_3-C_8)$-alkynyl; $(C_3-C_7)$-cycloalkyl; $(C_3-C_7)$-cycloalkyl-$(C_1-C_4)$-alkyl; $(C_3-C_7)$-halocycloalkyl; $(C_1-C_8)$-alkylcarbonyl; allylcarbonyl; benzylcarbonyl which is unsubstituted or monosubstituted in the phenyl ring by halogen, $(C_1-C_4)$-alkyl, $(C_1-C_4)$-alkoxy, $(C_1-C_4)$-haloalkyl, cyano or nitro; $(C_3-C_7)$-cycloalkylcarbonyl; benzoyl which is unsubstituted or monosubstituted in the phenyl ring by halogen, $(C_1-C_4)$-alkyl, $(C_1-C_4)$-alkoxy, $(C_1-C_4)$-haloalkyl, cyano or nitro; furoyl; thienoyl; $(C_1-C_4)$-alkyl substituted by phenyl, halophenyl, $(C_1-C_4)$-alkylphenyl, $(C_1-C_4)$-alkoxyphenyl, $(C_1-C_4)$-haloalkylphenyl, $(C_1-C_4)$-haloalkoxyphenyl, nitrophenyl, cyanophenyl, $(C_1-C_8)$-alkoxycarbonyl, $(C_1-C_4)$-alkoxy-$(C_1-C_4)$-alkoxycarbonyl, $(C_3-C_8)$-alkenyloxycarbonyl, $(C_3-C_8)$-alkynyloxycarbonyl, $(C_1-C_8)$-alkylthiocarbonyl, $(C_3-C_8)$-alkenylthiocarbonyl, $(C_3-C_8)$-alkynylthiocarbonyl, carbamoyl, $(C_1-C_4)$-alkylcarbamoyl, di-$(C_1-C_4)$-alkylcarbamoyl, hydroxyl, phenylcarbamoyl which is unsubstituted or monosubstituted in the phenyl ring by halogen, $(C_1-C_4)$-alkyl, $(C_1-C_4)$-alkoxy, cyano, nitro or $(C_1-C_4)$-haloalkyl; dioxolan-2-yl which is unsubstituted or substituted by one or two $(C_1-C_4)$-alkyl radicals; or 1,3-dioxan-2-yl which is unsubstituted or substituted by one or two $(C_1-C_4)$-alkyl radicals,
with the exception of 2-methoxy-4-amino-5-chlorobenzonitrile.

**9.** An aniline of formula IIIc

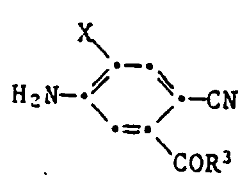

IIIC

in which
X is hydrogen; or halogen; and
$R^3$ is hydroxyl; $(C_1-C_8)$-alkoxy; $(C_1-C_4)$-alkoxy-$(C_1-C_4)$-alkoxy; $(C_1-C_4)$-alkylthio-$(C_1-C_4)$-alkoxy; mono- or di-$(C_1-C_4)$-alkylamino-$(C_1-C_4)$-alkoxy; $(C_1-C_8)$-cyanoalkoxy; $(C_3-C_8)$-alkenyloxy; $(C_3$-

$C_8$)-haloalkenyloxy; ($C_3$-$C_8$)-alkynyloxy; ($C_3$-$C_7$)-cycloalkoxy; ($C_3$-$C_7$)-cycloalkyl-($C_1$-$C_4$)-alkoxy; halo-($C_3$-$C_7$)-cycloalkoxy; benzyloxy which is unsubstituted or monosubstituted in the phenyl nucleus by halogen, ($C_1$-$C_4$)-alkyl,
($C_1$-$C_4$)-alkoxy, ($C_1$-$C_4$)-haloalkyl, cyano or nitro; phenoxy; halophenoxy; ($C_1$-$C_4$)-alkylphenoxy; ($C_1$-$C_4$)-alkoxyphenoxy; ($C_1$-$C_4$)-haloalkylphenoxy; cyanophenoxy; nitrophenoxy; phenylthio; halophenylthio; ($C_1$-$C_4$)-alkylphenylthio; ($C_1$-$C_4$)-alkoxyphenylthio; ($C_1$-$C_4$)-haloalkylthio; cyanophenylthio; nitrophenylthio; the salt groups -O-Na, -O-K, -O-$(Ca)_{0.5}$, -O-$(Mg)_{0.5}$ or O-$NH_4$; amino; ($C_1$-$C_4$)-alkylamino; di-($C_1$-$C_4$)-alkylamino; ($C_2$-$C_4$)-halo-alkylamino; di-($C_2$-$C_4$)-haloalkylamino; ($C_1$-$C_4$)-hydroxyalkylamino; di-($C_1$-$C_4$)-hydroxyalkylamino; ($C_3$-$C_4$)-alkenylamino; diallylamino; N-pyrrolidino; N-piperidino; N-morpholino; N-thiomorpholino; N-piperidazino; ($C_1$-$C_8$)-alkylthio; ($C_3$-$C_8$)-alkenylthio; benzylthio; ($C_1$-$C_4$)-alkylthio substituted by ($C_1$-$C_8$)-alkoxycarbonyl, ($C_1$-$C_4$)-alkoxy-($C_1$-$C_4$)-alkoxycarbonyl, ($C_3$-$C_8$)-alkenyloxycarbonyl, ($C_3$-$C_8$)-alkynyloxycarbonyl, ($C_1$-$C_8$)-alkylthiocarbonyl, ($C_3$-$C_8$)-alkenylthiocarbonyl or ($C_3$-$C_8$)-alkynylthiocarbonyl; or ($C_1$-$C_4$)-alkoxy substituted by ($C_1$-$C_8$)-alkoxycarbonyl, ($C_1$-$C_4$)-alkoxy-($C_1$-$C_4$)-alkoxy-carbonyl, ($C_3$-$C_8$)-alkenyloxycarbonyl, ($C_3$-$C_8$)-alkynyloxycarbonyl, ($C_1$-$C_8$)-alkylthiocarbonyl, ($C_3$-$C_8$)-alkenylthiocarbonyl, ($C_3$-$C_8$)-alkynylthiocarbonyl, ($C_1$-$C_4$)-alkylcarbamoyl, di-($C_1$-$C_4$)-alkylcarbamoyl or phenylcarbamoyl which is unsubstituted or monosubstituted in the phenyl nucleus by halogen, ($C_1$-$C_4$)-alkyl, ($C_1$-$C_4$)-alkoxy, ($C_1$-$C_4$)-haloalkyl, cyano or nitro.

**10.** A compound of formula VII

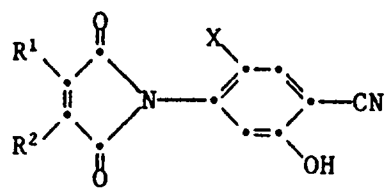

(VII)

in which
$R^1$ is hydrogen; or ($C_1$-$C_4$)-alkyl;
$R^2$ is ($C_1$-$C_4$)-alkyl; or
$R^1$ and $R^2$, when taken together, are a $(CH_2)_4$ group which can be substituted by 1 or 2 ($C_1$-$C_4$)-alkyl groups; and
X is hydrogen; or halogen.

**11.** A herbicidal or growth regulating composition which contains a compound of formula I according to any one of claims 1 to 4, together with further adjuvants and/or carriers.

**12.** A method of controlling undesirable plant growth, which comprises treating the plants to be controlled or their locus with a compound of formula I according to any one of claims 1 to 4 or a herbicidal composition according to claim 11.

**13.** Seeds containing a herbicidally effective amount of a compound of formula I according to any one of claims 1 to 4.

**14.** A method of defoliating useful plants, which comprises treating the plants to be defoliated with a compound of formula I according to any one of claims 1 to 4 or a growth regulating composition according to claim 11.

**Claims for the following Contracting State : ES**

**1.** A process for the preparation of a compound of formula I

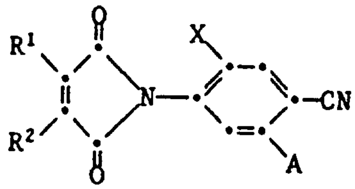

(I)

in which

$R^1$ is hydrogen; or $(C_1-C_4)$-alkyl;

$R^2$ is $(C_1-C_4)$-alkyl; or

$R^1$ and $R^2$, when taken together, are a $(CH_2)_4$ group which may be substituted by one or two $(C_1-C_4)$-alkyl groups;

X is hydrogen; or halogen;

A is

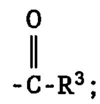

or $O-R^4$;

$R^3$ is hydroxyl; $(C_1-C_8)$-alkoxy; $(C_1-C_4)$-alkoxy-$(C_1-C_4)$-alkoxy; $(C_1-C_4)$-alkylthio-$(C_1-C_4)$-alkoxy; mono- or di-$(C_1-C_4)$-alkylamino-$(C_1-C_4)$-alkoxy; $(C_1-C_8)$-cyanoalkoxy; $(C_3-C_8)$-alkenyloxy; $(C_3-C_8)$-haloalkenyloxy; $(C_3-C_8)$-alkynyloxy; $(C_3-C_7)$-cycloalkoxy; $(C_3-C_7)$-cycloalkyl-$(C_1-C_4)$-alkoxy; halo-$(C_3-C_7)$-cycloalkoxy; benzyloxy which is unsubstituted or monosubstituted in the phenyl nucleus by halogen, $(C_1-C_4)$-alkyl, $(C_1-C_4)$-alkoxy, $(C_1-C_4)$-haloalkyl, cyano or nitro; phenoxy; halophenoxy; $(C_1-C_4)$-alkylphenoxy; $(C_1-C_4)$-alkoxyphenoxy; $(C_1-C_4)$-haloalkylphenoxy; cyanophenoxy; nitrophenoxy; phenylthio; halophenylthio; $(C_1-C_4)$-alkylphenylthio; $(C_1-C_4)$-alkoxyphenylthio; $(C_1-C_4)$-haloalkylthio; cyanophenylthio; nitrophenylthio; the salt groups -O-Na, -O-K, $-O-(Ca)_{0.5}$, $-O-(Mg)_{0.5}$ or $O-NH_4$; amino; $(C_1-C_4)$-alkylamino; di-$(C_1-C_4)$-alkylamino; $(C_2-C_4)$-haloalkylamino; di-$(C_2-C_4)$-haloalkylamino; $(C_1-C_4)$-hydroxyalkylamino; di-$(C_1-C_4)$-hydroxyalkylamino; $(C_3-C_4)$-alkenylamino; diallylamino; N-pyrrolidino; N-piperidino; N-morpholino; N-thiomorpholino; N-piperidazino; $(C_1-C_8)$-alkylthio; $(C_3-C_8)$-alkenylthio; benzylthio; $(C_1-C_4)$-alkylthio substituted by $(C_1-C_8)$-alkoxycarbonyl, $(C_1-C_4)$-alkoxy-$(C_1-C_4)$-alkoxycarbonyl, $(C_3-C_8)$-alkenyloxycarbonyl, $(C_3-C_8)$-alkynyloxycarbonyl, $(C_1-C_8)$-alkylthiocarbonyl, $(C_3-C_8)$-alkenylthiocarbonyl or $(C_3-C_8)$-alkynylthiocarbonyl; or $(C_1-C_4)$-alkoxy substituted by $(C_1-C_8)$-alkoxycarbonyl, $(C_1-C_4)$-alkoxy-$(C_1-C_4)$-alkoxycarbonyl, $(C_3-C_8)$-alkenyloxycarbonyl, $(C_3-C_8)$-alkynyloxycarbonyl, $(C_1-C_8)$-alkylthiocarbonyl, $(C_3-C_8)$-alkenylthiocarbonyl, $(C_3-C_8)$-alkynylthiocarbonyl, $(C_1-C_4)$-alkylcarbamoyl, di-$(C_1-C_4)$-alkylcarbamoyl or phenylcarbamoyl which is unsubstituted or monosubstituted in the phenyl nucleus by halogen, $(C_1-C_4)$-alkyl, $(C_1-C_4)$-alkoxy, $(C_1-C_4)$-haloalkyl, cyano or nitro;

$R^4$ is $(C_1-C_8)$-alkyl; $(C_1-C_4)$-alkoxy-$(C_1-C_4)$-alkyl; $(C_1-C_4)$-alkylthio-$(C_1-C_4)$-alkyl; mono- or di-$(C_1-C_4)$-alkylamino-$(C_1-C_4)$-alkyl; $(C_1-C_8)$-haloalkyl; $(C_1-C_8)$-cyanoalkyl; $(C_3-C_8)$-alkenyl; $(C_3-C_8)$-haloalkenyl; $(C_3-C_8)$-alkynyl; $(C_3-C_7)$-cycloalkyl; $(C_3-C_7)$-cycloalkyl-$(C_1-C_4)$-alkyl; $(C_3-C_7)$-halocycloalkyl; $(C_1-C_8)$-alkylcarbonyl; allylcarbonyl; benzylcarbonyl which is unsubstituted or monosubstituted in the phenyl ring by halogen, $(C_1-C_4)$-alkyl, $(C_1-C_4)$-alkoxy, $(C_1-C_4)$-haloalkyl, cyano or nitro; $(C_3-C_7)$-cycloalkylcarbonyl; benzoyl which is unsubstituted or monosubstituted in the phenyl ring by halogen, $(C_1-C_4)$-alkyl, $(C_1-C_4)$-alkoxy, $(C_1-C_4)$-haloalkyl, cyano or nitro; furoyl; thienoyl; $(C_1-C_4)$-alkyl substituted by phenyl, halophenyl, $(C_1-C_4)$-alkylphenyl, $(C_1-C_4)$-alkoxyphenyl, $(C_1-C_4)$-haloalkylphenyl, $(C_1-C_4)$-haloalkoxyphenyl, nitrophenyl, cyanophenyl, $(C_1-C_8)$-alkoxycarbonyl, $(C_1-C_4)$-alkoxy-$(C_1-C_4)$-alkoxycarbonyl, $(C_3-C_8)$-alkenyloxycarbonyl, $(C_3-C_8)$-alkynyloxycarbonyl, $(C_1-C_8)$-alkylthiocarbonyl, $(C_3-C_8)$-alkenyl-

thiocarbonyl, ($C_3$-$C_8$)-alkynylthiocarbonyl, carbamoyl, ($C_1$-$C_4$)-alkylcarbamoyl, di-($C_1$-$C_4$)-alkylcarbamoyl, hydroxyl, phenylcarbamoyl which is unsubstituted or monosubstituted in the phenyl ring by halogen, ($C_1$-$C_4$)-alkyl, ($C_1$-$C_4$)-alkoxy, cyano, nitro or ($C_1$-$C_4$)-haloalkyl; dioxolan-2-yl which is unsubstituted or substituted by one or two ($C_1$-$C_4$)-alkyl radicals; or 1,3-dioxan-2-yl which is unsubstituted or substituted by one or two ($C_1$-$C_4$)-alkyl radicals;

which comprises

a) reacting a furan-2,5-dione of formula II with an aniline III

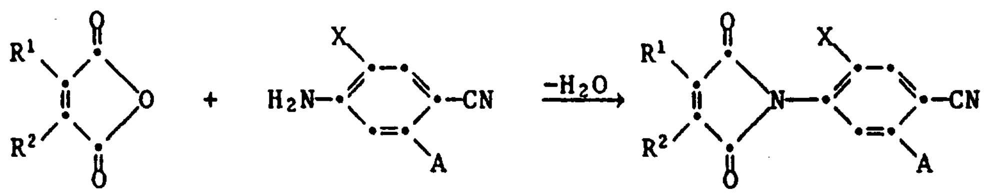


II III I

in which $R^1$, $R^2$, X and A are as previously defined; or

b) starting from a compound of formula IV

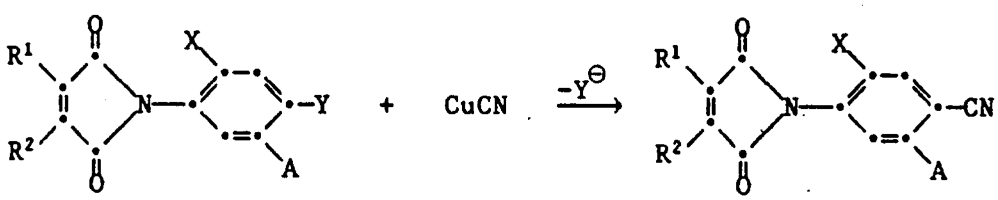


IV

in which $R^1$, $R^2$, X and A are as previously defined and Y is chlorine, bromine or iodine, replacing halogen with cyanide by reaction with CuCN.

**2.** A process according to claim 1 for the preparation of a compound of formula I

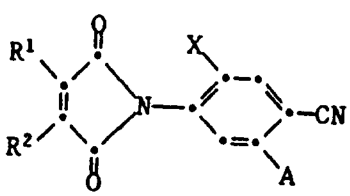

(I)

in which

$R^1$ is hydrogen; or ($C_1$-$C_4$)-alkyl;

$R^2$ ($C_1$-$C_4$)-alkyl; or

$R^1$ and $R^2$, when taken together, are a $(CH_2)_4$ group which may be unsubstituted or monosubstituted by ($C_1$-$C_4$)-alkyl;

X is hydrogen; fluorine; chlorine; or bromine;

A is

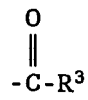 ;

or O-$R^4$;

$R^3$ is hydroxyl; ($C_1$-$C_5$)-alkoxy; ($C_1$-$C_4$)-alkoxy-($C_1$-$C_4$)-alkoxy; ($C_1$-$C_4$)-alkylthio-($C_1$-$C_4$)-alkoxy; mono- or di-($C_1$-$C_4$)-alkylamino-($C_1$-$C_4$)-alkoxy; ($C_1$-$C_4$)-cyanoalkoxy; ($C_3$-$C_5$)-haloalkenyloxy; ($C_3$-$C_5$)-alkynyloxy; ($C_3$-$C_5$)-alkenyloxy; ($C_3$-$C_6$)-cycloalkylmethyloxy; the salt groups -O-Na, -O-K, -O-$(Ca)_{0.5}$, -O-$(Mg)_{0.5}$ or -O-$NH_4$; amino; di-($C_1$-$C_4$)-alkylamino; diallylamino; benzyloxy; N-piperidino; N-morpholino; N-thiomorpholino; ($C_1$-$C_4$)-alkylthio; ($C_1$-$C_4$)-alkylthio, substituted by ($C_1$-$C_4$)-alkoxycarbonyl; ($C_1$-$C_4$)-alkoxy, substituted by ($C_1$-$C_4$)-alkoxycarbonyl, ($C_1$-$C_4$)-alkylthiocarbonyl, ($C_1$-$C_4$)-alkylcarbamoyl or di-($C_1$-$C_4$)-alkylcarbamoyl;

$R^4$ is ($C_1$-$C_5$)-alkyl; ($C_1$-$C_4$)-alkoxy ($C_1$-$C_4$)-alkyl; ($C_1$-$C_4$)-alkylthio-($C_1$-$C_4$)-alkyl; ($C_1$-$C_4$)-haloalkyl; ($C_3$-$C_5$)-alkenyl; ($C_3$-$C_5$)-haloalkenyl; ($C_3$-$C_5$)-alkynyl; cyclohexylmethyl; ($C_1$-$C_4$)-alkylcarbonyl; benzoyl which is unsubstituted or monosubstituted in the phenyl ring by fluorine, chlorine, ($C_1$-$C_4$)-alkyl, ($C_1$-$C_4$)-alkoxy, cyano or nitro; ($C_1$-$C_4$)-alkyl monosubstituted by phenyl, ($C_1$-$C_4$)-alkoxycarbonyl, ($C_1$-$C_4$)-alkylthiocarbonyl, carbamoyl, di-($C_1$-$C_4$)-alkylcarbamoyl or hydroxyl.

**3.** A process according to claim 1 for the preparation of a compound of formula I

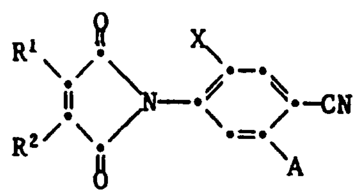 (I)

in which X is fluorine or hydrogen and $R^1$, $R^2$ and A have the meanings given in claim 1 or 2.

**4.** A process according to claim 1 for the preparation of a compound of formula I

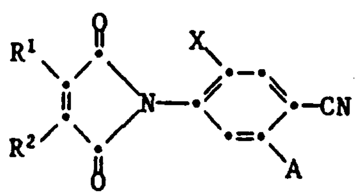 (I)

in which
A is $OR^4$ and $R^4$ is isopropyl or A is $COOR^3$ and $R^3$ is sec-butyl and $R^1$, $R^2$ and X have the meanings given in any one of claims 1 to 3.

**5.** A process for the preparation of a compound of formula I

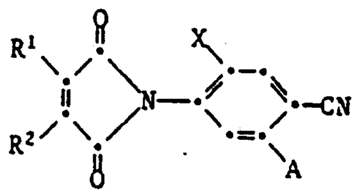

(I)

in which
A is $COR^3$ and $R^1$, $R^2$, $R^3$, and X have the meanings given in any one of claims 1 to 4, which comprises
a) condensing a compound of formula V, in which $R^1$, $R^2$ and X are as previously defined

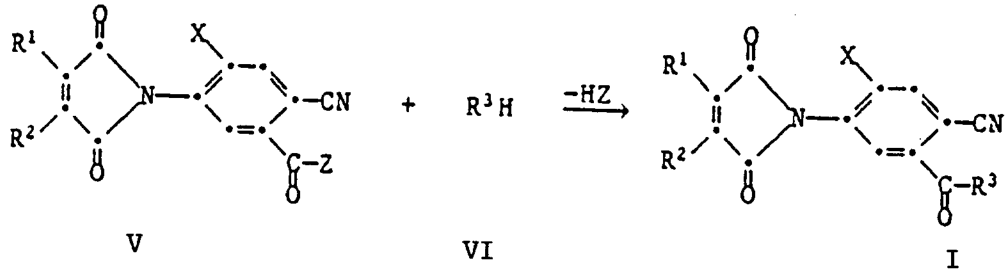

V VI I

and Z is a group which can be replaced under the reaction conditions, such as halogen or $(C_1-C_4)$-alkylcarbonyloxy, with a compound of formula VI, in which $R^3$ is as previously defined; or
b) esterifying a carboxylic acid of formula Ia', in which $R^1$, $R^2$ and X are as previously defined, with an alcohol or thioalcohol of formula VI

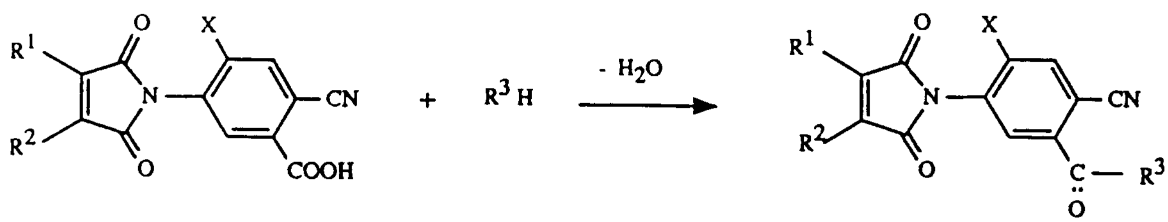


Ia' VI Ia

in which
$R^3$ is $(C_1-C_8)$-alkoxy; $(C_1-C_4)$-alkoxy-$(C_1-C_4)$-alkoxy; $(C_1-C_4)$-alkylthio-$(C_1-C_4)$-alkoxy; mono- or di-$(C_1-C_4)$-alkylamino-$(C_1-C_4)$-alkoxy; $(C_1-C_8)$-cyanoalkoxy; $(C_3-C_8)$-alkenyloxy; $(C_3-C_8)$-haloalkenyloxy; $(C_3-C_8)$-alkynyloxy; $(C_3-C_7)$-cycloalkoxy; $(C_3-C_7)$-cycloalkyl-$(C_1-C_4)$-alkoxy; halo-$(C_3-C_7)$-cycloalkoxy; benzyloxy which is unsubstituted or monosubstituted in the phenyl nucleus by halogen, $(C_1-C_4)$-alkyl, $(C_1-C_4)$-alkoxy, $(C_1-C_4)$-haloalkyl, cyano or nitro; phenoxy; halophenoxy; $(C_1-C_4)$-alkylphenoxy; $(C_1-C_4)$-alkoxyphenoxy; haloalkylphenoxy; cyanophenoxy; nitrophenoxy; phenylthio; halophenylthio; $(C_1-C_4)$-alkylphenylthio; $(C_1-C_4)$-alkoxyphenylthio; $(C_1-C_4)$-haloalkylthio; cyanophenylthio; nitrophenylthio; $(C_1-C_8)$-alkylthio; $(C_3-C_8)$-alkenylthio; benzylthio; $(C_1-C_4)$-alkylthio substituted by $(C_1-C_8)$-alkoxycarbonyl, $(C_1-C_4)$-alkoxy-$(C_1-C_4)$-alkoxycarbonyl, $(C_3-C_8)$-alkenyloxycarbonyl, $(C_3-C_8)$-alkynyloxycarbonyl, $(C_1-C_8)$-alkylthiocarbonylor $(C_3-C_8)$-alkylnylthiocarbonyl; or $(C_1-C_4)$-alkoxy substituted by $(C_1-C_8)$-alkoxycarbonyl, $(C_1-C_4)$-alkoxy-$(C_1-C_4)$-alkoxycarbonyl, $(C_3-C_8)$-alkenyloxycarbonyl, $(C_3-C_8)$-alkynyloxycarbonyl, $(C_1-C_8)$-alkylthiocarbonyl, $(C_3-C_8)$-alkenylthiocarbonyl, $(C_3-C_8)$-alkynylthiocarbonyl, $(C_1-C_4)$-alkylcarbamoyl, di-$(C_1-C_4)$-alkylcarbamoyl or phenylcarbamoyl which is unsubstituted or monosubstituted in the phenyl nucleus by halogen, $(C_1-C_4)$-alkyl, $(C_1-C_4)$-alkoxy, $(C_1-C_4)$-halogen, cyano or nitro,

to give an ester of formula Ia, in which $R^1$, $R^2$, $R^3$ and X are as previously defined.

**6.** A process for the preparation of a compound of formula I

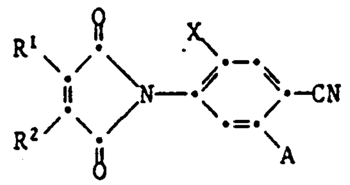

(I)

in which A is $OR^4$ and $R^1$, $R^2$, $R^4$ and X have the meanings given in any one of claims 1 to 4, which process comprises etherifying a phenol of formula VII, in which $R^1$, $R^2$ and X are as previously defined

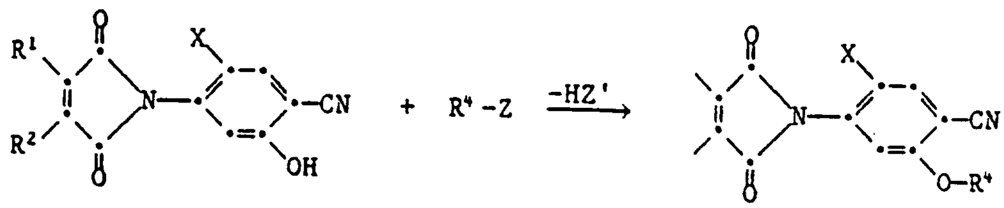


VII VIII Ib

with a compound of formula VIII, in which $R^4$ is as previously defined and Z is a group which can be removed under the reaction conditions, such as halogen or phenylsulfonyloxy or alkylated phenylsulfonyloxy.

**7.** A process for the preparation of an aniline of formula IIIb

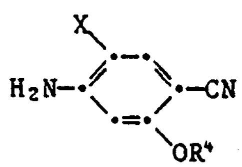

IIIb

in which

X is hydrogen; or halogen; and

$R^4$ is $(C_1$-$C_8)$-alkyl; $(C_1$-$C_4)$-alkoxy-$(C_1$-$C_4)$-alkyl; $(C_1$-$C_4)$-alkylthio-$(C_1$-$C_4)$-alkyl; mono- or di-$(C_1$-$C_4)$-alkylamino-$(C_1$-$C_4)$-alkyl; $(C_1$-$C_8)$-haloalkyl; $(C_1$-$C_8)$-cyanoalkyl; $(C_3$-$C_8)$-alkenyl; $(C_3$-$C_8)$-haloalkenyl; $(C_3$-$C_8)$-alkynyl; $(C_3$-$C_7)$-cycloalkyl; $(C_3$-$C_7)$-cycloalkyl-$(C_1$-$C_4)$-alkyl; $(C_3$-$C_7)$-halocycloalkyl; $(C_1$-$C_8)$-alkylcarbonyl; allylcarbonyl; benzylcarbonyl which is unsubstituted or monosubstituted in the phenyl ring by halogen, $(C_1$-$C_4)$-alkyl, $(C_1$-$C_4)$-alkoxy, $(C_1$-$C_4)$-haloalkyl, cyano or nitro; $(C_3$-$C_7)$-cycloalkylcarbonyl; benzoyl which is unsubstituted or monosubstituted in the phenyl ring by halogen, $(C_1$-$C_4)$-alkyl, $(C_1$-$C_4)$-alkoxy, $(C_1$-$C_4)$-haloalkyl, cyano or nitro; furoyl; thienoyl; $(C_1$-$C_4)$-alkyl substituted by phenyl, halophenyl, $(C_1$-$C_4)$-alkylphenyl, $(C_1$-$C_4)$-alkoxyphenyl, $(C_1$-$C_4)$-haloalkylphenyl, $(C_1$-$C_4)$-haloalkoxyphenyl, nitrophenyl, cyanophenyl, $(C_1$-$C_8)$-alkoxycarbonyl, $(C_1$-$C_4)$-alkoxy-$(C_1$-$C_4)$-alkoxycarbonyl, $(C_3$-$C_8)$-alkenyloxycarbonyl, $(C_3$-$C_8)$-alkynyloxycarbonyl, $(C_1$-$C_8)$-alkylthiocarbonyl, $(C_3$-$C_8)$-alkenylthiocarbonyl, $(C_3$-$C_8)$-alkylthiocarbonyl, carbamoyl, $(C_1$-$C_4)$-alkylcarbamoyl, di-$(C_1$-$C_4)$-alkylcarbamoyl, hydroxyl, phenylcarbamoyl which is unsubstituted or monosubstituted in the phenyl ring by halogen, $(C_1$-$C_4)$-alkyl, $(C_1$-$C_4)$-alkoxy, cyano, nitro or $(C_1$-$C_4)$-haloalkyl; dioxolan-2-yl which is unsubstituted or substituted by one or two $(C_1$-$C_4)$-alkyl radicals; or 1,3-dioxan-2-yl which is unsubstituted or substituted by one or two $(C_1$-$C_4)$-alkyl radicals,

with the exception of 2-methoxy-4-amino-5-chlorobenzonitrile,

which comprises

a) etherifying a phenol of formula

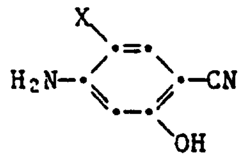

in which X is as previously defined with a compound of formula VIII

$R^4$-Z (VIII)

in which $R^4$ is as previously defined and Z is a group which can be removed under the reaction conditions, such as halogen or phenylsulfonyloxy, or

b) in a compound of formula

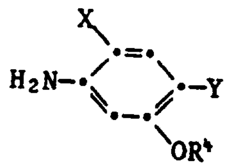

in which X and $R^4$ are as previously defined and Y is chlorine, bromine or iodine, replacing halogen with cyanide by reaction with CuCN, or

c) reducing a nitrobenzene of formula

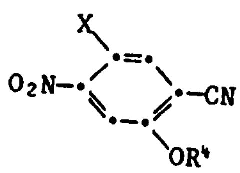

in which X and $R^4$ are as previously defined.

8. A process for the preparation of an aniline of formula IIIc

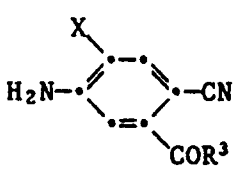

IIIC

in which

X is hydrogen; or halogen; and

$R^3$ is hydroxyl; ($C_1$-$C_8$)-alkoxy; ($C_1$-$C_4$)-alkoxy-($C_1$-$C_4$)-alkoxy; ($C_1$-$C_4$)-alkylthio-($C_1$-$C_4$)-alkoxy; mono- or di-($C_1$-$C_4$)-alkylamino-($C_1$-$C_4$)-alkoxy; ($C_1$-$C_8$)-cyanoalkoxy; ($C_3$-$C_8$)-alkenyloxy; ($C_3$-$C_8$)-haloalkenyloxy; ($C_3$-$C_8$)-alkynyloxy; ($C_3$-$C_7$)-cycloalkoxy; ($C_3$-$C_7$)-cycloalkyl-($C_1$-$C_4$)-alkoxy; halo-($C_3$-$C_7$)-cycloalkoxy; benzyloxy which is unsubstituted or monosubstituted in the phenyl nucleus by halogen, ($C_1$-$C_4$)-alkyl, ($C_1$-$C_4$)alkoxy, ($C_1$-$C_4$)-haloalkyl, cyano or nitro; phenoxy; halophenoxy; ($C_1$-$C_4$)-alkylphenoxy; ($C_1$-$C_4$)-alkoxyphenoxy; ($C_1$-$C_4$)-haloalkylphenoxy;

cyanophenoxy; nitrophenoxy; phenylthio; halophenylthio; ($C_1$-$C_4$)-alkylphenylthio; ($C_1$-$C_4$)-alkoxyphenylthio; ($C_1$-$C_4$)-haloalkylthio; cyanophenylthio; nitrophenylthio; the salt groups -O-Na, -O-K, -O-$(Ca)_{0.5}$, -O-$(Mg)_{0.5}$ or -O-$NH_4$; amino; ($C_1$-$C_4$)-alkylamino; di-($C_1$-$C_4$)-alkylamino; ($C_2$-$C_4$)-halo-alkylamino; di-($C_2$-$C_4$)-haloalkylamino; ($C_1$-$C_4$)-hydroxyalkylamino; di-($C_1$-$C_4$)-hydroxyalkylamino; ($C_3$-$C_4$)-alkenylamino; diallylamino; N-pyrrolidino; N-piperidino; N-morpholino; N-thiomorpholino; N-piperidazino; ($C_1$-$C_8$)-alkylthio; ($C_3$-$C_8$)-alkenylthio; benzylthio; ($C_1$-$C_4$)-alkylthio substituted by ($C_1$-$C_8$)-alkoxycarbonyl, ($C_1$-$C_4$)-alkoxy-($C_1$-$C_4$)-alkoxycarbonyl, ($C_3$-$C_8$)-alkenyloxycarbonyl, ($C_3$-$C_8$)-alkynyloxycarbonyl, ($C_1$-$C_8$)-alkylthiocarbonyl, ($C_3$-$C_8$)-alkenylthiocarbonyl or ($C_3$-$C_8$)-alkynylthiocarbonyl; or ($C_1$-$C_4$)-alkoxy substituted by ($C_1$-$C_8$)-alkoxycarbonyl, ($C_1$-$C_4$)-alkoxy-($C_1$-$C_4$)-alkoxy-carbonyl, ($C_3$-$C_8$)-alkenyloxycarbonyl, ($C_3$-$C_8$)-alkynyloxycarbonyl, ($C_1$-$C_8$)-alkylthiocarbonyl, ($C_3$-$C_8$)-alkenylthiocarbonyl, ($C_3$-$C_8$)-alkynylthiocarbonyl, ($C_1$-$C_4$)-alkylcarbamoyl, di-($C_1$-$C_4$)-alkylcarbamoyl or phenylcarbamoyl which is unsubstituted or monosubstituted in the phenyl nucleus by halogen, ($C_1$-$C_4$)-alkyl, ($C_1$-$C_4$)-alkoxy, ($C_1$-$C_4$)-haloalkyl, cyano or nitro,

which comprises

a) in a compound of formula

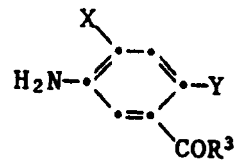

in which X and $R^3$ are as previously defined and Y is chlorine, bromine or iodine, replacing halogen with cyanide by reaction with CuCN, or

b) reducing a nitrobenzene of formula

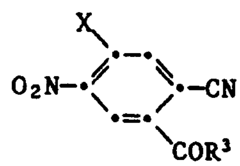

in which X and $R^3$ are as previously defined.

**9.** A process for the preparation of a compound of formula VII

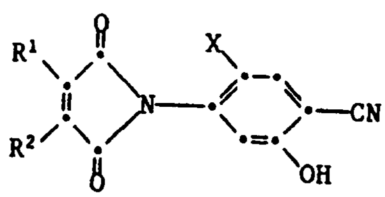

(VII)

in which

$R^1$ is hydrogen; or ($C_1$-$C_4$)-alkyl;

$R^2$ is ($C_1$-$C_4$)-alkyl; or

$R^1$ and $R^2$, when taken together, are a $(CH_2)_4$ group which can be substituted by 1 or 2 ($C_1$-$C_4$)-alkyl groups; and

X is hydrogen; or halogen,

which comprises

a) reacting a furan-2,5-dione of formula III with a 5-hydroxyaniline

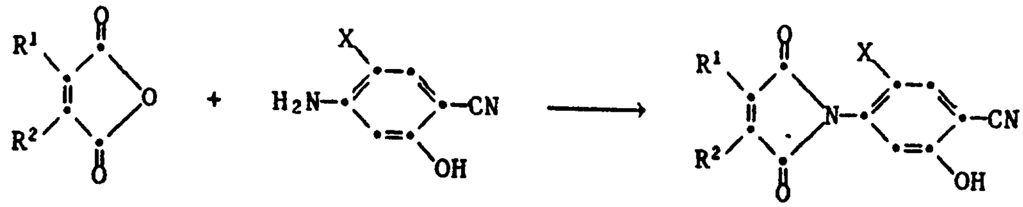

II

VII

in which $R^1$, $R^2$ and X are as previously described or

b) by replacing halogen with cyanide according to the equation below:

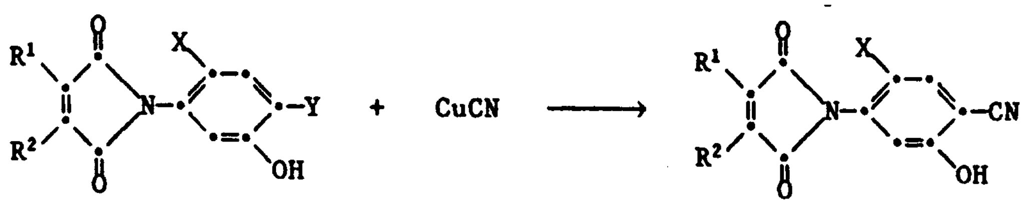

VII

in which $R^1$, $R^2$ and X are as previously defined and Y is chlorine, bromine or iodine.

**10.** A process for the preparation of a herbicidal or plant growth regulating composition, which comprises mixing a compound of formula I

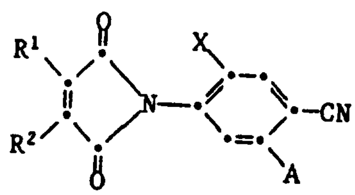

(I)

in which the radicals $R^1$, $R^2$, X and A may have the meanings given in any one of claims 1 to 4, with adjuvants and/or carriers.

**11.** A method of controlling undesirable plant growth, which comprises treating the plants to be controlled or their locus with a herbicidally effective amount of a compound of formula I

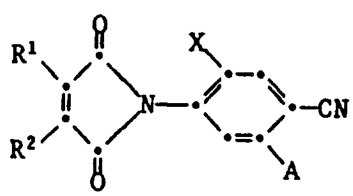

(I)

in which $R^1$, $R^2$, X and A are as defined in any one of claims 1 to 4.

**12.** A method of influencing growth of cultivated plants, which comprises treating the cultivated plant or its locus with a plant growth regulating effective amount of a compound of formula I

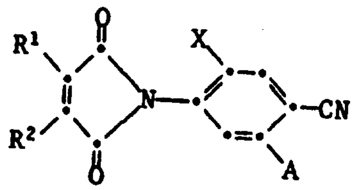

(I)

in which $R^1$, $R^2$, X and A are as defined in any one of claims 1 to 4.

**13.** A method of seed dressing, which comprises applying to the seed of the cultivated plant a herbicidally effective amount of a compound of formula I

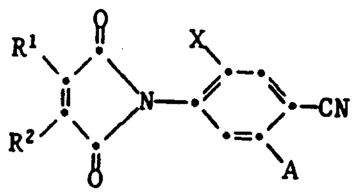

(I)

in which $R^1$, $R^2$, X and A are as defined in any one of claims 1 to 4.

## Revendications

**Revendications pour les Etats contractants suivants : AT, BE, CH, DE, FR, GB, GR, IT, LI, LU, NL, SE**

**1.** Composés de formule I

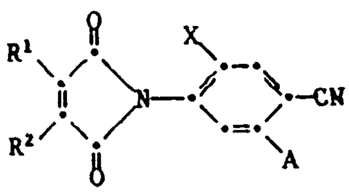

(I)

dans laquelle

$R^1$ représente un atome d'hydrogène ou un groupe alkyle en $C_1$-$C_4$;

$R^2$ représente un groupe alkyle en $C_1$-$C_4$; ou

$R^1$ et $R^2$ représentent ensemble un groupe $-(CH_2)_4-$ qui peut être jusqu'à deux fois substitué par un ou des groupes alkyle en $C_1$-$C_4$;

X représente un atome d'hydrogène ou d'halogène;

A représente

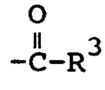

ou O-$R^4$;

$R^3$ représente un groupe hydroxy; alcoxy en $C_1$-$C_8$; alcoxy-($C_1$-$C_4$)-alcoxy($C_1$-$C_4$);alkyl($C_1$-$C_4$)-thio-alcoxy($C_1$-$C_4$); mono- ou dialkyl($C_1$-$C_4$)-amino-alcoxy($C_1$-$C_4$); cyanoalcoxy en $C_1$-$C_8$; alcényloxy en $C_3$-$C_8$; halogénoalcényloxy en $C_3$-$C_8$; alcynyloxy en $C_3$-$C_8$; cycloalkyloxy en $C_3$-$C_7$; cycloalkyl($C_3$-$C_7$)-alkyloxy($C_1$-$C_4$); halogénocycloalcoxy en $C_3$-$C_7$; benzyloxy qui est non substitué ou substitué une fois sur le noyau phényle par un atome d'halogène ou par un radical alkyle en $C_1$-$C_4$, alcoxy en $C_1$-$C_4$, halogénoalkyle en $C_1$-$C_4$, cyano ou nitro; le groupe phénoxy; un groupe halogénophénoxy; alkyl($C_1$-

$C_4$)-phénoxy; alkyloxy($C_1$-$C_4$)-phénoxy; halogénoalkyl($C_1$-$C_4$)-phénoxy; cyanophénoxy; nitrophénoxy; phénylthio; halogénophénylthio; alkyl($C_1$-$C_4$)-phénylthio; alcoxy($C_1$-$C_4$)-phénylthio; halogénoalkyl($C_1$-$C_4$)-thio; cyanophénylthio; nitrophénylthio; les groupes de type sel -O-Na, -O-K, -O-$(Ca)_{0,5}$, -O-$(Mg)_{0,5}$ ou -O-$NH_4$; le groupe amino; un groupe alkyl($C_1$-$C_4$)-amino; dialkyl($C_1$-$C_4$)-amino; halogénoalkyl($C_2$-$C_4$)-amino; dihalogénoalkyl-($C_2$-$C_4$)amino; hydroxyalkyl($C_1$-$C_4$)-amino; dihydroxyalkyl($C_1$-$C_4$)-amino; alcénylamino en $C_3$-$C_4$; diallylamino; -N-pyrrolidino; -N-pipéridino; -N-morpholino; -N-thiomorpholino; -N-pipéridazino; alkyl($C_1$-$C_8$)-thio; alcényl($C_3$-$C_8$)-thio; benzylthio; un groupe alkyl($C_1$-$C_4$)-thio substitué par un radical alcoxy($C_1$-$C_8$)-carbonyle, alcoxy($C_1$-$C_4$)-alcoxy($C_1$-$C_4$)-carbonyle, alcényloxy($C_3$-$C_8$)-carbonyle, alcynyloxy($C_3$-$C_8$)-carbonyle, alkylthio($C_1$-$C_8$)-carbonyle, alcénylthio($C_3$-$C_8$)-carbonyle ou alcynylthio($C_3$-$C_8$)-carbonyle; ou un radical alcoxy en $C_1$-$C_4$ substitué par un groupe alcoxy($C_1$-$C_8$)-carbonyle, alcoxy($C_1$-$C_4$)-alcoxy($C_1$-$C_4$)-carbonyle, alcényloxy($C_3$-$C_8$)-carbonyle, alcynyloxy($C_3$-$C_8$)-carbonyle, alkylthio($C_1$-$C_8$)-carbonyle, alcénylthio($C_3$-$C_8$)-carbonyle, alcynylthio($C_3$-$C_8$)-carbonyle, alkyl($C_1$-$C_4$)amino-carbonyle, dialkyl($C_1$-$C_4$)-aminocarbonyle ou phénylaminocarbonyle qui est non substitué ou substitué une fois sur le noyau phényle par un atome d'halogène ou par un groupe alkyle en $C_1$-$C_4$, alcoxy en $C_1$-$C_4$, halogénoalkyle en $C_1$-$C_4$, cyano ou nitro;

$R^4$ est groupe alkyle en $C_1$-$C_8$; alcoxy($C_1$-$C_4$)-alkyle($C_1$-$C_4$); alkyl($C_1$-$C_4$)thio-alkyle($C_1$-$C_4$), mono- ou dialkyl($C_1$-$C_4$)-amino-alkyle($C_1$-$C_4$); halogénoalkyle en $C_1$-$C_8$; cyanoalkyle en $C_1$-$C_8$; alcényle en $C_3$-$C_8$; halogénoalcényle en $C_3$-$C_8$; alcynyle en $C_3$-$C_8$; cycloalkyle en $C_3$-$C_7$; cycloalkyl($C_3$-$C_7$)-alkyle($C_1$-$C_4$); halogénocycloalkyle en $C_3$-$C_7$; alkyl($C_1$-$C_8$)-carbonyle; allylcarbonyle; un groupe benzylcarbonyle qui est non substitué ou substitué une fois sur le noyau phényle par un atome d'halogène ou par un radical alkyle en $C_1$-$C_4$, alcoxy en $C_1$-$C_4$, halogénoalkyle en $C_1$-$C_4$, cyano ou nitro; un groupe cycloalkyl($C_3$-$C_7$)-carbonyle; benzoyle qui est non substitué ou substitué une fois sur le noyau phényle par un atome d'halogène ou par un groupe alkyle en $C_1$-$C_4$, alcoxy en $C_1$-$C_4$, halogénoalkyle en $C_1$-$C_4$, cyano ou nitro; le groupe furoyle; thiénoyle; un groupe alkyle en $C_1$-$C_4$ substitué par un radical phényle, halogénophényle, alkyl($C_1$-$C_4$)-phényle, alcoxy($C_1$-$C_4$)-phényle, halogénoalkyl($C_1$-$C_4$)-phényle, halogénoalcoxy($C_1$-$C_4$)phényle; nitrophényle, cyanophényle, alcoxy($C_1$-$C_8$)-carbonyle, alcoxy($C_1$-$C_4$)-alcoxy($C_1$-$C_4$)-carbonyle, alcényloxy($C_3$-$C_8$)-carbonyle, alcynyloxy($C_3$-$C_8$)-carbonyle, alkyl($C_1$-$C_8$)-thiocarbonyle, alcényl($C_3$-$C_8$)-thiocarbonyle, alcynyl($C_3$-$C_8$)-thiocarbonyle, carbamoyle, alkyl-($C_1$-$C_4$)-aminocarbonyle, dialkyl($C_1$-$C_4$)-aminocarbonyle, hydroxy, phénylaminocarbonyle qui est non substitué ou substitué une fois sur le noyau phényle par un atome d'halogène ou par un radical alkyle en $C_1$-$C_4$, alcoxy en $C_1$-$C_4$, cyano, nitro ou halogénoalkyle en $C_1$-$C_4$; un groupe dioxolanne-2-yle qui est non substitué ou substitué par un ou deux radicaux alkyle en $C_1$-$C_4$, ou le groupe 1,3-dioxanne-2-yle qui est non substitué ou substitué par un ou deux radicaux alkyle en $C_1$-$C_4$.

**2.** Composés de formule I selon la revendication 1, dans lesquels X représente un atome de fluor ou d'hydrogène.

**3.** Composés de formule I selon la revendication 1 ou 2, dans lesquels A représente $OR^4$ et $R^4$ représente le groupe isopropyle, ou A représente $COOR^3$ et $R^3$ représente le groupe sec-butyle.

**4.** 1-(4-cyano-2-fluoro-5-isopropyloxyphényl)-3,4-diméthyl-pyrrole-(1H)2,5-dione, 2-(5-carboxy-4-cyano-2-fluorophényl)-4,5,6,7-tétrahydroisoindole-(2H)1,3-dione, 2-(4-cyano-2-fluoro-5-n-propyloxycarbonylphényl)-4,5,6,7-tétrahydroisoindole-(2H)1,3-dione, 2-(5-n-butyloxycarbonyl-4-cyano-2-fluorophényl)-4,5,6,7-tétrahydroisoindole-(2H)1,3-dione, 2-(4-cyano-2-fluoro-5-isopropyloxyphényl)-5-méthyl-4,5,6,7-tétrahydroisoindole-(2H)1,3-dione, 1-(4-cyano-2-fluoro-5-isopropyloxyphényl)-3-éthyl-4-méthyl-pyrrole-(1H)-2,5-dione, 2-(4-cyano-2-fluoro-5-isopropyloxyphényl)-4,5,6,7-tétrahydroisoindole-(2H)1,3-dione, 2-(4-cyano-2-fluoro-5-isopropyloxycarbonylphényl)-4,5,6,7-tétrahydroisoindole-(2H)1,3-dione ou 2-(5-sec-butyloxycarbonyl-4-cyano-2-fluorophényl)-4,5,6,7-tétrahydroisoindole-(2H)1,3-dione en tant que composé de formule I selon la revendication 1.

**5.** Procédé pour la préparation des composés de formule I selon la revendication 1, caractérisé

a) par la réaction d'une furanne-2,5-dione de formule II avec une aniline III

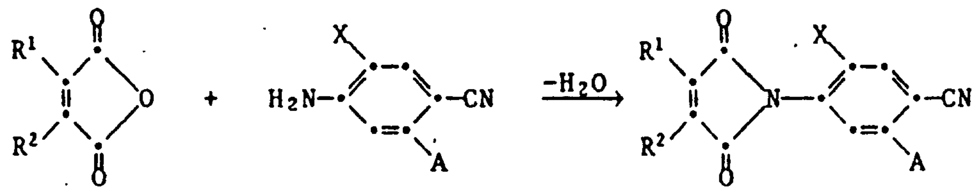


II III I,

les radicaux $R^1$, $R^2$, X et A étant tels que définis précédemment, ou
b) par un échange halogène-cyanure à partir d'un composé de formule IV

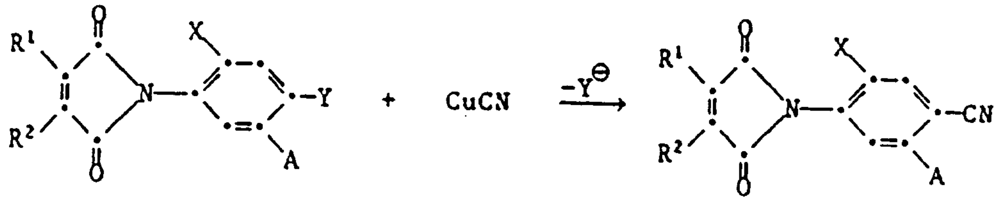


IV,

les radicaux $R^1$, $R^2$, X et A étant tels que définis précédemment, et Y représentant le chlore, le brome ou l'iode, avec CuCN.

**6.** Procédé pour la préparation de composés de formule I selon la revendication 1, dans lesquels A représente $COR^3$, caractérisé par:
a) la condensation d'un composé de formule V, dans lequel $R^1$, $R^2$ et X sont tels que définis précédemment,

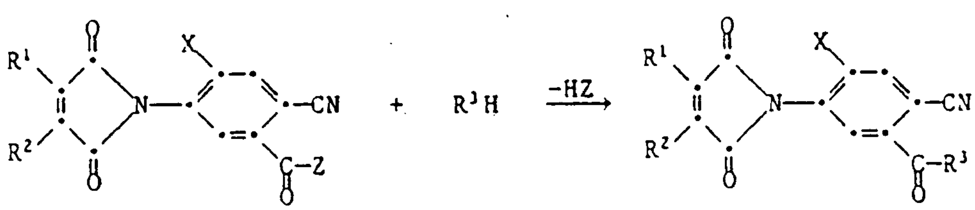


V VI I

et Z représente un groupe remplaçable dans les conditions réactionnelles, tel qu'un halogène ou un groupe alkyl($C_1$-$C_4$)-carbonyloxy, avec un composé de formule VI dans lequel $R^3$ est tel que défini précédemment, ou
b) l'estérification d'un acide carboxylique de formule Ia', dans lequel les radicaux $R^1$, $R^2$ et X sont tels que définis précédemment, avec un alcool ou thioalcool de formule VI

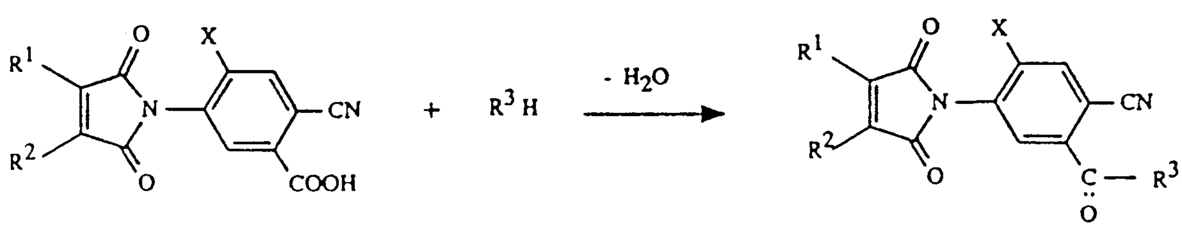


Ia' VI Ia

dans laquelle

$R^3$ représente un groupe alcoxy en $C_1$-$C_8$; alcoxy($C_1$-$C_4$)-alcoxy($C_1$-$C_4$); alkyl($C_1$-$C_4$)-thio-alcoxy($C_1$-$C_4$); mono- ou dialkyl($C_1$-$C_4$)-amino-alcoxy($C_1$-$C_4$); cyanoalcoxy en $C_1$-$C_8$; alcényloxy en $C_3$-$C_8$; halogénoalcényloxy en $C_3$-$C_8$; alcynyloxy en $C_3$-$C_8$; cycloalkyloxy en $C_3$-$C_7$; cycloalkyl($C_3$-$C_7$)-alkyloxy($C_1$-$C_4$); halogénocycloalcoxy en $C_3$-$C_7$; un groupe benzyloxy qui est non substitué ou substitué une fois sur le noyau phényle par un atome d'halogène ou par un radical alkyle en $C_1$-$C_4$, alcoxy en $C_1$-$C_4$, halogénoalkyle en $C_1$-$C_4$, cyano ou nitro; le groupe phénoxy; un groupe halogénophénoxy; un groupe alkyl($C_1$-$C_4$)-phénoxy; alkyloxy($C_1$-$C_4$)-phénoxy; halogénoalkylphénoxy; cyanophénoxy; nitrophénoxy; phénylthio; halogénophénylthio; alkyl($C_1$-$C_4$)-phénylthio; alcoxy($C_1$-$C_4$)-phénylthio; halogénoalkylthio en $C_1$-$C_4$; cyanophénylthio; nitrophénylthio; alkyl($C_1$-$C_8$)-thio; alcényl($C_3$-$C_8$)-thio; benzylthio; un groupe alkyl($C_1$-$C_4$)-thio substitué par un radical alcoxy($C_1$-$C_8$)-carbonyle, alcoxy($C_1$-$C_4$)-alcoxy($C_1$-$C_4$)-carbonyle, alcényloxy($C_3$-$C_8$)-carbonyle, alcynyloxy($C_3$-$C_8$)-carbonyle, alkyl($C_1$-$C_8$)-thiocarbonyle ou alcynyl($C_3$-$C_8$)-thiocarbonyle; ou un groupe alcoxy en $C_1$-$C_4$ substitué par un radical alcoxy($C_1$-$C_8$)-carbonyle, alcoxy($C_1$-$C_4$)-alcoxy-($C_1$-$C_4$)-carbonyle, alcényloxy($C_3$-$C_8$)-carbonyle, alcynyloxy($C_3$-$C_8$)-carbonyle, alkyl($C_1$-$C_8$)-thiocarbonyle, alcényl($C_3$-$C_8$)-thiocarbonyle, alcynyl($C_3$-$C_8$)-thiocarbonyle, alkyl($C_1$-$C_4$)-aminocarbonyle, dialkyl($C_1$-$C_4$)-aminocarbonyle ou phénylaminocarbonyle, qui est non substitué ou subtitué une fois sur le noyau phényle par un atome d'halogène ou par un groupe alkyle en $C_1$-$C_4$, alcoxy en $C_1$-$C_4$, halogénoalkyle en $C_1$-$C_4$, cyano ou nitro;

pour aboutir à un ester de formule Ia dans lequel les radicaux $R^1$, $R^2$, $R^3$ et X sont tels que définis précédemment.

**7.** Procédé pour la préparation de composés de formule I selon la revendication 1, dans lesquels A représente $OR^4$, caractérisé par l'éthérification d'un phénol de formule VII, dans laquelle $R^1$, $R^2$ et X sont tels que définis précédemment

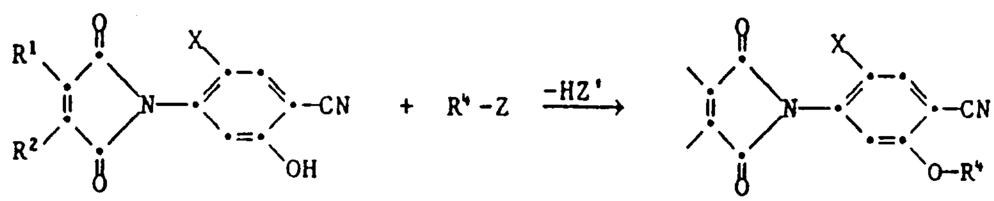


VII VIII Ib

avec un composé de formule VIII dans lequel $R^4$ est tel que défini précédemment et Z représente un groupe séparable dans les conditions réactionnelles, tel qu'un atome d'halogène ou un groupe phénylsulfonyloxy éventuellement alkylé.

**8.** Anilines de formule IIIb

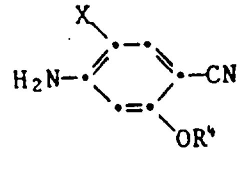

IIIb

dans laquelle

X représente un atome d'hydrogène ou d'halogène et

$R^4$ est groupe alkyle en $C_1$-$C_8$; alcoxy($C_1$-$C_4$)-alkyle($C_1$-$C_4$); alkyl($C_1$-$C_4$)thio-alkyle($C_1$-$C_4$), mono- ou dialkyl($C_1$-$C_4$)-amino-alkyle($C_1$-$C_4$); halogénoalkyle en $C_1$-$C_8$; cyanoalkyle en $C_1$-$C_8$; alcényle en $C_3$-$C_8$; halogénoalcényle en $C_3$-$C_8$; alcynyle en $C_3$-$C_8$; cycloalkyle en $C_3$-$C_7$; cycloalkyl($C_3$-$C_7$)-alkyle($C_1$-$C_4$); halogénocycloalkyle en $C_3$-$C_7$; alkyl($C_1$-$C_8$)-carbonyle; allylcarbonyle; un groupe benzylcarbonyle qui est non substitué ou substitué une fois sur le noyau phényle par un atome d'halogène ou par un radical alkyle en $C_1$-$C_4$, alcoxy en $C_1$-$C_4$,

halogénoalkyle en $C_1$-$C_4$, cyano ou nitro; un groupe cycloalkyl($C_3$-$C_7$)-carbonyle; benzoyle qui est non substitué ou substitué une fois sur le noyau phényle par un atome d'halogène ou par un groupe alkyle en $C_1$-$C_4$, alcoxy en $C_1$-$C_4$, halogénoalkyle en $C_1$-$C_4$, cyano ou nitro; le groupe furoyle; thiénoyle; un groupe alkyle en $C_1$-$C_4$ substitué par un radical phényle, halogénophényle, alkyl($C_1$-$C_4$)-phényle, alcoxy($C_1$-$C_4$)-phényle, halogénoalkyl($C_1$-$C_4$)-phényle, halogénoalcoxy($C_1$-$C_4$)-phényle; nitrophényle, cyanophényle, alcoxy($C_1$-$C_8$)-carbonyle, alcoxy($C_1$-$C_4$)-alcoxy($C_1$-$C_4$)-carbonyle, alcényloxy($C_3$-$C_8$)-carbonyle, alcynyloxy($C_3$-$C_8$)-carbonyle, alkyl($C_1$-$C_8$)-thiocarbonyle, alcényl($C_3$-$C_8$)-thiocarbonyle, alcynyl($C_3$-$C_8$)-thiocarbonyle, carbamoyle, alkyl($C_1$-$C_4$)-aminocarbonyle, dialkyl($C_1$-$C_4$)-aminocarbonyle, hydroxy, phénylaminocarbonyle qui est non substitué ou substitué une fois sur le noyau phényle par un atome d'halogène ou par un radical alkyle en $C_1$-$C_4$, alcoxy en $C_1$-$C_4$, cyano, nitro ou halogénoalkyle en $C_1$-$C_4$; un groupe dioxolanne-2-yle qui est non substitué ou substitué par un ou deux radicaux alkyle en $C_1$-$C_4$, ou le groupe 1,3-dioxanne-2-yle qui est non substitué ou substitué par un ou deux radicaux alkyle en $C_1$-$C_4$,

à l'exception du 2-méthoxy-4-amino-5-chlorobenzonitrile.

**9.** Anilines de formule IIIc

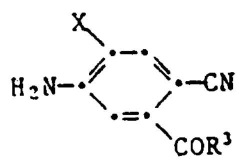

IIIc

dans laquelle

X représente un atome d'hydrogène ou d'halogène; et

$R^3$ représente un groupe hydroxy; alcoxy en $C_1$-$C_8$; alcoxy($C_1$-$C_4$)-alcoxy($C_1$-$C_4$); alkyl($C_1$-$C_4$)-thio-alcoxy($C_1$-$C_4$); mono- ou dialkyl($C_1$-$C_4$)-amino-alcoxy($C_1$-$C_4$); cyanoalcoxy en $C_1$-$C_8$; alcényloxy en $C_3$-$C_8$; halogénoalcényloxy en $C_3$-$C_8$; alcynyloxy en $C_3$-$C_8$; cycloalkyloxy en $C_3$-$C_7$; cycloalkyl($C_3$-$C_7$)-alkyloxy($C_1$-$C_4$); halogénocycloalcoxy en $C_3$-$C_7$; benzyloxy qui est non substitué ou substitué une fois sur le noyau phényle par un atome d'halogène ou par un radical alkyle en $C_1$-$C_4$, alcoxy en $C_1$-$C_4$, halogénoalkyle en $C_1$-$C_4$, cyano ou nitro; le groupe phénoxy; un groupe halogénophénoxy; alkyl($C_1$-$C_4$)-phénoxy; alkyloxy($C_1$-$C_4$)-phénoxy; halogénoalkyl($C_1$-$C_4$)-phénoxy; cyanophénoxy; nitrophénoxy; phénylthio; halogénophénylthio; alkyl($C_1$-$C_4$)-phénylthio; alcoxy($C_1$-$C_4$)-phénylthio; halogénoalkyl($C_1$-$C_4$)-thio; cyanophénylthio; nitrophénylthio; les groupes de type sel -O-Na, -O-K, -O-$(Ca)_{0,5}$, -O-$(Mg)_{0,5}$ ou -O-$NH_4$; le groupe amino; un groupe alkyl($C_1$-$C_4$)-amino; dialkyl($C_1$-$C_4$)-amino; halogénoalkyl($C_2$-$C_4$)-amino; dihalogénoalkyl($C_2$-$C_4$)-amino; hydroxyalkyl($C_1$-$C_4$)-amino; dihydroxyalkyl($C_1$-$C_4$)-amino; alcénylamino en $C_3$-$C_4$; diallylamino; -N-pyrrolidino; -N-pipéridino; -N-morpholino; -N-thiomorpholino; -N-pipéridazino; alkyl($C_1$-$C_8$)-thio; alcényl($C_3$-$C_8$)-thio; benzylthio; un groupe alkyl($C_1$-$C_4$)-thio substitué par un radical alcoxy($C_1$-$C_8$)-carbonyle, alcoxy($C_1$-$C_4$)-alcoxy($C_1$-$C_4$)-carbonyle, alcényloxy($C_3$-$C_8$)-carbonyle, alcynyloxy($C_3$-$C_8$)-carbonyle, alkylthio($C_1$-$C_8$)-carbonyle, alcénylthio($C_3$-$C_8$)-carbonyle ou alcynylthio($C_3$-$C_8$)-carbonyle; ou un radical alcoxy en $C_1$-$C_4$ substitué par un groupe alcoxy($C_1$-$C_8$)-carbonyle, alcoxy($C_1$-$C_4$)-alcoxy($C_1$-$C_4$)-carbonyle, alcényloxy($C_3$-$C_8$)-carbonyle, alcynyloxy($C_3$-$C_8$)-carbonyle, alkylthio($C_1$-$C_8$)-carbonyle, alcénylthio($C_3$-$C_8$)-carbonyle, alcynylthio($C_3$-$C_8$)-carbonyle, alkyl($C_1$-$C_4$)amino-carbonyle, dialkyl($C_1$-$C_4$)-aminocarbonyle ou phénylaminocarbonyle qui est non substitué ou substitué une fois sur le noyau phényle par un atome d'halogène ou par un groupe alkyle en $C_1$-$C_4$, alcoxy en $C_1$-$C_4$, halogénoalkyle en $C_1$-$C_4$, cyano ou nitro.

**10.** Composés de formule VII

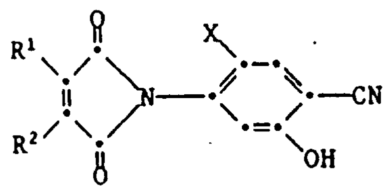

(VII)

dans laquelle

- $R^1$ représente un atome d'hydrogène ou un groupe alkyle en $C_1$-$C_4$;
- $R^2$ représente un groupe alkyle en $C_1$-$C_4$; ou
- $R^1$ et $R^2$ représentent ensemble un groupe $-(CH_2)_4-$ qui peut être substitué jusqu'à deux fois par un radical ou des radicaux alkyle en $C_1$-$C_4$;
- X représente un atome d'hydrogène ou d'halogène.

**11.** Produit herbicide ou régulateur de croissance, contenant un composé de formule I selon l'une des revendications 1 à 4, en plus d'autres adjuvants et/ou matériaux de support.

**12.** Procédé pour la lutte contre la croissance végétale indésirable, caractérisé en ce que l'on fait agir sur les plantes à combattre ou sur leur biotope un composé de formule I selon l'une des revendications 1 à 4 ou un produit herbicide selon la revendication 11.

**13.** Semences caractérisées par une teneur à action herbicide d'un composé de formule I selon l'une des revendications 1 à 4.

**14.** Procédé pour la défeuillaison de plantes utiles, caractérisé en ce que l'on fait agir sur les plantes à défeuiller un composé de formule I selon l'une des revendications 1 à 4 ou un produit régulateur de croissance selon la revendication 11.

**Revendications pour l'Etat contractant suivant : ES**

**1.** Procédé pour la préparation de composés de formule I

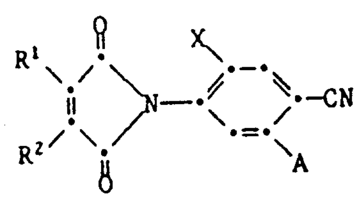

(I)

dans laquelle

- $R^1$ représente un atome d'hydrogène ou un groupe alkyle en $C_1$-$C_4$;
- $R^2$ représente un groupe alkyle en $C_1$-$C_4$; ou
- $R^1$ et $R^2$ représentent ensemble un groupe $-(CH_2)_4-$ qui peut être jusqu'à deux fois substitué par un ou des groupes alkyle en $C_1$-$C_4$;
- X représente un atome d'hydrogène ou d'halogène;
- A représente

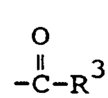

ou $O-R^4$;

- $R^3$ représente un groupe hydroxy; alcoxy en $C_1$-$C_8$; alcoxy($C_1$-$C_4$)-alcoxy($C_1$-$C_4$); alkyl($C_1$-

$C_4$)-thio-alcoxy($C_1$-$C_4$); mono- ou dialkyl($C_1$-$C_4$)-amino-alcoxy($C_1$-$C_4$); cyanoalcoxy en $C_1$-$C_8$; alcényloxy en $C_3$-$C_8$; halogénoalcényloxy en $C_3$-$C_8$; alcynyloxy en $C_3$-$C_8$; cycloalkyloxy en $C_3$-$C_7$; cycloalkyl($C_3$-$C_7$)-alkyloxy($C_1$-$C_4$); halogénocycloalcoxy en $C_3$-$C_7$; benzyloxy qui est non substitué ou substitué une fois sur le noyau phényle par un atome d'halogène ou par un radical alkyle en $C_1$-$C_4$, alcoxy en $C_1$-$C_4$, halogénoalkyle en $C_1$-$C_4$, cyano ou nitro; le groupe phénoxy; un groupe halogénophénoxy; alkyl($C_1$-$C_4$)-phénoxy; alkyloxy($C_1$-$C_4$)-phénoxy; halogénoalkyl($C_1$-$C_4$)-phénoxy; cyanophénoxy; nitrophénoxy; phénylthio; halogénophénylthio; alkyl($C_1$-$C_4$)-phénylthio; alcoxy($C_1$-$C_4$)-phénylthio; halogénoalkyl($C_1$-$C_4$)-thio; cyanophénylthio; nitrophénylthio; les groupes de type sel -O-Na, -O-K, -O-$(Ca)_{0,5}$, -O-$(Mg)_{0,5}$ ou -O-$NH_4$; le groupe amino; un groupe alkyl($C_1$-$C_4$)-amino; dialkyl($C_1$-$C_4$)-amino; halogénoalkyl($C_2$-$C_4$)-amino; dihalogénoalkyl-($C_2$-$C_4$)-amino; hydroxyalkyl($C_1$-$C_4$)-amino; dihydroxyalkyl($C_1$-$C_4$)-amino; alcénylamino en $C_3$-$C_4$; diallylamino; -N-pyrrolidino; -N-pipéridino; -N-morpholino; -N-thiomorpholino; -N-pipéridazino; alkyl($C_1$-$C_8$)-thio; alcényl($C_3$-$C_8$)-thio; benzylthio; un groupe alkyl($C_1$-$C_4$)-thio substitué par un radical alcoxy($C_1$-$C_8$)-carbonyle, alcoxy($C_1$-$C_4$)-alcoxy($C_1$-$C_4$)-carbonyle, alcényloxy($C_3$-$C_8$)-carbonyle, alcynyloxy($C_3$-$C_8$)-carbonyle, alkylthio($C_1$-$C_8$)-carbonyle, alcénylthio($C_3$-$C_8$)-carbonyle ou alcynylthio($C_3$-$C_8$)-carbonyle; ou un radical alcoxy en $C_1$-$C_4$ substitué par un groupe alcoxy($C_1$-$C_8$)-carbonyle, alcoxy($C_1$-$C_4$)-alcoxy($C_1$-$C_4$)-carbonyle, alcényloxy($C_3$-$C_8$)-carbonyle, alcynyloxy($C_3$-$C_8$)-carbonyle, alkylthio($C_1$-$C_8$)-carbonyle, alcénylthio($C_3$-$C_8$)-carbonyle, alcynylthio($C_3$-$C_8$)-carbonyle, alkyl($C_1$-$C_4$)amino-carbonyle, dialkyl($C_1$-$C_4$)-aminocarbonyle ou phénylaminocarbonyle qui est non substitué ou substitué une fois sur le noyau phényle par un atome d'halogène ou par un groupe alkyle en $C_1$-$C_4$, alcoxy en $C_1$-$C_4$, halogénoalkyle en $C_1$-$C_4$, cyano ou nitro;

$R^4$ est groupe alkyle en $C_1$-$C_8$; alcoxy($C_1$-$C_4$)-alkyle($C_1$-$C_4$); alkyl($C_1$-$C_4$)thio-alkyle($C_1$-$C_4$), mono- ou dialkyl($C_1$-$C_4$)-amino-alkyle($C_1$-$C_4$); halogénoalkyle en $C_1$-$C_8$; cyanoalkyle en $C_1$-$C_8$; alcényle en $C_3$-$C_8$; halogénoalcényle en $C_3$-$C_8$; alcynyle en $C_3$-$C_8$; cycloalkyle en $C_3$-$C_7$; cycloalkyl($C_3$-$C_7$)-alkyle($C_1$-$C_4$); halogénocycloalkyle en $C_3$-$C_7$; alkyl($C_1$-$C_8$)-carbonyle; allylcarbonyle; un groupe benzylcarbonyle qui est non substitué ou substitué une fois sur le noyau phényle par un atome d'halogène ou par un radical alkyle en $C_1$-$C_4$, alcoxy en $C_1$-$C_4$, halogénoalkyle en $C_1$-$C_4$, cyano ou nitro; un groupe cycloalkyl($C_3$-$C_7$)-carbonyle; benzoyle qui est non substitué ou substitué une fois sur le noyau phényle par un atome d'halogène ou par un groupe alkyle en $C_1$-$C_4$, alcoxy en $C_1$-$C_4$, halogénoalkyle en $C_1$-$C_4$, cyano ou nitro; le groupe furoyle; thiénoyle; un groupe alkyle en $C_1$-$C_4$ substitué par un radical phényle, halogénophényle, alkyl($C_1$-$C_4$)-phényle, alcoxy($C_1$-$C_4$)-phényle, halogénoalkyl($C_1$-$C_4$)-phényle, halogénoalcoxy($C_1$-$C_4$)-phényle; nitrophényle, cyanophényle, alcoxy($C_1$-$C_8$)-carbonyle, alcoxy($C_1$-$C_4$)-alcoxy($C_1$-$C_4$)-carbonyle, alcényloxy($C_3$-$C_8$)-carbonyle, alcynyloxy($C_3$-$C_8$)-carbonyle, alkyl($C_1$-$C_8$)-thiocarbonyle, alcényl($C_3$-$C_8$)-thiocarbonyle, alcynyl($C_3$-$C_8$)-thiocarbonyle, carbamoyle, alkyl-($C_1$-$C_4$)-aminocarbonyle, dialkyl($C_1$-$C_4$)-aminocarbonyle, hydroxy, phénylaminocarbonyle qui est non substitué ou substitué une fois sur le noyau phényle par un atome d'halogène ou par un radical alkyle en $C_1$-$C_4$, alcoxy en $C_1$-$C_4$, cyano, nitro ou halogénoalkyle en $C_1$-$C_4$; un groupe dioxolanne-2-yle qui est non substitué ou substitué par un ou deux radicaux alkyle en $C_1$-$C_4$, ou le groupe 1,3-dioxanne-2-yle qui est non substitué ou substitué par un ou deux radicaux alkyle en $C_1$-$C_4$;

caractérisé

a) par la réaction d'une furanne-2,5-dione de formule II avec une aniline III

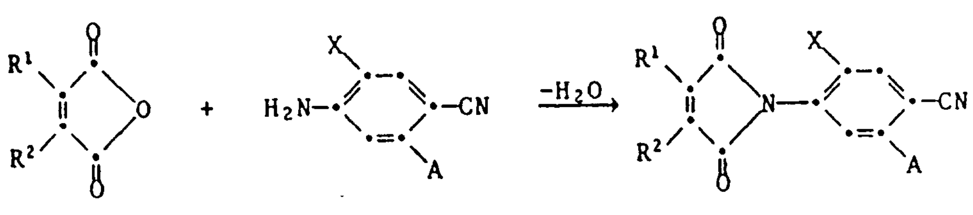


II III I,

les radicaux $R^1$, $R^2$, X et A étant tels que définis précédemment, ou
b) par un échange halogène-cyanure à partir d'un composé de formule IV

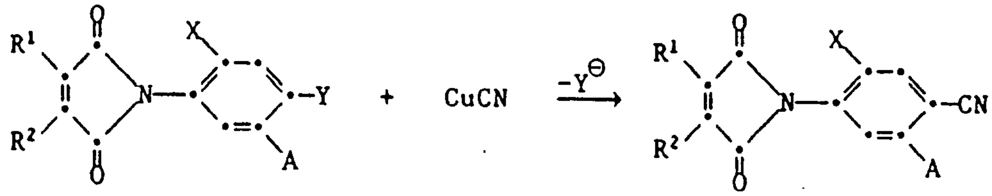


IV,

les radicaux $R^1$, $R^2$, X et A étant tels que définis précédemment, et Y représentant le chlore, le brome ou l'iode, avec CuCN.

**2.** Procédé selon la revendication 1, pour la préparation de composés de formule I

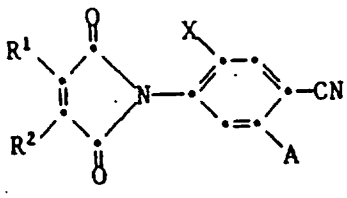

(I)

dans laquelle

| | |
|---|---|
| $R^1$ | représente un atome d'hydrogène ou un groupe alkyle en $C_1$-$C_4$; |
| $R^2$ | représente un groupe alkyle en $C_1$-$C_4$; ou |
| R et $R^2$ | représentent ensemble un groupe $-(CH_2)_4-$ qui peut éventuellement être substitué une fois par un groupe alkyle en $C_1$-$C_4$; |
| X | représente un atome d'hydrogène ou de fluor, de chlore ou de brome; |
| A | représente 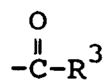 ou O-$R^4$; |
| $R^3$ | représente un groupe hydroxy; alcoxy en $C_1$-$C_5$; alcoxy($C_1$-$C_4$)-alcoxy($C_1$-$C_4$);alkyl($C_1$-$C_4$)-thio-alcoxy($C_1$-$C_4$); mono- ou dialkyl($C_1$-$C_4$)-amino-alcoxy($C_1$-$C_4$); cyanoalcoxy en $C_1$-$C_4$; halogénoalcényloxy en $C_3$-$C_5$; alcynyloxy en $C_3$-$C_5$; alcényloxy en $C_3$-$C_5$; cycloalkyl($C_3$-$C_6$)-méthyloxy; les groupes de type sel -O-Na, -O-K, -O-$(Ca)_{0,5}$, -O-$(Mg)_{0,5}$ ou -O-$NH_4$; le groupe amino; un groupe dialkyl($C_1$-$C_4$)-amino; diallylamino; benzyloxy; -N-pipéridino; -N-morpholino; -N-thiomorpholino; alkyl($C_1$-$C_4$)-thio; un groupe alkyl($C_1$-$C_4$)-thio substitué par un radical alcoxy($C_1$-$C_4$)-carbonyle; un groupe alcoxy($C_1$-$C_4$) substitué par un radical alcoxy($C_1$-$C_4$)-carbonyle, alkyl($C_1$-$C_4$)-thiocarbonyle, alkyl($C_1$-$C_4$)-aminocarbonyle ou dialkyl($C_1$-$C_4$)-aminocarbonyle; |
| $R^4$ | représente un groupe alkyle en $C_1$-$C_5$; alcoxy($C_1$-$C_4$)-alkyle($C_1$-$C_4$); alkyl($C_1$-$C_4$)-thio-alkyle($C_1$-$C_4$); halogénoalkyle en $C_1$-$C_4$; alcényle en $C_3$-$C_5$; halogénoalcényle en $C_3$-$C_5$; alcynyle en $C_3$-$C_5$; cyclohexylméthyle; alkyl($C_1$-$C_4$)-carbonyle; benzoyle qui est non substitué ou substitué une fois sur le noyau phényle par un atome de fluor ou de chlore ou par un radical alkyle en $C_1$-$C_4$, alcoxy en $C_1$-$C_4$, cyano ou nitro; un groupe alkyle en $C_1$-$C_4$, substitué une fois par un radical phényle, alcoxy($C_1$-$C_4$)-carbonyle, alkyl($C_1$-$C_4$)-thiocarbonyle, carbamoyle, dialkyl($C_1$-$C_4$)-aminocarbonyle ou hydroxy. |

3. Procédé selon la revendication 1, pour la préparation de composés de formule I

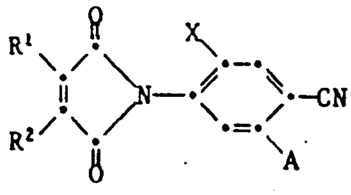

(I)

dans laquelle X représente un atome de fluor ou d'hydrogène et les radicaux $R^1$, $R^2$ et A ont les significations données dans la revendication 1 ou 2.

4. Procédé selon la revendication 1, pour la préparation de composés de formule I

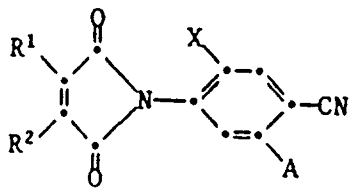

(I)

dans laquelle A représente $OR^4$ et $R^4$ représente le groupe isopropyle, ou A représente $COOR^3$ et $R^3$ représente le groupe sec-butyle, et les radicaux $R^1$, $R^2$ et X ont les significations données dans l'une des revendications 1 à 3.

5. Procédé pour la préparation de composés de formule I

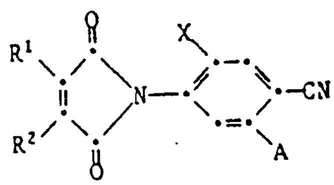

(I)

dans laquelle A représente $COR^3$ et les radicaux $R^1$, $R^2$, $R^3$ et X ont les significations données dans l'une des revendications 1 à 4, caractérisé par

a) la condensation d'un composé de formule V, dans lequel $R^1$, $R^2$ et X sont tels que définis précédemment,

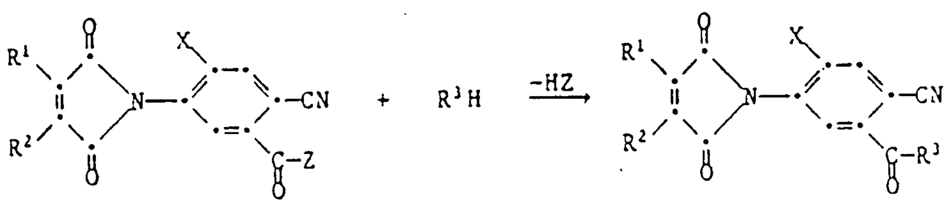


V VI I

et Z représente un groupe remplaçable dans les conditions réactionnelles, tel qu'un halogène ou un groupe alkyl($C_1$-$C_4$)-carbonyloxy, avec un composé de formule VI dans lequel $R^3$ est tel que défini précédemment, ou

b) l'estérification d'un acide carboxylique de formule Ia', dans lequel les radicaux $R^1$, $R^2$ et X sont tels que définis précédemment, avec un alcool ou thioalcool de formule VI

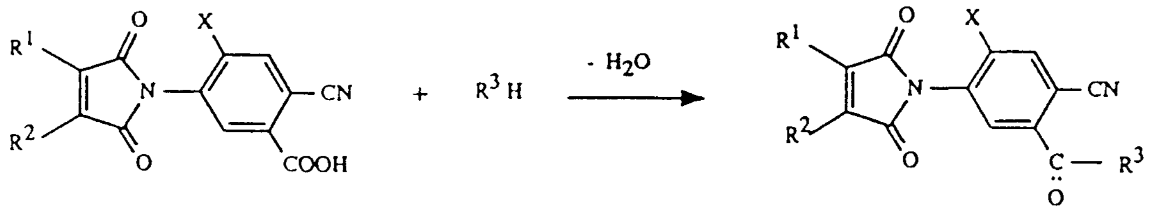


Ia' VI Ia

dans laquelle

$R^3$ représente un groupe alcoxy en $C_1$-$C_8$; alcoxy($C_1$-$C_4$)-alcoxy($C_1$-$C_4$); alkyl($C_1$-$C_4$)-thio-alcoxy($C_1$-$C_4$); mono- ou dialkyl($C_1$-$C_4$)-amino-alcoxy($C_1$-$C_4$); cyanoalcoxy en $C_1$-$C_8$; alcényloxy en $C_3$-$C_8$; halogénoalcényloxy en $C_3$-$C_8$; alcynyloxy en $C_3$-$C_8$; cycloalkyloxy en $C_3$-$C_7$; cycloalkyl($C_3$-$C_7$)-alkyloxy($C_1$-$C_4$); halogénocycloalcoxy en $C_3$-$C_7$; un groupe benzyloxy qui est non substitué ou substitué une fois sur le noyau phényle par un atome d'halogène ou par un radical alkyle en $C_1$-$C_4$, alcoxy en $C_1$-$C_4$, halogénoalkyle en $C_1$-$C_4$, cyano ou nitro; le groupe phénoxy; un groupe halogénophénoxy; un groupe alkyl($C_1$-$C_4$)-phénoxy; alkyloxy($C_1$-$C_4$)-phénoxy; halogénoalkylphénoxy; cyanophénoxy; nitrophénoxy; phénylthio; halogénophénylthio; alkyl($C_1$-$C_4$)-phénylthio; alcoxy($C_1$-$C_4$)-phénylthio; halogénoalkylthio en $C_1$-$C_4$; cyanophénylthio; nitrophénylthio; alkyl($C_1$-$C_8$)-thio; alcényl($C_3$-$C_8$)-thio; benzylthio; un groupe alkyl($C_1$-$C_4$)-thio substitué par un radical alcoxy($C_1$-$C_8$)-carbonyle, alcoxy($C_1$-$C_4$)-alcoxy($C_1$-$C_4$)-carbonyle, alcényloxy($C_3$-$C_8$)-carbonyle, alcynyloxy($C_3$-$C_8$)-carbonyle, alkyl($C_1$-$C_8$)-thiocarbonyle ou alcynyl($C_3$-$C_8$)-thiocarbonyle; ou un groupe alcoxy en $C_1$-$C_4$ substitué par un radical alcoxy($C_1$-$C_8$)-carbonyle, alcoxy($C_1$-$C_4$)-alcoxy-($C_1$-$C_4$)-carbonyle, alcényloxy($C_3$-$C_8$)-carbonyle, alcynyloxy($C_3$-$C_8$)-carbonyle, alkyl($C_1$-$C_8$)-thiocarbonyle, alcényl($C_3$-$C_8$)-thiocarbonyle, alcynyl($C_3$-$C_8$)-thiocarbonyle, alkyl($C_1$-$C_4$)-aminocarbonyle, dialkyl($C_1$-$C_4$)-aminocarbonyle ou phénylaminocarbonyle, qui est non substitué ou subtitué une fois sur le noyau phényle par un atome d'halogène ou par un groupe alkyle en $C_1$-$C_4$, alcoxy en $C_1$-$C_4$, halogénoalkyle en $C_1$-$C_4$, cyano ou nitro;

pour aboutir à un ester de formule Ia dans lequel les radicaux $R^1$, $R^2$, $R^3$ et X sont tels que définis précédemment.

6. Procédé pour la préparation de composés de formule I

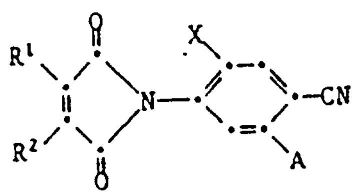

(I)

dans laquelle A représente $OR^4$ et les radicaux $R^1$, $R^2$, $R^4$ et X ont les significations données dans l'une des revendications 1 à 4, caractérisé par l'éthérification d'un phénol de formule VII, dans laquelle $R^1$, $R^2$ et X sont tels que définis précédemment

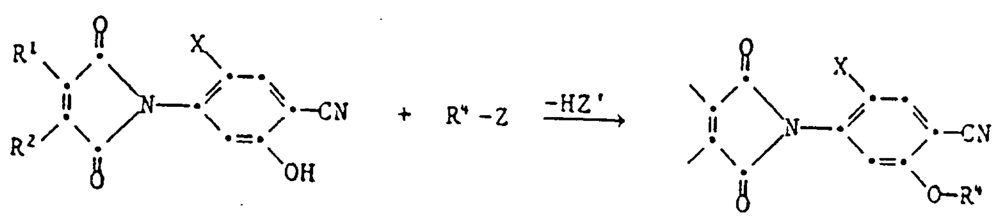


VII VIII Ib

avec un composé de formule VIII dans lequel $R^4$ est tel que défini précédemment et Z représente un

groupe séparable dans les conditions réactionnelles, tel qu'un atome d'halogène ou un groupe phénylsulfonyloxy éventuellement alkylé.

**7.** Procédé pour la préparation d'anilines de formule IIIb

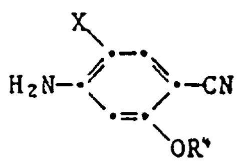

IIIb

dans laquelle

X représente un atome d'hydrogène ou d'halogène et

$R^4$ est groupe alkyle en $C_1$-$C_8$; alcoxy($C_1$-$C_4$)-alkyle($C_1$-$C_4$); alkyl($C_1$-$C_4$)thio-alkyle($C_1$-$C_4$), mono- ou dialkyl($C_1$-$C_4$)-amino-alkyle($C_1$-$C_4$); halogénoalkyle en $C_1$-$C_8$; cyanoalkyle en $C_1$-$C_8$; alcényle en $C_3$-$C_8$; halogénoalcényle en $C_3$-$C_8$; alcynyle en $C_3$-$C_8$; cycloalkyle en $C_3$-$C_7$; cycloalkyl($C_3$-$C_7$)-alkyle($C_1$-$C_4$); halogénocycloalkyle en $C_3$-$C_7$; alkyl($C_1$-$C_8$)-carbonyle; allylcarbonyle; un groupe benzylcarbonyle qui est non substitué ou substitué une fois sur le noyau phényle par un atome d'halogène ou par un radical alkyle en $C_1$-$C_4$, alcoxy en $C_1$-$C_4$, halogénoalkyle en $C_1$-$C_4$, cyano ou nitro; un groupe cycloalkyl($C_3$-$C_7$)-carbonyle; benzoyle qui est non substitué ou substitué une fois sur le noyau phényle par un atome d'halogène ou par un groupe alkyle en $C_1$-$C_4$, alcoxy en $C_1$-$C_4$, halogénoalkyle en $C_1$-$C_4$, cyano ou nitro; le groupe furoyle; thiénoyle; un groupe alkyle en $C_1$-$C_4$ substitué par un radical phényle, halogénophényle, alkyl($C_1$-$C_4$)-phényle, alcoxy($C_1$-$C_4$)-phényle, halogénoalkyl($C_1$-$C_4$)-phényle, halogénoalcoxy($C_1$-$C_4$)-phényle; nitrophényle, cyanophényle, alcoxy($C_1$-$C_8$)-carbonyle, alcoxy($C_1$-$C_4$)-alcoxy($C_1$-$C_4$)-carbonyle, alcényloxy($C_3$-$C_8$)-carbonyle, alcynyloxy($C_3$-$C_8$)-carbonyle, alkyl($C_1$-$C_8$)-thiocarbonyle, alcényl($C_3$-$C_8$)-thiocarbonyle, alcynyl($C_3$-$C_8$)-thiocarbonyle, carbamoyle, alkyl($C_1$-$C_4$)-aminocarbonyle, dialkyl($C_1$-$C_4$)-aminocarbonyle, hydroxy, phénylaminocarbonyle qui est non substitué ou substitué une fois sur le noyau phényle par un atome d'halogène ou par un radical alkyle en $C_1$-$C_4$, alcoxy en $C_1$-$C_4$, cyano, nitro ou halogénoalkyle en $C_1$-$C_4$; un groupe dioxolanne-2-yle qui est non substitué ou substitué par un ou deux radicaux alkyle en $C_1$-$C_4$, ou le groupe 1,3-dioxanne-2-yle qui est non substitué ou substitué par un ou deux radicaux alkyle en $C_1$-$C_4$,

à l'exception du 2-méthoxy-4-amino-5-chlorobenzonitrile;

caractérisé par

a) l'éthérification d'un phénol de formule

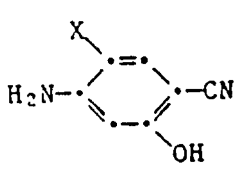

dans laquelle X est tel que défini précédemment, avec un composé de formule VIII

$R^4$-Z (VIII)

dans laquelle $R^4$ est tel que défini précédemment et Z représente un groupe séparable dans les conditions réactionnelles, tel qu'un halogène ou le groupe phénylsulfonyloxy, ou

b) l'échange halogène-cyanure dans un composé de formule

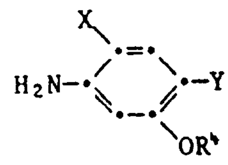

dans laquelle X et $R^4$ sont tels que définis précédemment et Y représente un atome de chlore, brome ou iode, avec CuCN, ou

c) la réduction d'un nitrobenzène de formule

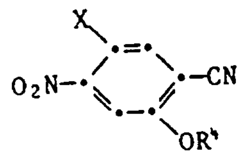

dans laquelle X et $R^4$ sont tels que définis précédemment.

**8.** Procédé pour la préparation d'anilines de formule

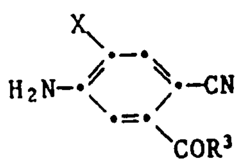

IIIc

dans laquelle

X représente un atome d'hydrogène ou d'halogène; et

$R^3$ représente un groupe hydroxy; alcoxy en $C_1$-$C_8$; alcoxy($C_1$-$C_4$)-alcoxy($C_1$-$C_4$); alkyl($C_1$-$C_4$)-thio-alcoxy($C_1$-$C_4$); mono- ou dialkyl($C_1$-$C_4$)-amino-alcoxy($C_1$-$C_4$); cyanoalcoxy en $C_1$-$C_8$; alcényloxy en $C_3$-$C_8$; halogénoalcényloxy en $C_3$-$C_8$; alcynyloxy en $C_3$-$C_8$; cycloalkyloxy en $C_3$-$C_7$ cycloalkyl($C_3$-$C_7$)-alkyloxy($C_1$-$C_4$); halogénocycloalcoxy en $C_3$-$C_7$; benzyloxy qui est non substitué ou substitué une fois sur le noyau phényle par un atome d'halogène ou par un radical alkyle en $C_1$-$C_4$, alcoxy en $C_1$-$C_4$, halogénoalkyle en $C_1$-$C_4$, cyano ou nitro; le groupe phénoxy; un groupe halogénophénoxy; alkyl($C_1$-$C_4$)-phénoxy; alkyloxy($C_1$-$C_4$)-phénoxy; halogénoalkyl($C_1$-$C_4$)-phénoxy; cyanophénoxy; nitrophénoxy; phénylthio; halogénophénylthio; alkyl($C_1$-$C_4$)-phénylthio; alcoxy($C_1$-$C_4$)-phénylthio; halogénoalkyl($C_1$-$C_4$)-thio; cyanophénylthio; nitrophénylthio; les groupes de type sel -O-Na, -O-K, -O-$(Ca)_{0,5}$, -O-$(Mg)_{0,5}$ ou -O-$NH_4$; le groupe amino; un groupe alkyl($C_1$-$C_4$)-amino; dialkyl($C_1$-$C_4$)-amino; halogénoalkyl($C_2$-$C_4$)-amino; dihalogénoalkyl($C_2$-$C_4$)-amino; hydroxyalkyl($C_1$-$C_4$)-amino; dihydroxyalkyl($C_1$-$C_4$)-amino; alcénylamino en $C_3$-$C_4$; diallylamino; -N-pyrrolidino; -N-pipéridino; -N-morpholino; -N-thiomorpholino; -N-pipéridazino; alkyl($C_1$-$C_8$)-thio; alcényl($C_3$-$C_8$)-thio; benzylthio; un groupe alkyl($C_1$-$C_4$)-thio substitué par un radical alcoxy($C_1$-$C_8$)-carbonyle, alcoxy($C_1$-$C_4$)-alcoxy($C_1$-$C_4$)-carbonyle, alcényloxy($C_3$-$C_8$)-carbonyle, alcynyloxy($C_3$-$C_8$)-carbonyle, alkylthio($C_1$-$C_8$)-carbonyle, alcénylthio($C_3$-$C_8$)-carbonyle ou alcynylthio($C_3$-$C_8$)-carbonyle; ou un radical alcoxy en $C_1$-$C_4$ substitué par un groupe alcoxy($C_1$-$C_8$)-carbonyle, alcoxy($C_1$-$C_4$)-alcoxy($C_1$-$C_4$)-carbonyle, alcényloxy($C_3$-$C_8$)-carbonyle, alcynyloxy($C_3$-$C_8$)-carbonyle, alkylthio($C_1$-$C_8$)-carbonyle, alcénylthio($C_3$-$C_8$)-carbonyle, alcynylthio($C_3$-$C_8$)-carbonyle, alkyl($C_1$-$C_4$)amino-carbonyle, dialkyl($C_1$-$C_4$)-aminocarbonyle ou phénylaminocarbonyle qui est non substitué ou substitué une fois sur le noyau phényle par un atome d'halogène ou par un groupe alkyle en $C_1$-$C_4$, alcoxy en $C_1$-$C_4$, halogénoalkyle en $C_1$-$C_4$, cyano ou nitro;

caractérisé par

a) l'échange halogène-cyanure dans un composé de formule

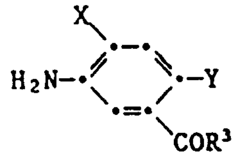

dans laquelle X et $R^3$ sont tels que définis précédemment et Y représente un atome de chlore, brome ou iode, avec CuCN, ou

b) la réduction d'un nitrobenzène de formule

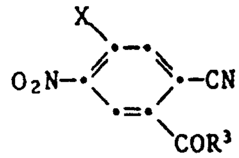

dans laquelle X et $R^3$ sont tels que définis précédemment.

**9.** Procédé pour la préparation de composés de formule VII

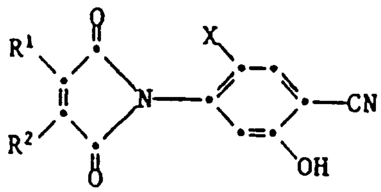

(VII)

dans laquelle

$R^1$ représente un atome d'hydrogène ou un groupe alkyle en $C_1$-$C_4$;

$R^2$ représente un groupe alkyle en $C_1$-$C_4$; ou

$R^1$ et $R^2$ représentent ensemble un groupe $-(CH_2)_4-$ qui peut être jusqu'à deux fois substitué par un radical ou des radicaux alkyle en $C_1$-$C_4$;

X représente un atome d'hydrogène ou d'halogène; caractérisé

a) par la réaction d'une furanne-2,5-dione de formule III avec une 5-hydroxyaniline

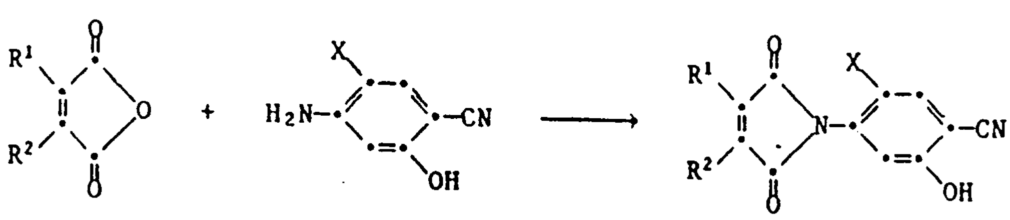

II

VII

les radicaux $R^1$, $R^2$ et X étant tels que définis précédemment, ou

b) par un échange halogène-cyanure selon l'équation suivante:

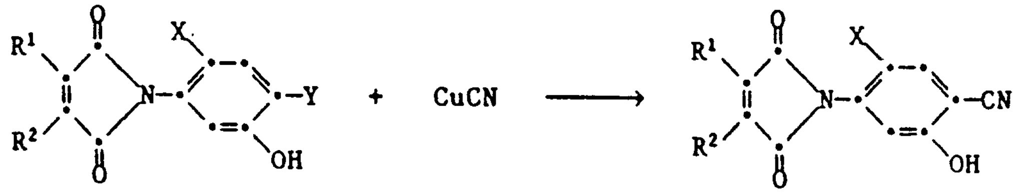

VII

dans laquelle $R^1$, $R^2$ et X sont tels définis précédemment et Y représente un atome de chlore, brome ou iode.

**10.** Procédé pour la préparation d'un produit herbicide ou régulateur de la croissance végétale, caractérisé en ce que l'on mélange avec les adjuvants et/ou matériaux de support un composé de formule I

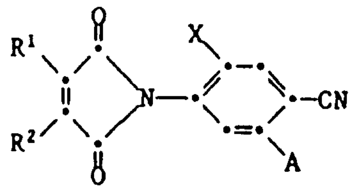

(I)

dans laquelle les radicaux $R^1$, $R^2$, X et A ont les significations données dans les revendications 1 à 4.

**11.** Procédé pour la lutte contre la croissance végétale indésirable, caractérisé en ce que l'on fait agir sur les plantes à combattre ou leur biotope une quantité à effet herbicide d'un composé de formule I

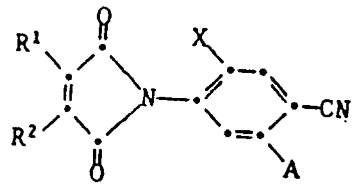

(I)

dans laquelle les radicaux $R^1$, $R^2$, X et A sont tels que définis dans l'une des revendications 1 à 4.

**12.** Procédé pour agir sur la croissance de plantes cultivées, caractérisé en ce que l'on fait agir sur la plante cultivée ou son biotope une quantité, à effet régulateur de la croissance végétale, d'un composé de formule I

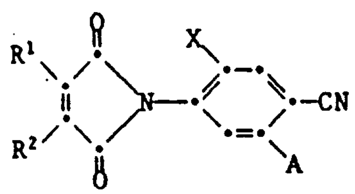

(I)

dans laquelle les radicaux $R^1$, $R^2$, X et A sont tels que définis dans l'une des revendications 1 à 4.

**13.** Procédé pour le traitement de semences, caractérisé en ce que l'on applique sur les semences des plantes cultivées une quantité à effet herbicide d'un composé de formule I

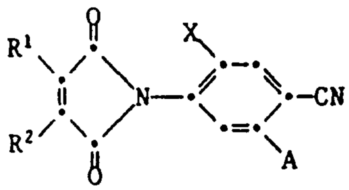

(I)

dans laquelle les radicaux $R^1$, $R^2$, X et A sont tels que définis dans l'une des revendications 1 à 4.